(19) Europäisches Patentamt
European Patent Office
Office européen des brevets

(11) EP 4 440 531 B1

(12) **EUROPEAN PATENT SPECIFICATION**

(45) Date of publication and mention of the grant of the patent:
**21.01.2026 Bulletin 2026/04**

(21) Application number: **22840841.5**

(22) Date of filing: **28.11.2022**

(51) International Patent Classification (IPC):
***A61K 8/06*** ^(2006.01) ***A61Q 19/00*** ^(2006.01)

(52) Cooperative Patent Classification (CPC):
**A61K 8/062; A61K 8/064; A61Q 19/00;**
A61K 2800/87; A61K 2800/882

(86) International application number:
**PCT/US2022/051049**

(87) International publication number:
**WO 2023/101902 (08.06.2023 Gazette 2023/23)**

(54) **KIT AND METHOD FOR TREATING SKIN**

KIT UND VERFAHREN ZUR HAUTBEHANDLUNG

KIT ET PROCÉDÉ DE TRAITEMENT DE LA PEAU

(84) Designated Contracting States:
**AT BE BG CH CY CZ DE DK EE ES FI FR GB GR HR HU IE IS IT LI LT LU LV MC ME MK MT NL NO PL PT RO RS SE SI SK SM TR**

(30) Priority: **30.11.2021 US 202163284239 P**
**31.01.2022 FR 2200833**

(43) Date of publication of application:
**09.10.2024 Bulletin 2024/41**

(73) Proprietor: **L'OREAL**
**75008 Paris (FR)**
Designated Contracting States:
**AT BE BG CH CY CZ DE DK EE ES FI FR GB GR HR HU IE IS IT LI LT LU LV MC ME MK MT NL NO PL PT RO RS SE SI SK SM TR**

(72) Inventors:
- **FAIG, Jonathan, James**
**Clark, NJ 07066 (US)**
- **WANG, Xiuxia**
**Shanghai, 201206 (CN)**
- **MA, Chi**
**Shanghai, 201206 (CN)**
- **HALPERN CHIRCH, Susan**
**Clark, NJ 07066 (US)**
- **GALDI, Angelike**
**Clark, NJ 07066 (US)**
- **CHANG, Ning**
**Shanghai, 201206 (CN)**
- **YOON, Yon, Jae**
**Clark, NJ 07066 (US)**

(74) Representative: **Lavoix**
**Bayerstraße 83**
**80335 München (DE)**

(56) References cited:
**WO-A1-2017/207805**

- **DATABASE GNPD [online] Mintel; 1 January 2014 (2014-01-01), ANONYMOUS: "System Red Regenerative Treatment", XP055969031, retrieved from https://www.gnpd.com/sinatra/recordpage/2247688/from_search/HUHju4hXDC/?page=1 Database accession no. 2247688**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 440 531 B1

## Description

## CROSS-REFERENCE TO RELATED APPLICATIONS

This application claims benefit of U.S. Serial No. 63/284,239 filed November 30, 2021, and benefit of French Application No. FR 2200833, filed on January 31 2022.

## FIELD OF THE DISCLOSURE

The instant disclosure relates to a kit comprising two or more separately contained cosmetic compositions that are miscible with one another upon mixing and to methods for treating skin using the kit and/or the two or more cosmetic compositions.

## SUMMARY

The instant disclosure is directed to, among other things, a kit comprising two or more separately contained cosmetic compositions and to methods for treating skin using the kit and/or the cosmetic compositions. The cosmetic compositions include surprisingly high amounts of various active agents, are stable, and miscible with one another. Active agents provide a myriad of cosmetic benefits to skin but have historically been difficult to solubilize and stabilize, especially in high amounts. It is also challenging to develop cosmetic compositions with differing active agents that are miscible with one another. Often a cosmetic composition that stabilizes and/or solubilizes a particular active agent does not homogenously mix with another cosmetic composition that stabilizes and/or solubilizes a different active agent, especially when the architecture of the cosmetic compositions is different. The mixing of disparate cosmetic compositions often results in visual coagulation, separation of components or phases (e.g., oil, water, silicone, etc.), precipitation of ingredients including active agents, etc.

WO2017207805 A1 discloses a kit comprising at least two cosmetic compositions contained separately in cartridges.

Mintel GNPD Record ID 2247688 "System Red Regenerative Treatment", Jan 2014 discloses a kit comprising two cosmetic compositions contained separately.

In an aspect, the instant disclosure is directed to, among other things, a kit comprising two or more separately contained cosmetic compositions that are miscible with one another upon mixing, wherein each of the two or more compositions comprise:

(a) a viscosity of at least 1.8 Pa.s (1,800 cPs) or a storage modulus (G') greater than
the loss modulus (G") for a viscosity below **1.8** Pa.s (1,800 cPs); and
(b) a maximum yield stress of 9,000 Pa, for example, a maximum yield stress below 7,500 Pa, below 5,000 Pa, below 2,500 Pa, below 2,000 Pa, below 1,500 Pa, or below 1,250 Pa;
wherein the two or more cosmetic compositions are chosen from:

(i) oil in water emulsions comprising at least 40 wt.% of water and about 1 to about 20 wt.% of an oil phase, based on the total weight of the oil in water emulsion;
(ii) water in silicone emulsions comprising at least 70 wt.% of an aqueous phase (internal phase), based on the total weight of the silicone emulsion; and
(iii) anhydrous compositions, wherein at least 90 wt.% of ingredients forming the anhydrous compositions have a log P value of 2 or less, for example, 1.5 or less, 1 or less, 0.75 or less, 0.5 or less, 0.25 less, or 0.

In an aspect, the instant disclosure is directed to, among other things, a method for treating skin comprising:

(A) mixing two or more cosmetic compositions that are miscible with one another upon mixing, wherein each of the two or more compositions comprise:

(a) a viscosity of at least **1.8** Pa.s (1,800 cPs) or a storage modulus (G')
greater than the loss modulus (G") for a viscosity below 1.8 Pa.s (1,800 cPs); and
(b) a maximum yield stress of 9,000 Pa, for example, a maximum yield stress below 7,500 Pa, below 5,000 Pa, below 2,500 Pa, below 2,000 Pa, below 1,500 Pa, or below 1,250 Pa; and
wherein the two or more cosmetic compositions are chosen from:

(i) oil in water emulsions comprising at least 40 wt.% of water and about 1 to about 20 wt.% of an oil phase,

based on the total weight of the oil in water emulsion;

(ii) water in silicone emulsions comprising at least 70 wt.% of an aqueous phase (internal phase), based on the total weight of the silicone emulsion; and

(iii) anhydrous compositions, wherein at least 90 wt.% of ingredients forming the anhydrous compositions have a log P value of 2 or less, for example, 1.5 or less, 1 or less, 0.75 or less, 0.5 or less, 0.25 less, or 0; and

(B) applying the mixture to the skin.

In certain embodiments, at least one of the two or more cosmetic compositions is oil in water emulsion, for example an oil in water emulsion as described herein.

In certain embodiments, at least one of the two or more cosmetic compositions is a water in silicone emulsion, for example, a water in silicone emulsion as described herein.

In certain embodiments, at least one of the two or more cosmetic compositions is an anhydrous composition, for example an anhydrous composition as described herein.

Nonlimiting examples of active agents that may be included in cosmetic compositions in the form of an oil in water emulsion include retinol, hydroxypropyl tetrahydropyrantriol, 4-t-butylcyclohexanol, trifluoromethylphenyl valylglycine, ceramides (e.g., ceramide-NP), madecassoside, and mixtures thereof.

A nonlimiting example of an active agent that may be included in cosmetic compositions in the form of a water in silicone emulsion include hydroxypropyl tetrahydropyrantriol, madecassoside, glycerin and mixtures thereof

Nonlimiting examples of active agents that may be included in anhydrous cosmetic compositions include ascorbic acid, ferulic acid, glycerin, polygonum cuspidatum root extract, and mixtures thereof.

In certain embodiments, the kit is an apparatus for independently dispensing a specified amount of each of the two or more cosmetic compositions, wherein the apparatus comprises a dispensing assembly configured to receive a plurality of cartridges in which the two or more cosmetic compositions are separately contained and a receiving area into which each of the two or more cosmetic compositions is dispensed. In an embodiment, each cartridge of the plurality of cartridges contains a different cosmetic composition. In another embodiment, the apparatus further comprises a memory configured to receive and store dispensing information which includes the specified amount of cosmetic composition to be dispensed from each cartridge; and circuitry configured to obtain the dispensing information from the memory and to control the dispensing assembly to dispense the specified amount of the cosmetic composition to be dispensed from each cartridge. Optionally, the receiving portion of the apparatus is configured to be part of a detachable portion of the apparatus and the detachable portion is configured to be an enclosed container that holds the dispensed cosmetic composition(s).

In an embodiment, the methods improve elasticity, promote the production of hyaluronic acid, synthesis of collagen, synthesis of epidermal structural components, regeneration of damaged tissue, and reduce the effects of aging of the skin of the face or body.

In certain embodiments the methods include reinforcing or improving the natural lipid barrier of the skin; treating dry and/or aging skin; maintaining and/or improving moisture balance of skin; and/or improving the overall appearance of skin.

In certain embodiments, especially when at least one of the two or more cosmetic compositions include hydroxypropyl tetrahydropyrantriol, the methods reduce of fine lines and wrinkles, improve production of hyaluronic acid via stimulation of glycosaminoglycan (GAG) synthesis, soften stratum corneum to relieve cumulative stress on the epidermis and dermis, *etc.*

In certain embodiments, especially when at least one of the two or more cosmetic compositions include retinol, the methods boost collagen and/or elastin production, reduce the appearance of fine lines, wrinkles, and uneven skin tone, tighten the skin, prevent and/or treat acne, decrease the development of melanin, and improve and brighten complexion.

In certain embodiments, especially when at least one of the two or more cosmetic compositions include ceramides (e.g., ceramide-NP), the methods hydrate skin and maintain skin's moisture balance, alleviate/reduce itching, chronic dryness, peeling, and scaling. Such methods are also useful for reinforcing the natural lipid barrier of skin, which helps treat dry and aging skin.

In certain embodiments, especially when at least one of the two or more cosmetic composition include 4-tert-butylcyclohexanol, the methods reduce skin irritation, sooth the skin, and/or reduce or alleviate stinging, burning, and tightness.

In certain embodiments, especially when at least one of the two or more cosmetic compositions include acetyl trifluoromethylphenyl valylglycine, the methods improve elasticity, promote the production of hyaluronic acid, synthesis of collagen, synthesis of epidermal structural components, regeneration of damaged tissue, and reduce the effects of aging of skin of the face or body.

Other features and iterations of the invention are described in more detail below.

## DESCRIPTION

A common problem associated with cosmetic compositions, especially composition comprising multiple components, is ensuring physical stability, chemical stability, solubility, and the like throughout the life of the cosmetic product. Many active agents are difficult to stabilize and solubilize, especially when used in high amounts. The consequence of stability and solubility problems is significant. For example, stability problems can cause partial, if not complete, loss of product integrity, color loss, malodor, viscosity changes, *etc.* Stability and solubility problems can cause an increased or a decreased amount of the component in question to be applied. With respect to active ingredients, stability and solubility problems reduce or eliminate activity, and prevent the active ingredients from reaching their intended target in the desired amount.

With aging, the outer skin layer (epidermis) thins, even though the number of cell layers remains unchanged. The number of pigment-containing cells (melanocytes), however, decreases. Therefore, the skin appears pale and translucent. Large pigmented spots (age spots, liver spots, or lentigos) may appear in sun-exposed areas. Changes in the connective tissue reduce the skin's strength and elasticity. This is known as elastosis. It is more noticeable in sun-exposed areas (solar elastosis). Elastosis produces the leathery, weather-beaten appearance common to farmers, sailors, and others who spend a large amount of time outdoors. Dehydration increases the risk of skin injury. Poor nutrition can also negatively influence the skin, causing dryness, rash, and puffiness.

Human skin acts as a primary barrier between the body and its environment. Crucial for this skin barrier function is the lipid matrix in the outermost layer of the skin (epidermis), the stratum corneum (SC). Two of its functions are (1) to prevent excessive water loss through the epidermis and (2) to avoid that compounds from the environment permeate into the viable epidermal and dermal layers and thereby provoke an immune response. The composition of the SC lipid matrix is dominated by three lipid classes: cholesterol, free fatty acids, and ceramides. These lipids adopt a highly ordered, 3-dimensional structure of stacked densely packed lipid layers (lipid lamellae): the lateral and lamellar lipid organization. The way in which these lipids are ordered depends on the composition of the lipids. One very common skin disease in which the SC lipid barrier is affected is atopic dermatitis (AD).

What is needed, among other things, are compositions containing high amounts of active agents that treat, nourish, and improve the look of skin, which are stable and compatible with one another. The cosmetic compositions of the instant case address these requirements. Although the compositions of the instant case include high amounts of one or more active agents, they are surprisingly stable. In addition, the cosmetic compositions are surprisingly compatible with one another, i.e., they are miscible with each other. The various cosmetic compositions can be mixed with one another to form a homogenous and aesthetically pleasing mixture for application to the skin. Different active agents provide different benefits to the skin. The various cosmetic compositions described herein (containing a variety of different active agents) can be selected and mixed with one another to create a unique mixture that addresses the specific needs of an individual consumer's skin. In other words, cosmetic compositions containing the needed active agents can be selected and mixed to provide a "consumer specific" mixture containing the plurality of active agents appropriate for an individual consumer's skin type.

In an embodiment, the kits of the instant disclosure include two or more separately contained cosmetic compositions that are miscible with one another upon mixing, wherein each of the two or more compositions comprise:

(a) a viscosity of at least **1.8** Pa.s (1,800 cPs) or a storage modulus (G') greater than the loss modulus (G") for a viscosity below **1.8** Pa.s (1,800 cPs); and
(b) a maximum yield stress of 9,000 Pa, for example, a maximum yield stress below 7,500 Pa, below 5,000 Pa, below 2,500 Pa, below 2,000 Pa, below 1,500 Pa, or below 1,250 Pa; and
wherein the two or more cosmetic compositions are chosen from:

(i) oil in water emulsions comprising at least 40 wt.% of water and about 1 to about 20 wt.% of an oil phase, based on the total weight of the oil in water emulsion;
(ii) water in silicone emulsions comprising at least 70 wt.% of an aqueous phase (internal phase), based on the total weight of the silicone emulsion; and
(iii) anhydrous compositions, wherein at least 90 wt.% of ingredients forming the anhydrous compositions have a log P value of 2 or less, for example, 1.5 or less, 1 or less, 0.75 or less, 0.5 or less, 0.25 less, or 0.

In an embodiment, the methods of the instant disclosure comprise:

(A) mixing two or more cosmetic compositions that are miscible with one another upon mixing, wherein each of the two or more compositions comprise:

(a) a viscosity of at least **1.8** Pa.s (1,800 cPs) or a storage modulus (G')

greater than the loss modulus (G") for a viscosity below 1.8 Pa.s (1,800 cPs); and

(b) a maximum yield stress of 9,000 Pa, for example, a maximum yield stress below 7,500 Pa, below 5,000 Pa, below 2,500 Pa, below 2,000 Pa, below 1,500 Pa, or below 1,250 Pa; and

wherein the two or more cosmetic compositions are chosen from:

(i) oil in water emulsions comprising at least 40 wt.% of water and about 1 to about 20 wt.% of an oil phase, based on the total weight of the oil in water emulsion;

(ii) water in silicone emulsions comprising at least 70 wt.% of an aqueous phase (internal phase), based on the total weight of the silicone emulsion; and

(iii) anhydrous compositions, wherein at least 90 wt.% of ingredients forming the anhydrous compositions have a log P value of 2 or less, for example, 1.5 or less, 1 or less, 0.75 or less, 0.5 or less, 0.25 less, or 0; and

(B) applying the mixture to the skin.

The viscoelastic properties of a material are conventionally defined by two characteristic values: (1) the storage modulus, which represents the elastic behavior of a material for a given frequency conventionally written as G'; and (2) the loss modulus, which represents the viscous behavior of a material for a given frequency conventionally written as G". These magnitudes are defined, for example, in the "Handbook of Pressure Sensitive Adhesive Technology" 3rd edition, D. Satas, chap. 9, pp. 155 to 157. Viscoelastic properties can be ascertained using rheology, for example with a DHR-2 Rheometer (TA Instruments, New Castle, Delaware, USA) at 20°C. A 2° cone plate (diameter = 40 mm) is used for these rheology measurements. A strain sweep is performed from 0.01% to 1000% strain at a fixed frequency $\omega$ = 1 rad/s.

In certain embodiments, at least one of the two or more cosmetic compositions is oil in water emulsion. In further embodiments, at least one of the two or more cosmetic compositions is an oil in water emulsion and at least one of the two or more cosmetic compositions is a water in silicone emulsion of an anhydrous composition.

In certain embodiments, at least one of the two or more cosmetic compositions is a water in silicone emulsion. In further embodiments, at least one of the two or more cosmetic composition is a water in silicone emulsion and at least one of the cosmetic compositions is an oil in water emulsion or an anhydrous composition.

In certain embodiments, at least one of the two or more cosmetic compositions is an anhydrous composition. In further embodiments, at least one of the two or more cosmetic compositions is an anhydrous composition and at least one of the two or more compositions is an oil in water emulsion or a water in silicone emulsion.

In certain embodiments, the kits and methods employ three or more separately contained cosmetic compositions that are miscible with one another upon mixing. In a preferred embodiment, at least one of the three or more cosmetic compositions is in a form that differs from another form of another cosmetic composition of the three or more cosmetic compositions. In other words, not all the three or more compositions are oil in water emulsions; or not all of the three or more compositions are water in silicone emulsions; or not all the three or more compositions are anhydrous compositions.

In embodiments wherein at least one of the cosmetic compositions is an oil in water emulsion, the oil in water emulsion includes retinol, hydroxypropyl tetrahydropyrantriol, 4-t-butylcyclohexanol, trifluoromethylphenyl valylglycine, cermides, or a mixture thereof. In an embodiment, the oil in water emulsion includes hydroxypropyl tetrahydropyrantriol. In an embodiment, the oil in water emulsion includes 4-t-butylcyclohexanol. In an embodiment, the oil in water emulsion includes both hydroxypropyl tetrahydropyrantriol and 4-t-butylcyclohexanol. In an embodiment, the oil in water emulsion includes trifluoromethylphenyl valylglycine. In an embodiment, the oil in water emulsion includes ceramides, preferably ceramide-NP.

In embodiments wherein at least one of the cosmetic compositions is a water in silicone emulsion, the water in silicone emulsion includes hydroxypropyl tetrahydropyrantriol.

In embodiments wherein at least one of the cosmetic compositions is an anhydrous composition, the anhydrous composition includes ascorbic acid, ferulic acid, or a mixture thereof. In an embodiment, the anhydrous composition includes ascorbic acid. In an embodiment, the anhydrous composition includes ferulic acid. In an embodiment, the anhydrous composition includes both ascorbic acid and ferulic acid.

The term "kit" refers to a combination of parts, compositions, and/or packaging, which may be in the form of an assembly, a device, a packaged product, and the like. The kits of the instant case include two or more separately contained cosmetic compositions that are miscible with one another upon mixing. In an embodiment, the kit may be a packaged product, wherein the packaging includes two or more independent containers or bottles, each housing a different cosmetic composition. In another embodiment, the two or more separately contained cosmetic compositions may be included in a single container or bottle having two or more different chambers for separately housing each cosmetic composition.

In another embodiment, the kit is an apparatus, for example, an apparatus that separately houses the two or more cosmetic compositions, for example, in two or more cartridges within the apparatus; and independently dispenses the two or more cosmetic composition. For example, the apparatus may include a dispensing assembly configured to receive a plurality of cartridges in which the two or more cosmetic compositions are separately contained and a receiving area into

which each of the two or more cosmetic compositions is dispensed. In a preferred embodiment, the apparatus dispenses a specified amount of each of the two or more cosmetic compositions. In a further embodiment, each cartridge of the plurality of cartridges contains a different cosmetic composition. The certain embodiments, the apparatus further includes a memory configured to receive and store dispensing information, such as the specified amount of cosmetic composition to be dispensed from each cartridge; and circuitry configured to obtain the dispensing information from the memory and to control the dispensing assembly to dispense the specified amount of the cosmetic composition to be dispensed from each cartridge. In an embodiment, the apparatus comprises a receiving into which the two or more cosmetic composition are dispensed. In certain embodiment, the receiving area is configured to be part of a detachable portion of the apparatus. The detachable portion may optionally be configured to be an enclosed container that holds the dispensed cosmetic composition(s).

In an embodiment, the apparatus includes a dispensing assembly configured to receive at least two cartridges, each containing a cosmetic composition, and to dispense a specified amount of the cosmetic compositions from the cartridges into a receiving area; a memory configured to receive and store dispensing information which includes the specified amount of the cosmetic compositions to be dispensed for each cartridge disposed in the dispensing assembly to achieve a specified single use of a combination of the cosmetic compositions; and circuitry configured to obtain the dispensing information from the memory and to control the dispensing assembly to dispense the cosmetic compositions from each cartridge disposed in the dispensing assembly into the receiving area according to the one or more specified amounts included in the dispensing information.

In an embodiment, the apparatus comprises a dispensing assembly configured to receive two or more (a plurality) cartridges that each contain a different cosmetic composition and to dispense a specified amount of the different cosmetic compositions from the two or more cartridges into a receiving area; a memory configured to receive and store dispensing information which includes the specified amount of each cosmetic composition to be dispensed for each cartridge disposed in the dispensing assembly to achieve a specified single use mixture of the two or more cosmetic compositions; and circuitry configured to obtain the dispensing information from the memory and to control the dispensing assembly to dispense the two or more cosmetic compositions from each cartridge disposed in the dispensing assembly into the receiving area according to the one or more specified amounts included in the dispensing information.

In an embodiment, the apparatus further comprises a manifold having a plurality of manifold through holes, the manifold connected to and disposed on a nozzle of each of the two or more cartridges, the receiving area being connected to and disposed above the manifold, wherein the circuitry controls the dispensing assembly to dispense a quantity of the two or more cosmetic compositions from the nozzles of the two or more cartridges through a manifold through hole of the manifold, and into the receiving area.

In an embodiment, the dispensing assembly is configured to receive three or more cartridges, each containing a different cosmetic composition, and to simultaneously dispense a specified amount of the three or more cosmetic compositions from each of the cartridges into the receiving area.

In an embodiment, the enclosed container includes: a top lid; a base, having a plurality of base through holes; and a bottom lid, having a plurality of bottom lid through holes, wherein the top lid is connected to a first side of the base, the bottom lid is connected to a second side of the base, the bottom lid is connected to the manifold, the plurality of manifold through holes are aligned with and connected to the plurality of bottom lid through holes, the plurality of base through holes aligned with and connected to the plurality of base through holes. In a further embodiment, the enclosed container further comprises: a plurality of mounting magnets, disposed between the base and the bottom lid, wherein the plurality of mounting magnets magnetically secure the enclosed container to the manifold. In yet another embodiment, the enclosed container further comprises: a plurality of lid magnets, disposed between the base and the bottom lid, wherein the plurality of lid magnets magnetically secure the top lid to the base and the bottom lid. In another embodiment, the enclosed container further comprises: a plurality of hinge magnets, wherein half of the plurality of hinge magnets are disposed between the base and the bottom lid, half of the plurality of hinge magnets are disposed within the top lid, the plurality of hinge magnets disposed within the top lid having opposite magnetic polarity of the corresponding plurality of hinge magnets disposed between the base and the bottom lid, the top lid magnetically hinged and disposed about the base and the bottom lid in at least one position. In an embodiment, the top lid is only magnetically connected to the base and the bottom lid, and the top lid is fully removable from the base and the bottom lid.

The instant disclosure relates to methods of treating skin using the kits/apparatus/device and methods described herein. The methods include applying a mixture of cosmetic compositions according to the instant disclosure to the skin, and optionally allowing the mixture of cosmetic compositions to remain on the skin for a period of time. The mixture of cosmetic compositions is typically applied directly to the skin using the hand or a cloth. The skin may be optionally washed or rinsed prior to application. The method for treating the skin can be carried out once daily or may be carried out multiple times. For example, the method for treating skin may be carried out once daily, twice daily, weekly, bi-weekly for an extended period of time, for example, for about 1, 2, 3, 4, 5, or 6 months up to 1 year, or longer. In an embodiment, the methods include dispensing two or more cosmetic compositions from an apparatus that separately houses the two or more cosmetic compositions, for example in two or more cartridges; mixing the two or more cosmetic compositions; and

applying the mixture to skin. In an embodiment, the apparatus dispenses a specified amount of each of the two or more cosmetic compositions. In another embodiment, the apparatus dispenses amounts of the two or more cosmetic composition sufficient for a single application or treatment with a mixture of the amounts of the two or more cosmetic compositions. In an embodiment, the apparatus dispenses the two or more cosmetic compositions into a receiving portion of the apparatus. The two or more cosmetic composition may be mixed together in the receiving portion of the apparatus or may be transferred to another area for mixing, for example, into the hand or hands of an individual. In an embodiment, the individual mixes the two or more cosmetic compositions together, for example, in the individual's hands, and subsequently applies the mixture to the skin.

The two or more cosmetic compositions are typically mixed with each other shortly before application to the skin. In certain embodiments, the two or more cosmetic compositions are mixed with each other within 24 hours of application to the skin. In further embodiments, the two or more cosmetic composition are mixed with each other within 12 hours, 6 hours, 1 hour, 30 minutes, 10 minutes, 5 minutes, or 1 minute of application to the skin. In a preferred embodiment, the two or more cosmetic compositions are mixed immediately before application, i.e., within about 1 minute, about 30 seconds, or about 20 seconds before application.

Cosmetic compositions containing hydroxypropyl tetrahydropyrantriol provide benefits such as reduction of fine lines and wrinkles, improving production of hyaluronic acid via stimulation of glycosaminoglycan (GAG) synthesis, softening of stratum corneum to relieve cumulative stress on the epidermis and dermis, *etc.* Thus, the methods of the instant case relate to improving production of hyaluronic acid via stimulation of glycosaminoglycan (GAG) synthesis, softening of stratum corneum to relieve cumulative stress on the epidermis and dermis, *etc.*

Cosmetic composition containing retinol provide boost collagen and/or elastin production, reduce the appearance of fine lines, wrinkles, and uneven skin tone, tighten the skin, prevent and/or treat acne, decrease the development of melanin, and improve and brighten complexion. Thus, the methods of the instant disclosure relate to reducing the appearance of fine lines, wrinkles, and uneven skin tone, tightening the skin, preventing and/or treating acne, decreasing the development of melanin, and improving and brightening complexion.

Cosmetic compositions comprising ceramides (e.g., ceramide-NP) hydrate skin and maintain moisture balance, alleviate/reduce itching, chronic dryness, peeling, and scaling. Ceramide-NP is also helpful for reinforcing the natural lipid barrier of skin, which helps treat dry and aging skin. Thus, the methods of the instant disclosure relate to hydrating skin and maintaining of moisture balance, alleviating/reducing itching, chronic dryness, peeling, and scaling; and to reinforcing the natural lipid barrier of skin, to treat dry and aging skin.

Cosmetic compositions comprising 4-tert-butylcyclohexanol reduce skin irritation, sooth the skin, and/or reduce or alleviate stinging, burning, and tightness. Thus, the methods of the instant disclosure relate to reducing skin irritation, soothing the skin, and/or reducing or alleviating stinging, burning, and tightness

Cosmetic compositions comprising acetyl trifluoromethylphenyl valylglycine improve elasticity, promote the production of hyaluronic acid, synthesis of collagen, synthesis of epidermal structural components, regeneration of damaged tissue, and reduce the effects of aging of skin of the face or body. Thus, the methods of the instant disclosure relate to improving elasticity, promoting the production of hyaluronic acid, synthesis of collagen, synthesis of epidermal structural components, regeneration of damaged tissue, and reducing the effects of aging of skin of the face or body.

The cosmetic compositions useful in the kits and methods mentioned above are described in more detail below, under the heaings "Oil in Water Emulsion," "Water in Silicone Emulsions," and "Anhydrous Compositions."

## OIL IN WATER EMULSIONS

The cosmetic compositions in the form of oil in water phase emulsions are characterized in that they comprise at least 40 wt.% of water and about 1 to about 20 wt.% of an oil phase, based on the total weight of the oil in water emulsion. Furthermore, the oil in water emulsions (or simply, cosmetic compositions) include one or more active agents, preferably one or more active agents that are stabilized and/or solubilized in oil phase or the water phase of the emulsions. In a preferred embodiment, at least one of the one or more active agents is retinol, hydroxypropyl tetrahydropyrantriol, 4-t-butylcyclohexanol, trifluoromethylphenyl valylglycine, ceramides, or a mixture thereof.

### *Oil in Water Emulsions Comprising Retinol*

In an embodiment, the cosmetic compositions in the form of oil in water emulsions include retinol. The amount of retinol will vary. Nonetheless, in various embodiments, the amount of retinol is from about 0.05 to about 6 wt.%, based on the total weight of the composition. In further embodiments, the amount of retinol in the compositions is from about 0.05 to about 5 wt.%, about 0.05 to about 4 wt.%, about 0.05 to about 3 wt.%, about 0.05 to about 2 wt.%, about 0.1 to about 6 wt.%, about 0.1 to about 5 wt.%, about 0.1 to about 4 wt.%, about 0.1 to about 3 wt.%, about 0.1 to about 2 wt.%, about 0.5 to about 5 wt.%, about 0.5 to about 4 wt.%, about 0.5 to about 3 wt.%, or about 0.5 to about 2 wt.%, based on the total weight of the

In an embodiment, the cosmetic composition includes:

(a) retinol, preferably in the amounts set forth above;

(b) a plurality of nonionic emulsifiers comprising:

(i) one or more ethoxylated fatty acids; and

(ii) one or more nonionic emulsifiers having a Hydrophile-Lipophile Balance (HLB) from about 9 to about 12;

(iii) optionally, one or more glyceryl esters;

(iv) optionally, one or more additional nonionic emulsifiers;

(c) one or more fatty alcohols;

(d) one or more fatty compounds;

(e) one or more thickening polymers; and

(f) water.

The plurality of nonionic emulsifiers represents all of the nonionic emulsifiers in the cosmetic compositions.

The total amount of the plurality of nonionic emulsifiers (all nonionic emulsifiers) in the cosmetic compositions will vary. Nonetheless, in certain embodiments, the total amount of the plurality of nonionic emulsifiers is from about 1 to about 8 wt.%, based on the total weight of the cosmetic composition. In further embodiments, the total amount of the plurality of nonionic emulsifiers is from about 1 to about 6 wt.%, about 1 to about 5 wt.%, about 1 to about 4 wt.%, about 1 to about 3 wt.%, about 1.1 to about 8 wt.%, about 1.1 to about 6 wt.%, about 1.1 to about 5 wt.%, about 1.1 to about 4 wt.%, about 1.1 to about 3 wt.%, about 1.1 to about 2 wt.%, about 1.5 to about 8 wt.%, about 1.5 to about 6 wt.%, about 1.5 to about 5 wt.%, about 1.5 to about 4 wt.%, or about 1.5 to about 3 wt.%, 2 to about 8 wt.%, about 2 to about 6 wt.%, about 2 to about 5 wt.%, about 2 to about 4 wt.%, or about 2 to about 3 wt.%, based on the total weight of the cosmetic composition.

Nonlimiting examples of ethoxylated fatty acids include the adducts of ethylene oxide with esters of lauric acid, palmitic acid, stearic acid or behenic acid, and mixtures thereof, especially those containing from 9 to 100 oxyethylene groups, such as PEG-9 to PEG-50 laurate (as the INCI names: PEG-9 laurate to PEG-50 laurate); PEG-9 to PEG-50 palmitate (as the INCI names: PEG-9 palmitate to PEG-50 palmitate); PEG-9 to PEG-50 stearate (as the INCI names: PEG-9 stearate to PEG-50 stearate); PEG-9 to PEG-50 palmitostearate; PEG-9 to PEG-50 behenate (as the INCI names: PEG-9 behenate to PEG-50 behenate); polyethylene glycol 100 EO monostearate (INCI name: PEG-100 stearate); and mixtures thereof. A more exhaustive but nonlimiting list of useful ethoxylated fatty acids are provided under the heading "Ethoxylated Fatty Acids."

The total amount of the one or more ethoxylated fatty acids may be from about 0.1 to about 5 wt.%, based on the total weight of the cosmetic composition. In various embodiments, the total amount of the one or more ethoxylated fatty acids may be from about 0.1 to about 4 wt.%, about 0.1 to about 3 wt.%, about 0.1 to about 2 wt.%, about 0.2 to about 5 wt.%, about 0.2 to about 4 wt.%, about 0.2 to about 3 wt.%, or about 0.2 to about 2 wt.%, about 0.4 to about 5 wt.%, about 0.4 to about 4 wt.%, about 0.4 to about 3 wt.%, or about 0.4 to about 2 wt.%, based on the total weight of the cosmetic composition.

Nonlimiting examples of nonionic emulsifiers having a Hydrophile-Lipophile Balance (HLB) from about 9 to about 12 include alkylpolyglucosides (e.g., C12-20 alkyl glucoside), polyglycerol-based emulsifiers (e.g., polygyceryl-3 methylglucose distearate), sorbitan fatty esters, sugar fatty esters, polyol fatty esters, ethoxylates thereof, or a mixture thereof. A more exhaustive but nonlimiting list of nonionic emulsifiers having an HLB from about 9 to about 12 are included under the heading, "Nonionic Emulsifiers having an HLB from 9 to 12."

The total amount of the one or more nonionic emulsifiers having an HLB from 9 to 12 will vary. Nonetheless, in certain embodiments, the total amount of the one or more nonionic emulsifiers having an HLB from 9 to 12 is from about 0.1 to about 5 wt.%, based on the total weight of the cosmetic composition. In further embodiments, the total amount of the one or more nonionic emulsifiers having an HLB from 9 to 12 is from about 0.1 to about 4 wt.%, about 0.1 to about 3 wt.%, about 0.1 to about 2 wt.%, about 0.2 to about 5 wt.%, about 0.2 to about 4 wt.%, about 0.2 to about 3 wt.%, or about 0.2 to about 2 wt.%, about 0.3 to about 5 wt.%, about 0.3 to about 4 wt.%, about 0.3 to about 3 wt.%, or about 0.3 to about 2 wt.%, about 0.5 to about 5 wt.%, about 0.5 to about 4 wt.%, about 0.5 to about 3 wt.%, or about 0.5 to about 2 wt.%, about 1 to about 5 wt.%, about 1 to about 4 wt.%, about 1 to about 3 wt.%, or about 1 to about 2 wt.%, based on the total weight of the cosmetic composition.

Nonlimiting examples of glyceryl esters include bis-diglyceryl polyacyladipate-2, glyceryl behenate, glyceryl

caprate, glyceryl cocoate, glyceryl erucate, glyceryl hydroxystearate, glyceryl isostearate, glyceryl lanolate, glyceryl laurate, glyceryl linoleate, glyceryl myristate, glyceryl oleate, glyceryl palmitate lactate, glyceryl sesquioleate, glyceryl stearate, glyceryl dioleate, glyceryl distearate, glyceryl laurate, or a mixture thereof. In at least one instance the glyceryl ester comprises glyceryl stearate, bis-diglyceryl polyacyladipate, glyceryl ricinoleate, and mixtures thereof. A more exhaustive but nonlimiting list of glyceryl esters is included under the heading "Glyceryl Fatty Esters."

The total amount of the one or more glyceryl esters, if present, will vary. Nonetheless, in certain embodiments, the total amount of the one or more glyceryl esters, when present, is from about 0.1 to about 5 wt.%, based on the total weight of the cosmetic composition. In further embodiments, the total amount of the one or more glyceryls esters is from about 0.1 to about 4 wt.%, about 0.1 to about 3 wt.%, about 0.1 to about 2 wt.%, about 0.2 to about 5 wt.%, about 0.2 to about 4 wt.%, about 0.2 to about 3 wt.%, or about 0.2 to about 2 wt.%, about 0.3 to about 5 wt.%, about 0.3 to about 4 wt.%, about 0.3 to about 3 wt.%, or about 0.3 to about 2 wt.% based on the total weight of the cosmetic composition.

Nonlimiting examples of fatty alcohols include fatty alcohols having from 12 to 24 carbon atoms, in particular, C14-24 alcohols, cetyl alcohol, stearyl alcohol, cetearyl alcohol, behenyl alcohol, lauryl alcohol, myristic or myristyl alcohol, arachidyl alcohol, lignoceryl alcohol, and mixtures thereof. A more exhaustive but nonlimiting list of fatty alcohols is included under the heading "Fatty Alcohols"

The total amount of the one or more fatty alcohols in the cosmetic compositions will vary. Nonetheless, in certain embodiments, the total amount of the one or more fatty alcohols is from 0.1 wt.% to about 8 wt.%, based on the total weight of the composition. In further embodiments, the total amount of one or more fatty alcohols in the composition is from about 0.1 to about 6 wt.%, about 0.1 to about 5 wt.%, about 0.1 to about 4 wt.%, about 0.5 to about 8 wt.%, about 0.5 to about 6 wt.%, about 0.5 to about 5 wt.%, about 0.5 to about 4 wt.%, about 1 to about 8 wt.%, about 1 to about 6 wt.%, about 1 to about 5 wt.%, about 1 to about 4 wt.%, or about 1 to about 3 wt.%, based on the total weight of the composition.

Nonlimiting examples of fatty compounds include fatty esters (e.g., diisopropyl sebacate, isononyl isononanoate), polyolefins (petrolatum), waxes, squalane, squalene, hydrogenated polyisobutene, hydrogenated polydecene, polybutene, mineral oil, pentahydrosqualene, plant and/or vegetable oil (e.g., soybean oil), hydrocarbon-based oils (isohexadecane), silicone oils (e.g., dimethicone, dimethiconol, and a mixture thereof. A more exhaustive but nonlimiting list of fatty compounds is provided under the heading "Fatty Compounds."

The amount of the one or more fatty compounds in the cosmetic compositions will vary. Nonetheless, in certain embodiments, the total amount of the one or more fatty compounds is from about 1 wt.% to about 20 wt.%, based on the total weight of the cosmetic composition. In further embodiments, the amount of the one or more fatty compounds in the cosmetic compositions is from about 1 to 18 wt.%, about 1 to about 15 wt.%, about 5 to about 20 wt.%, about 5 to about 20 wt.%, about 5 to about 15 wt.%, about 6 to about 20 wt.%, about 6 to about 18 wt.%, about 6 to about 15 wt.%, about 8 to about 20 wt.%, about 8 to about 18 wt.%, or about 8 to about 15 wt.%, based on the total weight of the cosmetic composition.

Nonlimiting examples of thickening polymers include taurate copolymers, polyacrylates, polyacrylamides, etc., e.g., polyacrylate crosspolymer-6, sodium polyacrylate, polyacrylamide. Nonlimiting examples of taurate copolymers include acrylamide/sodium acryloyl dimethyl taurate copolymer, hydroxyethyl acrylate/sodium acryloyl dimethyl taurate copolymer, ammonium acryloyldimethyl taurate/VP copolymer, sodium acrylate/sodium acryloyl dimethyl taurate copolymer, hydroxyethyl acrylate/ sodium acryloyldimethyl taurate copolymer, and mixtures thereof. A more exhaustive but nonlimiting list of thickening polymers is provided under the heading "Thickening Polymers."

The amount of the one or more thickening polymers will vary depending on the type of thickening polymers used; and depending on the desired viscosity of the cosmetic composition. Therefore, in an embodiment, the total amount of the one or more thickening agents is sufficient to achieve the viscosities set forth throughout the instant disclosure. Nonetheless, in various embodiments, the total amount of the one or more thickening agents is from about 0.1 to about 8 wt.%, based on the total weight of the cosmetic composition. In further embodiments, the total amount of the one or more thickening agents is from about 0.1 to about 6 wt.%, about 0.1 to about 5 wt.%, about 0.1 to about 4 wt.%, about 0.1 to about 3 wt.%, about 0.5 to about 8 wt.%, about 0.5 to about 6 wt.%, about 0.5 to about 5 wt.%, about 0.5 to about 4 wt.%, about 0.5 to about 3 wt.%, about 1 to about 8 wt.%, about 1 to about 6 wt.%, about 1 to about 5 wt.%, about 1 to about 4 wt.%, about 1 to about 3 wt.%, or about 1 to about 2 wt.%, based on the total weight of the cosmetic composition.

The amount of water in the cosmetic compositions can and will vary depending on the amount of the other components in the cosmetic compositions. In various embodiments, the total amount of water in the compositions is from about 55 to about 85 wt.%, based on the total weight of the cosmetic composition. In further embodiments, the amount of water in the cosmetic composition is from about 55 to about 80 wt.%, about 55 to about 75 wt.%, about 60 to about 85 wt.%, about 60 to about 80 wt.%, about 60 to about 75 wt.%, about 65 to about 85 wt.%, about 65 to about 80 wt.%, about 65 to about 75 wt.%, based on the total weight of the cosmetic composition.

In an embodiment, the cosmetic compositions in the form of oil in water emulsions include one or more water-soluble solvents. Nonlimiting examples of water-soluble solvents include glycerin, $C_2$-$C_5$ mono-alcohols, polyols (polyhydric alcohols), glycols, and a mixture thereof. In an embodiment, the one or more water-soluble solvents are chosen from propylene glycol, butylene glycol, pentylene glycole, dipropylene glycol, ethanol, isopropanol, t-butyl alcohol, and

mixtures thereof. A more exhaustive but nonlimiting list of water-soluble solvents is provided under the heading "Water-Soluble Solvents."

In an embodiment, the cosmetic compositions may optionally include one or more miscellaneous ingredients. Nonlimiting examples of miscellaneous ingredients include miscellaneous emulsifiers/surfactants other than those set forth above, preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, and mixtures thereof. In an embodiment, the cosmetic composition include at least one further skin active agent, for example, madecassoside. A more exhaustive but nonlimiting list of miscellaneous ingredients is provided under the heading "Miscellaneous Ingredients"

Miscellaneous ingredients can be included in the cosmetic composition, for example, in an amount of about 0.01 to about 10 wt.%, based on the total weight of the cosmetic composition. The total amount of the one or more miscellaneous ingredients may be about 0.01 to about 8 wt.%, about 0.01 to about 5 wt.%, about 0.01 to about 3 wt.%, about 0.1 to about 10 wt.%, about 0.1 to about 8 wt.%, about 0.1 to about 5 wt.%, about 0.1 to about 3 wt.%, about 1 to about 10 wt.%, about 1 to about 8 wt.%, about 1 to about 5 wt.%, or about 1 to about 3 wt.%, based on the total weight of the cosmetic composition.

In an embodiment, the cosmetic compositions in the form of oil in water emulsions include:

(a) about 0.05 to about 6 wt.% of retinol;

(b) about 1 to about 8 wt.% of a plurality of nonionic emulsifiers comprising:

(i) one or more ethoxylated fatty acids; and

(ii) one or more nonionic emulsifiers having a Hydrophile-Lipophile Balance (HLB) from about 9 to about 12;

(iii) optionally one or more glyceryl esters;

(iv) optionally, one or more additional nonionic emulsifiers;

(c) about 0.1 to about 8 wt.% of one or more fatty alcohols;

(d) about 1 to about 20 wt.% of one or more fatty compounds;

(e) one or more thickening polymers; and

(f) water;
wherein the composition is in the form of an oil in water emulsion and all percentages by weight are based on the total weight of the cosmetic composition.

In certain embodiments, the cosmetic composition comprises or consists of:

(a) about 0.05 to about 6 wt.%, preferably from about 0.1 to about 5 wt.%, more preferably about 0.5 to about 3 wt.% of retinol;

(b) about 1 to about 8 wt.%, preferably about 1.3 to about 8 wt.%, more preferably about 1.3 to about 6 wt.%, even more preferably about 1.3 to about 5 wt.% of a plurality of nonionic emulsifiers comprising:

(i) one or more ethoxylated fatty acids, for example, adducts of ethylene oxide with esters of lauric acid, palmitic acid, stearic acid or behenic acid, and mixtures thereof, having from 9 to 100 oxyethylene groups;

(ii) one or more nonionic emulsifiers having a Hydrophile-Lipophile Balance (HLB) from about 9 to about 12, for example, chosen from: (1) polyglyceryl fatty acid esters, polyoxyalkylenated alkyl glycerides, and polyoxyalkylenated fatty ethers; (2) mixed esters of fatty acid or of fatty alcohol, of carboxylic acid and of glycerol; (3) fatty acid esters of sugars and fatty alcohol ethers of sugars; (4) fatty esters of sorbitan and oxyalkylenated fatty esters of sorbitan; and mixtures thereof;

(iii) optionally, one or more glyceryl esters, for example, glyceryl behenate, glyceryl erucate, glyceryl hydroxystearate, glyceryl isostearate, glyceryl lanolate, glyceryl laurate, glyceryl myristate, glyceryl palmitate lactate,

glyceryl sesquioleate, glyceryl stearate, glyceryl distearate, glyceryl laurate, or a mixture thereof. In at least one instance the glyceryl ester comprises glyceryl stearate, glyceryl ricinoleate, and mixtures thereof;

(iv) optionally, one or more additional nonionic emulsifiers;

(c) about 0.1 to about 8 wt.%, preferably about 0.5 to about 6 wt.%, more preferably about 1 to about 5 wt.% of one or more fatty alcohols, for example, fatty alcohols chosen from fatty alcohols having from 8 to 24 carbon atoms, in particular, C14-24 alcohols, cetyl alcohol, stearyl alcohol, cetearyl alcohol, behenyl alcohol, lauryl alcohol, myristic or myristyl alcohol, arachidyl alcohol, lignoceryl alcohol, and mixtures thereof;

(d) about 1 to about 20 wt.%, preferably about 5 to about 18 wt.%, more preferably about 5 to about 15 wt.% of one or more fatty compounds, for example, chosen from fatty esters (e.g., isononyl isononanoate, diisopropyl sebacate), polyolefins (petrolatum), waxes, squalane, squalene, hydrogenated polyisobutene, hydrogenated polydecene, polybutene, mineral oil, pentahydrosqualene, plant and/or vegetable oil (e.g., soybean oil), hydrocarbon-based oils (e.g., isohexadecane), silicone oils, and a mixture thereof;

(e) about 0.1 to about 8 wt.%, preferably about 0.5 to about 5 wt.%, more preferably about 1 to about 3 wt.% of one or more thickening polymers, preferably one or more taurate copolymers, in particular, one or more taurate copolymers chosen from acrylamide/sodium acryloyl dimethyl taurate copolymer, hydroxyethyl acrylate/sodium acryloyl dimethyl taurate copolymer, ammonium acryloyldimethyl taurateNP copolymer, sodium acrylate/sodium acryloyl dimethyl taurate copolymer, hydroxyethyl acrylate/ sodium acryloyldimethyl taurate copolymer, and a mixture thereof; and

(f) about 55 to about 85 wt.%, preferably about 60 to about 80 wt.%, more preferably about 65 to about 75 wt.% of water;

(g) optionally, one or more water-soluble solvents, for example, one or more water-soluble solvents chosen glycerin, mono-alcohols (for example, C1-5 mono-alcohols), organic solvents, polyols (polyhydric alcohols), glycols (e.g., propylene glycol, butylene glycol, pentylene glycol, etc.), and a mixture thereof, preferably glycerin, one or more monoalcohols chosen from ethanol, isopropanol, and t-butyl alcohol, and one or more glycols chosen from propylene glycol, butylene glycol, and pentylene glycol, wherein if present, the amount of the one or more water-soluble solvents is from about 1 to about 20 wt.%, preferably about 5 to about 18 wt.%, more preferably about 5 to about 15 wt.%;

(h) optionally, one or more miscellaneous ingredients, for example, one or more miscellaneous ingredients chosen from miscellaneous emulsifiers/surfactants other than the nonionic emulsifiers of plurality of nonionic emulsifiers of (b), preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, wherein if present, the one or more miscellaneous ingredients comprise about 0.01 to about 0.1 to about 10 wt.%, preferably about 0.1 to about 8 wt.%, more preferably about 1 to about 8 wt.% of the cosmetic composition;
wherein the composition is in the form of an oil in water emulsion and all percentages by weight are based on the total weight of the cosmetic composition.

In certain further embodiments, the plurality of nonionic emulsifiers (b) may comprise or consist of:

(i) about 0.1 to about 5 wt.%, preferably about 0.1 to about 3 wt.%, more preferably about 0.2 to about 2 wt.% of one or more ethoxylated fatty acids, for example, adducts of ethylene oxide with esters of lauric acid, palmitic acid, stearic acid or behenic acid, and mixtures thereof, having from 9 to 100 oxyethylene groups;

(ii) about 0.1 to about 5 wt.%, preferably about 0.1 to about 3 wt.%, more preferably about 0.2 to about 2 wt.% of one or more nonionic emulsifiers having a Hydrophile-Lipophile Balance (HLB) from about 9 to about 12, for example, chosen from: (1) polyglyceryl fatty acid esters, polyoxyalkylenated alkyl glycerides, and polyoxyalkylenated fatty ethers; (2) mixed esters of fatty acid or of fatty alcohol, of carboxylic acid and of glycerol; (3) fatty acid esters of sugars and fatty alcohol ethers of sugars; (4) fatty esters of sorbitan and oxyalkylenated fatty esters of sorbitan; and mixtures thereof;

(iii) optionally, about 0.1 to about 5 wt.%, preferably about 0.1 to about 3 wt.%, more preferably about 0.2 to about 2 wt.% of one or more glyceryl esters, for example, glyceryl behenate, glyceryl erucate, glyceryl hydroxystearate, glyceryl isostearate, glyceryl laurate, glyceryl linoleate, glyceryl myristate, glyceryl palmitate lactate, glyceryl stearate,

glyceryl distearate, glyceryl laurate, or a mixture thereof, preferably chosen from glyceryl stearate, glyceryl ricinoleate, and mixtures thereof;

(iv) optionally, one or more additional nonionic emulsifiers, wherein if present, the one or more additional nonionic emulsifiers may be in an amount of about 0.01 to about 5 wt.%, preferably about 0.01 to about 3 wt.%,

wherein all percentages by weight are based on the total weight of the cosmetic composition (not the total weight of the plurality of nonionic surfactants).

In certain embodiments, the cosmetic composition comprises or consists of:

(a) about 0.05 to about 6 wt.%, preferably from about 0.1 to about 5 wt.%, more preferably about 0.5 to about 3 wt.% of retinol;

(b) about 1 to about 8 wt.%, preferably about 1.3 to about 8 wt.%, more preferably about 1.3 to about 6 wt.%, even more preferably about 1.3 to about 5 wt.% of a plurality of nonionic emulsifiers comprising:

(ii) about 0.1 to about 5 wt.%, preferably about 0.1 to about 3 wt.%, more preferably about 0.2 to about 2 wt.% of one or more ethoxylated fatty acids, for example, adducts of ethylene oxide with esters of lauric acid, palmitic acid, stearic acid or behenic acid, and mixtures thereof, having from 9 to 100 oxyethylene groups;

(ii) about 0.1 to about 5 wt.%, preferably about 0.1 to about 3 wt.%, more preferably about 0.2 to about 2 wt.% of one or more nonionic emulsifiers having a Hydrophile-Lipophile Balance (HLB) from about 9 to about 12, for example, chosen from: (1) polyglyceryl fatty acid esters, polyoxyalkylenated alkyl glycerides, and polyoxyalkylenated fatty ethers; (2) mixed esters of fatty acid or of fatty alcohol, of carboxylic acid and of glycerol; (3) fatty acid esters of sugars and fatty alcohol ethers of sugars; (4) fatty esters of sorbitan and oxyalkylenated fatty esters of sorbitan; and mixtures thereof, preferably at least one of the one or more nonionic emulsifiers having a Hydrophile-Lipophile Balance (HLB) from about 9 to about 12 is chosen from alkylpolyglucosides (e.g., C12-20 alkyl glucoside), polyglyceryl-10 laurate, polyglyceryl-2 dipolyhydroxystearate, polyglyceryl-2 polyhydroxystearate, polyglyceryl-3 laurate, polyglyceryl-3 methylglucose distearate, polyglyceryl-3 oleate, polyglyceryl-3 palmitate, polyglyceryl-3 polyricinoleate, polyglyceryl-3 ricinoleate, and mixture sthereof;

(iii) optionally, about 0.1 to about 5 wt.%, preferably about 0.1 to about 3 wt.%, more preferably about 0.2 to about 2 wt.% of one or more glyceryl esters, for example, glyceryl behenate, glyceryl erucate, glyceryl hydroxystearate, glyceryl isostearate, glyceryl laurate, glyceryl myristate, glyceryl palmitate lactate, glyceryl stearate, glyceryl distearate, glyceryl laurate, or a mixture thereof, preferably chosen from glyceryl stearate, glyceryl ricinoleate, and mixtures thereof;

(iv) optionally, one or more additional nonionic emulsifiers, wherein if present, the one or more additional nonionic emulsifiers may be in an amount of about 0.01 to about 5 wt.%, preferably about 0.01 to about 3 wt.%;

(c) about 0.1 to about 8 wt.%, preferably about 0.5 to about 6 wt.%, more preferably about 1 to about 5 wt.% of one or more fatty alcohols, for example, fatty alcohols chosen from fatty alcohols having from 8 to 24 carbon atoms, in particular, C14-24 alcohols, cetyl alcohol, stearyl alcohol, cetearyl alcohol, behenyl alcohol, lauryl alcohol, myristic or myristyl alcohol, arachidyl alcohol, lignoceryl alcohol, and mixtures thereof;

(d) about 1 to about 20 wt.%, preferably about 5 to about 18 wt.%, more preferably about 5 to about 15 wt.% of one or more fatty compounds, for example, chosen from fatty esters (e.g., isononyl isononanoate), polyolefins (petrolatum), waxes, squalane, squalene, hydrogenated polyisobutene, hydrogenated polydecene, polybutene, mineral oil, pentahydrosqualene, plant and/or vegetable oil (e.g., soybean oil), hydrocarbon-based oils (e.g., isohexadecane), silicone oils, and a mixture thereof;

(e) about 0.1 to about 8 wt.%, preferably about 0.5 to about 5 wt.%, more preferably about 1 to about 3 wt.% of one or more thickening polymers, wherein at least one of the one or more thickening polymers is a taurate copolymers chosen from acrylamide/sodium acryloyl dimethyl taurate copolymer, hydroxyethyl acrylate/sodium acryloyl dimethyl taurate copolymer, ammonium acryloyldimethyl taurateNP copolymer, sodium acrylate/sodium acryloyl dimethyl taurate copolymer, hydroxyethyl acrylate/ sodium acryloyldimethyl taurate copolymer, and a mixture thereof; and

(f) about 55 to about 85 wt.%, preferably about 60 to about 80 wt.%, more preferably about 65 to about 75 wt.% of

water;

(e) optionally, one or more water-soluble solvents, for example, one or more water-soluble solvents chosen glycerin, mono-alcohols (for example, C1-5 mono-alcohols), organic solvents, polyols (polyhydric alcohols), glycols (e.g., propylene glycol, butylene glycol, pentylene glycol, etc.), and a mixture thereof, preferably glyceryn, one or more monoalcohols chosen from ethanol, isopropanol, and t-butyl alcohol, and one or more glycols chosen from propylene glycol, butylene glycol, and pentylene glycol, wherein if present, the amount of the one or more water-soluble solvents is preferably about 1 to about 20 wt.%, preferably about 5 to about 18 wt.%, more preferably about 5 to about 15 wt.%;

(h) optionally, one or more miscellaneous ingredients, for example, one or more miscellaneous ingredients chosen from miscellaneous emulsifiers/surfactants other than the nonionic emulsifiers of plurality of nonionic emulsifiers of (b), preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, wherein if present, the one or more miscellaneous ingredients comprise about 0.01 to about 0.1 to about 10 wt.%, preferably about 0.1 to about 8 wt.%, more preferably about 1 to about 8 wt.% of the cosmetic composition;
wherein the composition is in the form of an oil in water emulsion and all percentages by weight are based on the total weight of the cosmetic composition.

The cosmetic compositions are stable, and retinol is preserved. With respect to physical stability, in certain embodiments, the cosmetic compositions do not visually phase separate or form visibly observable particulates for at least 2 weeks, 4 weeks, and/or 8 weeks in storage at 4°C, 25°C, 37°C, and/or 45°C.

In another embodiment, the cosmetic compositions do not visually phase separate or form visibly observable particulates for at least 10 cycles of freeze-thaw testing, wherein the freeze-thaw testing comprises placing the cosmetic composition in a stability chamber and subjecting it to temperature fluctuation at 12-hour intervals, for a first interval of 12 hours at -20°C followed by a second interval of 12 hours at 25°C.

In another embodiment, the viscosity of the cosmetic compositions does not change by more than 20%, 15%, 10%, or 5%, for at least 2 weeks, 4 weeks, and/or 8 weeks in storage at 4°C, 25°C, 37°C, and/or 45°C.

With respect to chemical stability of retinol, in various embodiments, at least 70%, 75%, 80%, 85%, 90%, or 95% of retinol is preserved at 8 weeks when the cosmetic composition is incorporated into a sealed polyethylene terephthalate glycol (PETG) container and stored at 25°C and/or 45°C.

In general, the cosmetic compositions preferably have a viscosity of about 10,000 to about 200,000 Pa.s at 25°C, and shear rate of 1 $s^{-1}$ at 25°C. However, the cosmetic compositions may have a viscosity of about 10,000 to about 200,000 Pa.s, about 10,000 to about 180,000 Pa.s, about 10,000 to about 150,000 Pa.s, about 10,000 to about 120,000 Pa.s, about 15,000 to about 200,000 Pa.s, about 15,000 to about 180,000 Pa.s, about 15,000 to about 150,000 Pa.s, about 15,000 to about 120,000 Pa.s, about 20,000 to about 200,000 Pa.s, about 20,000 to about 180,000 Pa.s, about 20,000 to about 150,000 Pa.s, or about 20,000 to about 120,000 Pa.s at 25°C, and shear rate of 1 $s^{-1}$ at 25°C.

**Example 1**

**(Oil in Water Emulsion with Retinol)**

| | | | | Inventive | | |
|---|---|---|---|---|---|---|
| | | | | **A** | **B** | **C** |
| (a) | | Active | RETINOL | 0.6 | 0.6 | 0.6 |
| (b) | (i) | Ethoxylated Fatty Acid | PEG-40 STEARATE | 0.4 | 0.4 | 0.4 |
| | (ii) | Nonionic Emulsifier (HLB 9-12) | POLYGLYCERYL-3 METHYLGLUCOSE DISTEARATE | 0.5 | 0.5 | 0.5 |
| | | | C12-20 ALKYL GLUCOSIDE | 0.2 | 0.2 | 0.2 |
| | (iii) | Glyceryl Ester | GLYCERYL STEARATE | 0.3 | 0.3 | 0.3 |
| | (iv) | Nonionic Emulsifier | POLYSORBATE 60 | 0.04 | 0.04 | 0.04 |

(continued)

| | | | Inventive | | |
|---|---|---|---|---|---|
| | | | A | B | C |
| (c) | Fatty Alcohol | CETYL ALCOHOL, AND C14-22 ALCOHOLS | 1.1 | 1.1 | 1.1 |
| (d) | Fatty Compounds | GLYCINE SOJA (SOYBEAN) OIL, | 5.4 | 5.4 | 5.4 |
| | | ISONONYL ISONONANOATE, | 3 | 3 | 3 |
| | | ISOHEXADECANE, AND/OR DIISOPROPYL SEBACATE | 3 | 3 | 3 |
| | Silicone Oil | DIMETHICONOL AND/OR DIMETHICONE | 1 | 0.5 | 0.5 |
| (e) | Thickening Polymers | HYDROXYETHYL ACRYLATE/SODIUM ACRYLOYLDIMETHYL TAURATE COPOLYMER AND/OR AMMONIUM ACRYLOYLDIMETHYLT AURATENP COPOLYMER | 1.4 | 1.4 | 1.4 |
| (g) | Water-Soluble Solvent | T-BUTYL ALCOHOL, GLYCERIN, AND/OR PROPYLENE GLYCOL | 12 | 12 | 12 |
| (h) | Miscellaneous emulsifers/surfactants, salts, preservatives, pH adjusters, fragrances, colorants, antioxidants, chelants, and/or extracts, etc. | | ≤4 | ≤ 4 | ≤4 |
| (f) | WATER | | ~ 70 | ~ 70 | ~ 55 |
| | HYDROXYPROPYL TETRAHYDROPYRANTRIOL | | | | 15 |
| **Physically Stable** | | | **Y** | **Y** | **Y** |
| **Chemically Stable (retinol)** | | | **Y** | **Y** | **Y** |

**Example 2**

**(Stability Data)**

Compositions A and B of Example 1 were subjected to physical stability studies and visually evaluated for phase separation and assessed under a microscope for particulate formation. The compositions were analyzed upon initial manufacture of the composition ($T_0$). The compositions were again analyzed after 10 days of freeze-thaw testing. For freeze-thaw testing, the compositions were placed in a stability chamber and subjected to temperature fluctuation at 12-hour intervals. For 12 hours, the compositions were held at -20°C. For the next 12 hours, the compositions were held at 25°C. The cycle was repeated 10 times (for 10 days). Separately, the compositions of Example 1 were evaluated after 4 weeks (1 month) in storage at 4°C, 25°C, 37°C, and 45°C and again at 8 weeks (2 months) at 4°C, 25°C, 37°C, and 45°C and visually evaluated for phase separation and assessed under a microscope for particulate formation.

The inventive compositions were deemed stable ("Y") (yes) because they did not visually phase separate and did not form particulates.

Chemical stability was also evaluated, in particular, the chemical stability of retinol in the cosmetic composition (resistance to degradation). The compositions of Example 1 were individually added into sealed polyethylene terephthalate glycol (PETG) cartridges and stored at 25°C and 45°C and the amount of remaining retinol determined at 8 weeks. The amount of retinol was determined by HPLC coupled with UV-Vis. A calibration curve was prepared to quantify the area under the curve for each sample

| | % Retinol at 8 Weeks | |
|---|---|---|
| | **25°C** | **45°C** |
| **Composition A** | 77% | 89% |
| **Composition B** | 81% | 78% |

***Oil in Water Emulsions with Hydroxypropyl Tetrahydropyrantriol & optionally 4-T-Butylcyclohexanol***

In an embodiment, the cosmetic compositions in the form of oil in water emulsions include hydroxypropyl tetrahydropyrantriol, and optionally, 4-T-butylcyclohexanol. The amount of hydroxypropyl tetrahydropyrantriol in the cosmetic compositions will vary but in certain embodiments it is from about 10 wt.% to about 40 wt.% based on the total weight of the composition. In further embodiments, the amount of hydroxypropyl tetrahydropyrantriol in the composition is from about 10 wt.% to about 35 wt.%, from about 10 to about 30 wt.%, from about 10 to about 25 wt.%, from about 10 to about 20 wt.%, about 12 to about 35 wt.%, about 12 to about 30 wt.%, about 12 to about 25 wt.%, about 12 to about 20 wt.%, from about 12 to about 18 wt.%, from about 14 to about 30 wt.%, from about 14 to about 25 wt.%, from about 14 to about 20 wt.%, or from about 14 to about 18 wt.%, based on the total weight of the composition.

In an embodiment, the cosmetic composition in the form of an oil in water emulsion includes:

(a) hydroxypropyl tetrahydropyrantriol, preferably in amounts set forth above;

(b) water;

(c) one or more nonionic emulsifiers chosen from glyceryl esters having an HLB of about 3 to about 8;

(d) one or more nonionic emulsifiers having an HLB of about 16 to about 18;

(e) one or more nonionic emulsifiers having an HLB of about 9 to about 15;

(f) one or more fatty alcohols;

(g) one or more fatty compounds; and

(h) one or more thickening polymers.
wherein all percentages by weight are based on the total weight of the cosmetic composition.

The amount of water in the cosmetic compositions will vary depending on the amount of the other components in the cosmetic compositions. In general, the amount of water in the composition is from about 30 to about 85 wt.%, based on the total weight of the cosmetic composition. In certain embodiments, the amount of water in the cosmetic composition is from about 30 to about 80 wt.%, about 30 to about 70 wt.%, about 30 to about 60 wt.%, about 30 to about 50 wt.%, about 40 to about 80 wt.%, about 40 to about 70 wt.%, about 40 to about 60 wt.%, about 40 to about 50 wt.%, about 50 to about 80 wt.%, about 50 to about 75 wt.%, about 50 to about 70 wt.%, about 55 to about 85 wt.%, about 55 to about 80 wt.%, about 55 to about 75 wt.%, about 55 to about 70 wt.%, about 60 to about 85 wt.%, about 60 to about 80 wt.%, about 60 to about 75 wt.%, or about 60 to about 70 wt.%, based on the total weight of the cosmetic composition.

Nonlimiting examples of nonionic emulsifiers that are glyceryl esters having an HLB of about 3 to about 8 include glyceryl behenate, glyceryl erucate, glyceryl hydroxystearate, glyceryl lanolate, glyceryl laurate, glyceryl linoleate, glyceryl myristate, glyceryl palmitate lactate, glyceryl stearate, glyceryl distearate, or a mixture thereof. preferred glyceryl esters include glyceryl stearate, glyceryl ricinoleate, or a mixture thereof.

In various embodiments, the one or more glyceryl esters having an HLB of about 3 to about 8 are chosen from glyceryl esters that are solid at a temperature of below 30°C.

The total amount of the one or more nonionic emulsifiers that are glyceryl esters having an HLB of about 3 to about 8 can vary. In certain embodiments, the total amount of the one or more nonionic emulsifier that are glyceryl esters having an HLB of about 3 to about 8 is from 0.1 wt.% to about 5 wt.% based on the total weight of the cosmetic composition. In further embodiments, the amount of the nonionic emulsifier chosen from one or more glyceryl esters having an HLB of about 3 to about 8 is from about 0.1 to about 4 wt.%, from about 0.1 to about 3 wt%, from about 0.1 to about 2 wt%, about 0.2 wt.% to about 5 wt.%, about 0.2 to about 4 wt.%, from about 0.2 to about 3 wt%, from about 0.2 to about 2 wt%, about 0.3 wt.% to about 5 wt.%, about 0.3 to about 4 wt.%, from about 0.3 to about 3 wt%, from about 0.3 to about 2 wt%, about 0.5 wt.% to about 5 wt.%, about 0.5 to about 4 wt.%, from about 0.5 to about 3 wt%, from about 0.5 to about 2 wt%, based on the total weight of the cosmetic composition.

Nonlimiting examples of nonionic emulsifiers having an HLB of about 16 to about 18 include ethoxylated emulsifiers, for example, ethoxylated fatty acids, ethoxylated sorbitan fatty esters, and mixtures thereof. Ethoxylated fatty acids are particularly preferred. A more exhaustive but nonlimiting list of nonionic emulsifiers having an HLB of about 16 to 18 is provided under the heading "Nonionic Emulsifiers having an HLB of about 16 to about 18."

The total amount of the one or more nonionic emulsifiers having an HLB of about 16 to about 18 will vary. Nonetheless, in certain embodiments, the total amount of the one or more nonionic emulsifiers having an HLB of about 16

to about 18 is from about 0.1 to about 5 wt.%, based on the total weight of the cosmetic composition. In further embodiments, the amount of the one or more nonionic emulsifiers having an HLB of about 16 to about 18 range from about 0.1 to about 4 wt%, from about 0.1 to about 3 wt%, from about 0.1 to about 2 wt%, about 0.2 wt.% to about 5 wt.%, about 0.2 to about 4 wt.%, from about 0.2 to about 3 wt%, from about 0.2 to about 2 wt%, about 0.3 wt.% to about 5 wt.%, about 0.3 to about 4 wt.%, from about 0.3 to about 3 wt%, from about 0.3 to about 2 wt%, about 0.5 wt.% to about 5 wt.%, about 0.5 to about 4 wt.%, from about 0.5 to about 3 wt%, from about 0.5 to about 2 wt%, based on the total weight of the cosmetic composition.

Nonlimiting examples of nonionic emulsifiers having an HLB of about 9 to about 15 include alkylpolyglucosides (cetearyl glucoside), polyglycerol-based emulsifiers (polygyceryl-3 methylglucose distearate), sorbitan fatty esters (polysorbate 60), sugar esters or ethers, sugar-based esters or ethers, polyol fatty esters or ethers, glyceryl fatty esters or ethers, ethoxylates thereof, or mixtures thereof. A more exhaustive but nonlimiting list of nonionic emulsifiers having an HLB of about 9 to about 15 are provided under the heading, "Nonionic Emulsifiers having an HLB of about 9 to about 15."

The total amount of the one or more nonionic emulsifiers having an HLB of about 9 to about 15 will vary. Nonetheless, in various embodiments, the amount of the one or more nonionic emulsifiers having an HLB of about 9 to about 15 is from about 0.1 to about 5 wt.%, based on the total weight of the cosmetic composition. In further embodiments, the one or more nonionic emulsifiers having an HLB of about 9 to about 15 is from about 0.1 to about 4 wt%, from about 0.1 to about 3 wt%, from about 0.1 to about 2 wt%, about 0.3 to about 5 wt.%, about 0.3 to about 4 wt.%, about 0.3 to about 2 wt.%, about 0.5 to about 5 wt.%, about 0.5 to about 4 wt.%, about 0.5 to about 3 wt.%, or about 0.5 to about 2 wt.%, about 1.0 to about 5 wt.%, about 1.0 to about 4 wt.%, about 1.0 to about 3 wt.%, or about 1.0 to about 2 wt.%, based on the total weight of the cosmetic composition.

Nonlimiting examples of fatty alcohols include fatty alcohols having from 8 to 24 carbon atoms, in particular, cetyl alcohol, stearyl alcohol, cetearyl alcohol, behenyl alcohol, lauryl alcohol, myristic or myristyl alcohol, arachidyl alcohol, and mixtures thereof. A more exhaustive but nonlimiting list of fatty alcohols is provided under the heading "Fatty Alcohols."

The total amount of the one or more fatty alcohols will vary. Nonetheless, in various embodiments, the total amount of the one or more fatty alcohols in the cosmetic compositions is from 1 wt.% to about 10 wt.%, based on the total weight of the composition. In further embodiments, the total amount of one or more fatty alcohols in the composition ranges from about 1 to about 8 wt.%, about 1 to about 5 wt.%, about 1 to about 4 wt.%, about 1 to about 3 wt.%, based on the total weight of the composition.

Nonlimiting examples of fatty compounds include fatty esters (e.g., isononyl isononanoate), polyolefins (petrolatum), waxes, squalane, squalene, hydrogenated polyisobutene, hydrogenated polydecene, polybutene, mineral oil, pentahydrosqualene, plant and/or vegetable oil (e.g., soybean oil), hydrocarbon-based oils (e.g., isohexadecane), and a mixture thereof. A more exhaustive but nonlimiting list of fatty compounds is provided under the heading "Fatty Compounds."

The total amount of the one or more fatty compounds in the cosmetic compositions will vary. Nonetheless, in certain embodiments, the total amount of the one or more fatty compounds is from about 5 wt.% to about 20 wt.%, based on the total weight of the cosmetic composition. In further embodiments, the total amount of the one or more fatty compounds in the cosmetic compositions is from about 5 to 15 wt.%, about 5 to about 12 wt.%, about 6 to about 20 wt.%, about 6 to about 15 wt.%, about 6 to about 12 wt.%, based on the total weight of the cosmetic composition.

Useful thickening polymers include, among others, taurate copolymers. Nonlimiting examples of taurate copolymers include acrylamide/sodium acryloyl dimethyl taurate copolymer, hydroxyethyl acrylate/sodium acryloyl dimethyl taurate copolymer, ammonium acryloyldimethyl taurateNP copolymer, sodium acrylate/sodium acryloyl dimethyl taurate copolymer, hydroxyethyl acrylate/ sodium acryloyldimethyl taurate copolymer, and mixtures thereof. A more exhaustive but nonlimiting list of thickening polymers is provided under the heading "Thickening Polymers."

Generally, the amount of the one or more thickening polymers will vary depending on the type of thickening polymers used; and depending on the desired viscosity of the cosmetic composition. Therefore, in an embodiment, the total amount of the one or more thickening agents is sufficient to achieve the viscosities set forth throughout the instant disclosure. Nonetheless, in various embodiments, the total amount of the one or more thickening polymers may range from about 1 to about 8 wt.%, based on the total weight of the cosmetic composition. In various embodiments, the amount of the one or more thickening polymers may range from about 1 to about 6 wt%, from about 1 to about 5 wt%, from about 1 to about 3 wt%, based on the total weight of the cosmetic composition.

Silicones can optionally be included in the cosmetic compositions but in some embodiments the compositions are free or essentially free from silicones. Silicones are synthetic polymers made up of repeating units of siloxane, elemental silicon and oxygen, combined with other elements, most often carbon and hydrogen. Thus, silicones are also called polysiloxanes. In some instances, the cosmetic compositions of the instant case are free or essentially free from silicones, such as dimethicones, amomdimethicones, dimethiconols, cyclosiloxanes, siloxanes, etc. In another embodiments, the compositions include one or more silicones.

The total amount of the one or more silicones in the cosmetic composition, if present, will vary. Nonetheless, in certain embodiments, the total amount of the one or more silicones is from about 0.01 to about 15 wt.%, about 0.01 to about

10 wt.%, about 0.01 to about 5 wt.%, about 0.01 to about 3 wt.%, about 0.01 to about 2 wt.%, about 0.1 to about 15 wt.%, about 0.1 to about 10 wt.%, about 0.1 to about 5 wt.%, about 0.1 to about 3 wt.%, about 0.1 to about 2 wt.%, based on the total weight of the cosmetic composition.

The cosmetic compositions do not require, nor do they necessarily include 4-tert-butylcyclohexanol - it is optional. Nonetheless, in certain embodiments it is preferably to include 4-tert-butylcyclohexanol. 4-tert-butylcyclohexanol may be in a cis configuration, a trans configuration, or a mixture of cis and trans configurations. Among other things, 4-tert-butylcyclohexanol is useful for reducing skin irritation, for soothing the skin, and/or for reducing or alleviating stinging, burning, and tightness.

The total amount of the 4-tert-butylcyclohexanol will vary. Nonetheless, in certain embodiments, the total amount of the 4-tert-butylcyclohexanol in the cosmetic composition is from about 0.1 wt.% to about 5 wt.% based on the total weight of the cosmetic composition. In further embodiments, the total amount of the 4-tert-butylcyclohexanol is from about from about 0.1 to about 4 wt%, about 0.1 to about 3 wt.%, about 0.1 to about 2 wt.%, about 0.5 to about 5 wt.%, about 0.5 to about 4 wt.%, about 0.5 to about 3 wt.%, about 0.5 to about 2 wt.%, about 1 to about 5 wt.%, about 1 to about 4 wt.%, about 1 to about 3 wt.%, or about 1 to about 2 wt.%, based on the total weight of the cosmetic composition.

In certain embodiments, the cosmetic compositions include one or more water-soluble solvents. Nonlimiting examples of water-soluble solvents include glycerin, mono-alcohols, polyols (polyhydric alcohols), glycols, and a mixture thereof. In further embodiments, the one or more water-soluble solvents may be chosen from propylene glycol, butylene glycol, pentylene glycole, dipropylene glycol, ethanol, isopropanol, t-butyl alcohol, and a mixture thereof. A more exhaustive but nonlimiting list of useful water-soluble solvents are provided under the heading "Water-Soluble Solvents."

The total amount of the one or more water-soluble solvents will vary. Nonetheless, in certain embodiments, the total amount of the one or more water-soluble is from about 0.1 to about 20 wt.%, based on the total weight of the cosmetic composition. In further embodiments, the total amount of the one or more water-soluble solvents is about 0.1 to about 20 wt.%, about 0.1 to about 15 wt.%, about 0.1 to about 10 wt.%, about 0.1 to about 5 wt.%, from about 0.1 wt% to about 1 wt%, about 2 to about 20 wt.%, about 2 to about 15 wt.%, about 2 to about 10 wt.%, about 2 to about 5 wt.%, about 5 to about 20 wt.%, about 5 to about 15 wt.%, or about 5 to about 10 wt.%, based on the total weight of the cosmetic composition.

In an embodiment, the cosmetic compositions include one or more miscellaneous ingredients. Nonlimiting examples of miscellaneous ingredients include miscellaneous emulsifiers/surfactants other than the dimethicone copolyol emulsifiers of (d), preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, and mixtures thereof. In an embodiment, the cosmetic composition includes at least one further skin active agent, for example, madecassoside. A more exhaustive but nonlimiting list of miscellaneous ingredients is provided under the heading "Miscellaneous Ingredients"

Miscellaneous ingredients, when present, can be in an amount from about 0.01 to about 10 wt.%, based on the total weight of the cosmetic composition. The total amount of the one or more miscellaneous ingredients may be about 0.01 to about 8 wt.%, about 0.01 to about 5 wt.%, about 0.01 to about 3 wt.%, about 0.1 to about 10 wt.%, about 0.1 to about 8 wt.%, about 0.1 to about 5 wt.%, about 0.1 to about 3 wt.%, about 1 to about 10 wt.%, about 1 to about 8 wt.%, about 1 to about 5 wt.%, or about 1 to about 3 wt.%, based on the total weight of the cosmetic composition.

In an embodiment, the compositions in the form of oil in water emulsions include:

(a) about 10 to about 40 wt.% of hydroxypropyl tetrahydropyrantriol;

(b) water;

(c) about 0.1 to about 5 wt.% of nonionic emulsifier chosen from glyceryl esters having an HLB of about 3 to about 8;

(d) about 0.1 to about 5 wt.% of one or more nonionic emulsifiers having an HLB of about 16 to about 18;

(e) about 0.1 to about 5 wt.% of one or more nonionic emulsifiers having an HLB of about 9 to about 15;

(f) about 1 to about 10 wt.% of one or more fatty alcohols;

(g) about 5 to about 20 wt.% of one or more fatty compounds; and

(h) one or more thickening polymers;
wherein the composition is in the form of an oil in water emulsion, preferably a gel emulsion, and all percentages by weight are based on the total weight of the cosmetic composition.

A gel emulsion is an oil in water emulsion, wherein the aqueous phase is a gel and the oil droplets/particulates are dispersed throughout the gel matrix.

In certain embodiments, the cosmetic composition in the form of an oil in water emulsion comprises or consists of:

(a) about 10 to about 40 wt.%, preferably about 10 to about 20 wt.%, more preferably about 12 to about 18 wt.% of hydroxypropyl tetrahydropyrantriol;

(b) about 50 to about 85 wt.%, preferably about 55 to about 80 wt.%, more preferably about 55 to about 75 wt.% of water;

(c) about 0.1 to about 5 wt.%, preferably about 0.1 to about 4, more preferably about 0.2 to about 3 wt.% of one or more nonionic emulsifiers chosen from glyceryl esters having an HLB of about 3 to about 8, for example, glyceryl esters chosen from, glyceryl behenate, glyceryl erucate, glyceryl hydroxystearate, glyceryl lanolate, glyceryl laurate, glyceryl myristate, glyceryl palmitate lactate, glyceryl stearate, glyceryl distearate, glyceryl laurate, and a mixture thereof, preferably one or more glyceryl esters chosen from glyceryl stearate, glyceryl ricinoleate, and a mixture thereof;

(d) about 0.1 to about 5 wt.%, preferably about 0.1 to about 4, more preferably about 0.2 to about 3 wt.% of one or more nonionic emulsifiers having an HLB of about 16 to about 18, preferably one or more ethoxylated emulsifiers chosen from ethoxylated fatty acids, ethoxylated sorbitan fatty esters, and a mixture thereof, preferably one or more ethoxylated fatty acids;

(e) about 0.1 to about 5 wt.%, preferably about 0.5 to about 5 wt.%, more preferably about 0.5 to about 4 wt.% of one or more nonionic emulsifiers having an HLB of about 9 to about 15, for example, chosen from alkylpolyglucosides (cetearyl glucoside), polyglycerol-based emulsifiers (polygyceryl-3 methylglucose distearate), sorbitan fatty esters (polysorbate 60), sugar esters or ethers, sugar-based esters or ethers, polyol fatty esters or ethers, glyceryl fatty esters or ethers, ethoxylates thereof, or mixtures thereof, preferably chosen from sugar esters or ethers and sugar-based esters or ethers;

(f) about 1 to about 10 wt.%, preferably about 1 to about 8 wt.%, more preferably about 1 to about 6 wt.% of one or more fatty alcohols, preferably chosen from fatty alcohols having from 8 to 24 carbon atoms, preferably chosen from cetyl alcohol, stearyl alcohol, cetearyl alcohol, behenyl alcohol, lauryl alcohol, myristic or myristyl alcohol, arachidyl alcohol, lignoceryl alcohol, and mixtures thereof;

(g) about 5 to about 20 wt.%, preferably about 5 to about 15 wt.%, more preferably about 6 to about 12 wt.% of one or more fatty compounds, for example, chosen from fatty esters (e.g., isononyl isononanoate), polyolefins (petrolatum), waxes, squalane, squalene, hydrogenated polyisobutene, hydrogenated polydecene, polybutene, mineral oil, pentahydrosqualene, plant and/or vegetable oil (e.g., soybean oil), hydrocarbon-based oils (e.g., isohexadecane), and a mixture thereof;

(h) one or more thickening polymers, preferably one or more taurate copolymers, in particular, one or more taurate copolymers chosen from acrylamide/sodium acryloyl dimethyl taurate copolymer, hydroxyethyl acrylate/sodium acryloyl dimethyl taurate copolymer, ammonium acryloyldimethyl taurateNP copolymer, sodium acrylate/sodium acryloyl dimethyl taurate copolymer, hydroxyethyl acrylate/ sodium acryloyldimethyl taurate copolymer, and a mixture thereof, wherein the amount of the one or more thickening polymers may optional be from about 1 to about 8 wt.%, preferably about 1 to about 5 wt.%, more preferably about 1 to about 3 wt.%;

(i) optionally, 4-tert-butylcyclohexanol, wherein if present is preferably in an amount of about 0.1 to about 5 wt.%, preferably about 0.5 to about 4 wt.%, more preferably, 0.5 to about 3wt.%;

(j) optionally, one or more water-soluble solvents, for example, one or more water-soluble solvents chosen glycerin, alcohols (for example, C1-30, C1-15, C1-10, or C1-4 alcohols), organic solvents, polyols (polyhydric alcohols, e.g., ethanol, isopropanol, t-butyl alcohol, etc.), glycols (e.g., propylene glycol, butylene glycol, pentylene glycol, etc.), and a mixture thereof, preferably one or more monoalcohols chosen from ethanol, isopropanol, and t-butyl alcohol, and one or more glycols chosen from propylene glycol, butylene glycol, and pentylene glycol, wherein if present, the one or more water-soluble solvents comprise about 0.1 to about 20 wt.%, preferably about 0.1 to about 15 wt.%, more preferably about 1 to about 15 wt.% of the cosmetic composition;

(k) optionally, one or more silicones, for example, dimethicone, dimethiconol, cyclomethicone, polysilicone-11, phenyl trimethicone, and amodimethicone, preferably dimethicone, wherein if present, the one or more silicones may be in an amount of about 0.01 to about 10 wt.%, about 0.1 to about 5 wt.%, more preferably about 0.1 to about 3 wt.%;

(l) optionally, one or more miscellaneous ingredients, for example, one or more miscellaneous ingredients chosen from miscellaneous emulsifiers/surfactants, preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, wherein if present, the one or more miscellaneous ingredients comprise about 0.01 to about 0.1 to about 10 wt.%, preferably about 0.1 to about 8 wt.%, more preferably about 1 to about 8 wt.% of the cosmetic composition

wherein the composition is an oil in water emulsion, preferably a gel emulsion, and all percentages by weight are based on the total weight of the cosmetic composition.

In further embodiments, the cosmetic compositions in the form of oil in water emulsions comprises or consists of:

(a) about 10 to about 40 wt.%, preferably about 10 to about 20 wt.%, more preferably about 12 to about 18 wt.% of hydroxypropyl tetrahydropyrantriol;

(b) about 50 to about 85 wt.%, preferably about 55 to about 80 wt.%, more preferably about 55 to about 75 wt.% of water;

(c) about 0.1 to about 5 wt.%, preferably about 0.1 to about 4, more preferably about 0.2 to about 3 wt.% of one or more nonionic emulsifiers chosen from glyceryl esters having an HLB of about 3 to about 8, in particular chosen from glyceryl stearate, glyceryl ricinoleate, and a mixture thereof, preferably glyceryl stearate;

(d) about 0.1 to about 5 wt.%, preferably about 0.1 to about 4, more preferably about 0.2 to about 3 wt.% of one or more nonionic emulsifiers having an HLB of about 16 to about 18 chosen from ethoxylated fatty acids, ethoxylated sorbitan fatty esters, and a mixture thereof, preferably one or more ethoxylated fatty acids;

(e) about 0.1 to about 5 wt.%, preferably about 0.5 to about 5 wt.%, more preferably about 0.5 to about 4 wt.% of one or more nonionic emulsifiers having an HLB of about 9 to about 15, for example, chosen from alkylpolyglucosides (cetearyl glucoside), polyglycerol-based emulsifiers (polygyceryl-3 methylglucose distearate), sorbitan fatty esters (polysorbate 60), sugar esters or ethers, sugar-based esters or ethers, polyol fatty esters or ethers, glyceryl fatty esters or ethers, ethoxylates thereof, or mixtures thereof, preferably chosen from sugar esters or ethers and sugar-based esters or ethers;

(f) about 1 to about 10 wt.%, preferably about 1 to about 8 wt.%, more preferably about 1 to about 6 wt.% of one or more fatty alcohols having from 8 to 24 carbon atoms, preferably chosen from cetyl alcohol, cetearyl alcohol, behenyl alcohol, and mixtures thereof;

(g) about 5 to about 20 wt.%, preferably about 5 to about 15 wt.%, more preferably about 6 to about 12 wt.% of one or more fatty compounds, for example, chosen from fatty esters (e.g., isononyl isononanoate), polyolefins (petrolatum), waxes, squalane, squalene, hydrogenated polyisobutene, hydrogenated polydecene, polybutene, mineral oil, pentahydrosqualene, plant and/or vegetable oil (e.g., soybean oil), hydrocarbon-based oils (e.g., isohexadecane), and a mixture thereof;

(h) about 1 to about 8 wt.%, preferably about 1 to about 5 wt.%, more preferably about 1 to about 3 wt.% of one or more taurate copolymers, in particular, one or more taurate copolymers chosen from acrylamide/sodium acryloyl dimethyl taurate copolymer, hydroxyethyl acrylate/sodium acryloyl dimethyl taurate copolymer, ammonium acryloyldimethyl taurateNP copolymer, sodium acrylate/sodium acryloyl dimethyl taurate copolymer, hydroxyethyl acrylate/ sodium acryloyldimethyl taurate copolymer, and a mixture thereof, preferably chosen from hydroxyethyl acrylate/ sodium acryloyldimethyl taurate copolymer, ammonium acryloyldimethyl taurateNP copolymer, and a mixture thereof;

(i) optionally, 4-tert-butylcyclohexanol, wherein if present is preferably in an amount of about 0.1 to about 5 wt.%, preferably about 0.5 to about 4 wt.%, more preferably, 0.5 to about 3wt.%;

(j) about 0.1 to about 20 wt.%, preferably about 0.1 to about 15 wt.%, more preferably about 1 to about 5 wt.% of, one or

more water-soluble solvents chosen from one or more monoalcohols (e.g., ethanol, isopropanol, and t-butyl alcohol) one or more glycols (e.g., propylene glycol, butylene glycol, and pentylene glycol), and a mixture thereof;

(k) about 0.01 to about 10 wt.%, about 0.1 to about 5 wt.%, more preferably about 0.1 to about 3 wt.% of one or more silicones, for example, dimethicone, dimethiconol, cyclomethicone, polysilicone-11, phenyl trimethicone, and amodimethicone, preferably dimethicone;

(l) optionally, one or more miscellaneous ingredients, for example, one or more miscellaneous ingredients chosen from miscellaneous emulsifiers/surfactants, preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, wherein if present, the one or more miscellaneous ingredients comprise about 0.01 to about 0.1 to about 10 wt.%, preferably about 0.1 to about 8 wt.%, more preferably about 1 to about 8 wt.% of the cosmetic composition
wherein the composition is an oil in water emulsion, preferably a gel emulsion, and all percentages by weight are based on the total weight of the cosmetic composition.

The composition preferably has a pH of about 4 to about 8, preferably about 5 to about 8, more preferably about 4.5 to about 7.5.

The cosmetic compositions are stable. For example, in an embodiment the cosmetic compositions do not visually phase separate or form visibly observable particulates for at least 2 weeks, 4 weeks, and/or 8 weeks in storage at 4°C, 25°C, 37°C, and/or 45°C.

In another embodiment, the cosmetic compositions do not visually phase separate or form visibly observable particulates for at least 10 cycles of freeze-thaw testing, wherein the freeze-thaw testing comprises placing the cosmetic composition in a stability chamber and subjecting it to temperature fluctuation at 12-hour intervals, for a first interval of 12 hours at -20°C followed by a second interval of 12 hours at 25°C.

In an embodiment, the viscosity of the cosmetic compositions does not change by more than 20%, 15%, 10%, or 5%, for at least 2 weeks, 4 weeks, and/or 8 weeks in storage at 4°C, 25°C, 37°C, and/or 45°C.

In certain embodiments, the cosmetic composition preferably has a viscosity of about 5,000 to about 200,000 Pa.s at 25°C, and shear rate of 1 $s^{-1}$ at 25°C. However, the cosmetic compositions may have a viscosity of about 10,000 to about 200,000 Pa.s, about 10,000 to about 180,000 Pa.s, about 10,000 to about 150,000 Pa.s, about 10,000 to about 120,000 Pa.s, about 15,000 to about 200,000 Pa.s, about 15,000 to about 180,000 Pa.s, about 15,000 to about 150,000 Pa.s, about 15,000 to about 120,000 Pa.s, about 20,000 to about 200,000 Pa.s, about 20,000 to about 180,000 Pa.s, about 20,000 to about 150,000 Pa.s, or about 20,000 to about 120,000 Pa.s at 25°C, and shear rate of 1 $s^{-1}$ at 25°C.

**Example 3**

**(Oil in Water Emulsion with Hydroxypropyl Tetrahydropyrantriol and 4-T-Butylcyclohexanol)**

| | | | A | B | C | D |
|---|---|---|---|---|---|---|
| (a) | Active | HYDROXYPROPYL TETRAHYDROPYRANTRIOL | 15 | 15 | 15 | 15 |
| (b) | Water | WATER | 63.5 | 63.9 | 63.5 | 63.9 |
| (c) | Glyceryl Ester | GLYCERYL STEARATE (HLB 3.8±1) | 0.3 | 0.3 | 0.3 | 0.3 |
| (d) | High HLB Emulsifier | PEG-40 STEARATE (HLB 17.5) | 0.4 | 0.4 | 0.4 | 0.4 |
| (e) | Mid-HLB Emulsifer | POLYGLYCERYL-3 METHYLGLUCOSE DISTEARATE (HLB-12) | 0.5 | 0.5 | 0.5 | 0.5 |
| | | CETEARYL GLUCOSIDE (HLB 11 ±1) | 0.2 | 0.2 | 0.2 | 0.2 |
| | | POLYSORBATE 60 (HLB 14.9±1) | 0.04 | 0.04 | 0.04 | 0.04 |

(continued)

| | | | A | B | C | D |
|---|---|---|---|---|---|---|
| (f) | Fatty Alcohol | CETYL ALCOHOL | 0.3 | 0.3 | 0.8 | 0.8 |
| | | BEHENYL ALCOHOL | 1.0 | 1.0 | 1.0 | 1.0 |
| | | CETEARYL ALCOHOL | 0.8 | 0.8 | 0.3 | 0.3 |
| (g) | Fatty Compound | SQUALANE, ISONONYL ISONONANOATE, AND SOYBEAN OIL | 9 | 9 | 9 | 9 |
| (h) | Thickening Polymers | HYDROXYETHYL ACRYLATE/SODIUM ACRYLOYLDIMETHYL TAURATE COPOLYMER | 0.7 | 0.7 | 0.7 | 0.7 |
| | | AMMONIUM ACRYLOYLDIMETHYLTAURA TENP COPOLYMER | 0.7 | 0.7 | 0.7 | 0.7 |
| | | POLY C10-30 ALKYL ACRYLATE | 0.5 | 0.5 | 0.5 | 0.5 |
| (i) | Active | 4-T-BUTYLCYCLOHEXANOL | 1.1 | 1.1 | 1.1 | 1.1 |
| (j) | Water-Soluble Solvent | PROPYLENE GLYCOL, PENTYLENE GLYCOL, AND T-BUTYL ALCOHOL | 4.2 | 4.2 | 4.2 | 4.2 |
| (k) | Silicone | DIMETHICONE | 0.5 | 0.5 | 0.5 | 0.5 |
| (l) | Miscellaneou s Emulsifiers | SORBITAN ISOSTEARATE (HLB 4.7) | 0.04 | 0.04 | 0.04 | 0.04 |
| | | HYDROGENATED LECITHIN | 0.3 | | 0.3 | |
| | Other miscellaneous emulsifiers/surfactants, Salts, Preservatives, pH Adjusters, Fragrances, Colorants, Chelants, Extracts, Fillers, Absorbants, Additional Skin Actives, etc. | | ≤4 | ≤4 | ≤4 | ≤4 |
| Stability | | | **Yes** | **Yes** | **Yes** | **Yes** |

**Example 4**

**(Comparative Compositions)**

| | | | C-1 | C-2 | C-3 |
|---|---|---|---|---|---|
| (a) | Active | HYDROXYPROPYL TETRAHYDROPYRANTRIOL | 15 | 15 | 15 |
| (b) | Water | WATER | 69.5 | 61.5 | 55.8 |
| (c) | Glyceryl Ester | GLYCERYL STEARATE (HLB = 3.8) | | 1.6 | |
| (d) | High HLB | STEARETH-100 (HLB = 18.8) | 0.4 | | |
| (e) | Mid-HLB Emulsifiers | ARACHIDYL GLUCOSIDE (HLB ~10) | | | |
| | | C12-20 ALKYL GLUCOSIDE (HLB = 10.3) | | | 0.4 |
| | | CETEARYL GLUCOSIDE (HLB = 11) | 0.1 | | |
| | | POLYSORBATE 80 (HLB = 15) | | 0.2 | |
| | | POLYSORBATE 60 (HLB = 14.9) | | 0.04 | |
| (f) | Fatty Alcohols | ARACHIDYL ALCOHOL, BEHENYL ALCOHOL, CETEARYL ALCOHOL, CETYL ALCOHOL, AND/OR C14-22 ALCOHOLS | 3.2 | 1.1 | 4.1 |
| (g) | Fatty Compounds | SQUALANE. SOYBEAN OIL, ISONONYL ISONONANOATE, ISOHEXADECANE, DICAPRYLYL ETHER, AND/OR CAPRYLIC/CAPRIC TRIGLYCERIDE | 2 | 9.5 | 4 |

(continued)

| | | | C-1 | C-2 | C-3 |
|---|---|---|---|---|---|
| (h) | Thickening Polymers | ACRYLAMIDE/SODIUM ACRYLOYLDIMETHYLTAURATE COPOLYMER | | 1 | |
| | | HYDROXYETHYL ACRYLATE/SODIUM ACRYLOYLDIMETHYL TAURATE COPOLYMER | | 0.7 | |
| | | AMMONIUM ACRYLOYLDIMETHYLTAURATENP COPOLYMER | | 1.2 | |
| | | SODIUM POLYACRYLATE | 1.3 | | |
| | | XANTHAN GUM | | | 0.3 |
| | | POLYACRYLATE CROSSPOLYMER-6 | | | 0.8 |
| | | ALUMINUM STARCH OCTENYLSUCCINATE | 1 | | 1 |
| (i) | Active | 4-T-BUTYLCYCLOHEXANOL | 1.1 | 1.1 | 1.1 |
| (j) | Water-Soluble Solvent | T-BUTYL ALCOHOL, CAPRYLYL GLYCOL, PENTYLENE GLYCOL, AND/OR PROPYLENE GLYCOL | 4.5 | 4.2 | 13.5 |
| (k) | Silicone | DIMETHICONE | | | 0.5 |
| (l) | Miscellaneous Emulsifiers | SORBITAN ISOSTEARATE (HLB = 4.7) | | 0.04 | 0.3 |
| | | SORBITAN OLEATE (HLB = 4.3) | | 0.1 | |
| | | DISODIUM ETHYLENE DICOCAMIDE PEG-15 DISULFATE | | 0.5 | |
| | Other Miscellaneous emulsifiers/surfactants, Salts, Preservatives, pH Adjusters, Fragrances, Colorants, Chelants, Extracts, Fillers, Absorbants, and/or Additional Skin Actives, etc. | | ≤4 | ≤4 | ≤4 |
| **Stability** | | | **NO** | **NO** | **NO** |

**Example 5**

The compositions of Example 3 and Example 4 were subjected to stability studies and visually evaluated for phase separation and assessed under a microscope for particulate formation. The compositions were analyzed upon initial manufacture of the composition ($T_0$). The compositions were again analyzed after 10 days of freeze-thaw testing. For freeze-thaw testing, the compositions were placed in a stability chamber and subjected to temperature fluctuation at 12-hour intervals. For 12 hours, the compositions were held at -20°C. For the next 12 hours, the compositions were held at 25°C. The cycle was repeated 10 times (for 10 days). Separately, the compositions of Example 1 were evaluated after 4 weeks (1 month) in storage at 4°C, 25°C, 37°C, and 45°C and again at 8 weeks (2 months) at 4°C, 25°C, 37°C, and 45°C and visually evaluated for phase separation and assessed under a microscope for particulate formation.

The inventive compositions were deemed stable ("Y") (yes) because they did not visually phase separate and did not form particulates. The Comparative Compositions (C-1 through C-3) were deemed not stable ("N") (no) because they phase separated and/or formed particulates. The data show the importance of the one or more nonionic emulsifier chosen from glyceryl esters having HLB of about 3 to about 8 (c) and the importance of the one or more nonionic emulsifiers having an HLB of about 16 to about 18 (d). If one of these types of emulsifiers is not included, the resulting composition lacks stability, i.e., exhibits phase separation and particulate formation, as shown by the data for the Comparative Compositions (C-1, C-2, and C-3).

***Oil in Water Emulsions Comprising Trifluoromethylphenyl Valylglycine***

In certain embodiments, the cosmetic compositions in the form of oil in water emulsions include acetyl trifluoromethylphenyl valylglycine. The amount of acetyl trifluoromethylphenyl valylglycine will vary but in various embodiment is from about 1 to about 5 wt.%, based on the total weight of the cosmetic composition. In various embodiments, the amount of the acetyl trifluoromethylphenyl valylglycine is from about 1.5 to about 5 wt.%, about 2 to about 5 wt.%, about 2.5 to about 5 wt.%, about 1 to about 4 wt.%, about 1.5 to about 4 wt.%, about 2 to about 4 wt.%, about 2.5 to about 4 wt.%, or about 3 wt.%, based on the total weight of the cosmetic compositions. In another embodiment, the total amount of acetyl trifluoromethylphenyl valylglycine is from about 2.6 to about 3.4 wt.%, based on the total weight of

the cosmetic composition.

In certain embodiments, the cosmetic compositions include:

(a) acetyl trifluoromethylphenyl valylglycine, preferably in amounts set forth above;

(b) hydroxypropyl tetrahydropyrantriol;

(c) two or more taurate copolymers;

(d) one or more fatty alcohols;

(e) one or more fatty compounds;

(f) one or more nonionic emulsifiers; and

(g) water;
wherein all percentages by weight are based on the total weight of the cosmetic composition.

The amount of hydroxypropyl tetrahydropyrantriol in the cosmetic compositions will vary but in certain embodiments is from about 10 wt.% to about 40 wt.%, based on the total weight of the composition. In further embodiments, the amount of hydroxypropyl tetrahydropyrantriol in the composition is from about 10 wt.% to about 35 wt.%, from about 10 to about 30 wt.%, from about 10 to about 25 wt.%, from about 10 to about 20 wt.%, about 12 to about 35 wt.%, about 12 to about 30 wt.%, about 12 to about 25 wt.%, about 12 to about 20 wt.%, from about 12 to about 18 wt.%, from about 14 to about 30 wt.%, from about 14 to about 25 wt.%, from about 14 to about 20 wt.%, or from about 14 to about 18 wt.%, based on the total weight of the composition.

Nonlimiting examples of taurate copolymers include acrylamide/sodium acryloyl dimethyl taurate copolymer, hydroxyethyl acrylate/sodium acryloyl dimethyl taurate copolymer, ammonium acryloyldimethyl taurate/VP copolymer, sodium acrylate/sodium acryloyl dimethyl taurate copolymer, hydroxyethyl acrylate/ sodium acryloyldimethyl taurate copolymer, and a mixture thereof.

In a particularly preferred embodiment at least one of the two or more taurate copolymers is chosen from taurate copolymers that function as polymeric emulsifiers, in particular, chosen from hydroxyethyl acrylate/sodium acryloyl dimethyl taurate copolymer, ammonium acryloyldimethyltaurate/steareth-25 methacrylate crosspolymer,ammonium acryloyldimethyltaurate/beheneth-25 methacrylate crosspolymer, and mixtures thereof.

Nonlimiting examples taurate copolymers include acrylamide/sodium acryloyl dimethyl taurate copolymer, hydroxyethyl acrylate/sodium acryloyl dimethyl taurate copolymer, ammonium acryloyldimethyl taurateNP copolymer, sodium acrylate/sodium acryloyl dimethyl taurate copolymer, hydroxyethyl acrylate/ sodium acryloyldimethyl taurate copolymer, and a mixture thereof

The total amount of the two or more taurate copolymers will vary but in certain embodiments the total amount of the two or more taurate copolymers is from about 2 wt.% to about 15 wt.% based on the total weight of the cosmetic composition. In further embodiments, the amount of the two or more taurate copolymers in the cosmetic composition is from about 2 to about 12 wt.%, about 2 to about 10 wt.%, about 2 to about 8 wt.%, about 2 to about 5 wt.%, about 2 to about 4 wt.%, about 2 to about 3 wt.%, about 2.5 to about 15 wt.%, about 2.5 to about 12 wt.%, about 2.5 to about 10 wt.%, about 2.5 to about 8 wt.%, about 2.5 to about 5 wt.%, or about 2.5 to about 4 wt.%, based on the total weight of the cosmetic composition.

In a preferred embodiment, each of the individual taurate copolymers included in the two or more taurate copolymers of cosmetic compositions is in a minimum amount of at least 0.7 wt.%, preferably at least 0.8 wt.%, based on the total weight of the cosmetic composition. In other words, none of the individual taurate copolymers is present in an amount of less than 0.7 wt.%, based on the total weight of the cosmetic composition. Therefore, in certain embodiments, the total amount of the two or more taurate copolymers in the cosmetic composition is from about 2 wt.% to about 15 wt.% based on the total weight of the cosmetic composition, provided that each individual taurate copolymer of the two or more taurate copolymers is in an amount of at least 0.7 wt.%, preferably at least 0.8 wt.%, based on the total weight of the cosmetic composition.

In further embodiments, the amount of the two or more taurate copolymers in the cosmetic composition is from about 2 to about 12 wt.%, about 2 to about 10 wt.%, about 2 to about 8 wt.%, about 2 to about 5 wt.%, about 2 to about 4 wt.%, about 2 to about 3 wt.%, about 2.5 to about 15 wt.%, about 2.5 to about 12 wt.%, about 2.5 to about 10 wt.%, about 2.5 to about 8 wt.%, about 2.5 to about 5 wt.%, or about 2.5 to about 4 wt.%, based on the total weight of the cosmetic composition, provided that each individual taurate copolymer of the two or more taurate copolymers is in an amount of at least 0.7 wt.%, preferably at least 0.8 wt.%, based on the total weight of the cosmetic composition.

In certain embodiments, it is preferable that the cosmetic composition includes three or more taurate copolymers. In particular, it is useful to include three or more taurate copolymers chosen from acrylamide/sodium acryloyl dimethyl taurate copolymer, hydroxyethyl acrylate/sodium acryloyl dimethyl taurate copolymer, ammonium acryloyldimethyl taurateNP copolymer, sodium acrylate/sodium acryloyl dimethyl taurate copolymer, hydroxyethyl acrylate/ sodium acryloyldimethyl taurate copolymer, and a mixture thereof. More preferably, is it useful to use three or more taurate copolymers comprising ammonium acryloyldimethyl taurateNP copolymer, acrylamide/sodium acryloyl dimethyl taurate copolymer, and hydroxyethyl acrylate/ sodium acryloyldimethyl taurate copolymer. In a particularly preferred embodiment, these three taurate copolymers are included in the following amounts, based on the total weight of the cosmetic compositions:

- 0.1 to 4 wt.%, preferably 0.5 to 3 wt.%, more preferably 0.6 to 2 wt.% of ammonium acryloyldimethyl taurateNP copolymer,

- 0.7 to 4 wt.%, preferably 0.7 to 3 wt.%, more preferably, 0.8 to 2 wt.% of acrylamide/sodium acryloyl dimethyl taurate copolymer, and

- 0.1 to about 4 wt.%, preferably 0.5 to 3 wt.%, more preferably 0.6 to 2 wt.% of hydroxyethyl acrylate/ sodium acryloyldimethyl taurate copolymer.

In a preferred embodiment, the cosmetic composition comprises:

- 0.6 to 2 wt.% of ammonium acryloyldimethyl taurateNP copolymer,

- 0.8 to 2 wt.% of acrylamide/sodium acryloyl dimethyl taurate copolymer, and

- 0.6 to 2 wt.% of hydroxyethyl acrylate/ sodium acryloyldimethyl taurate copolymer.

Nonlimiting examples of fatty alcohols include those chosen from $C_6$-$C_{20}$ fatty alcohols, for example, decyl alcohol, undecyl alcohol, dodecyl alcohol, myristyl alcohol, cetyl alcohol, stearyl alcohol, isostearyl alcohol, isocetyl alcohol, behenyl alcohol, arachidyl alcohol, eicosyl alcohol, myristyl alcohol, 2-dodecylhexadecanol, 2-tetradecyl-1-octadecanol, 2-tetradecyl-1-eicosanol, 2-hexadecyl-1-octadecanol, 2-hexadecyl-1-eicosanol, octyldodecanol, 2-octyl-1-dodecanol, and a mixture thereof. A more exhaustive but nonlimiting list of useful fatty alcohols is included under the heading "Fatty Alcohols."

The total amount of the one or more fatty alcohols will vary but in certain embodiments is from about 0.5 wt.% to about 10 wt.%, based on the total weight of the composition. In further embodiments, the amount of one or more fatty alcohols is from about 0.5 to about 8 wt.%, about 0.5 to about 5 wt.% 0.5 to about 4 wt.%, about 0.5 to about 3 wt.%, about 1 to about 10 wt.%, about 1 to about 8 wt.%, about 1 to about 5 wt.%, about 1 to about 4 wt.%, about 1 wt.% to about 3 wt.%, about 1.5 to about 10 wt.%, about 1.5 to about 8 wt.%, about 1.5 to about 5 wt.%, about 1.5 to about 4 wt.%, about 1.5 to about 3 wt.%, based on the total weight of the composition.

Nonlimiting examples of fatty compounds include fatty esters (such as isononyl isononanoate), polyolefins (such as petrolatum), waxes, squalane, squalene, hydrogenated polyisobutene, hydrogenated polydecene, polybutene, mineral oil, pentahydrosqualene, plant and/or vegetable oil, hydrocarbon-based oils (such as isohexadecane), and a mixture thereof. A more exhaustive but nonlimiting list of fatty compounds is included under the heading "Fatty Compounds"

The amount of the one or more fatty compounds in the cosmetic compositions will vary but in certain embodiments is from about 2 wt.% to about 25 wt.%, based on the total weight of the cosmetic composition. In further embodiments, the total amount of the one or more fatty compounds is from about 2 to about 20 wt.%, about 2 to about 15 wt.%, about 2 to about 12 wt.%, about 3 to about 20 wt.%, about 3 to about 15 wt.%, about 3 to about 12 wt.%, about 5 to about 20 wt.%, about 5 to about 15 wt.%, about 5 to about 12 wt.%, about 6 to about 15 wt.%, or about 6 to about 12 wt.%, based on the total weight of the cosmetic composition.

Nonlimiting examples of nonionic emulsifiers include alkanolamides, sorbitan fatty esters (such as sorbitan isostearate and sorbitan oleate), ethoxylated sorbitan fatty esters (such as polysorbate 80), polyol esters, glyceryl esters, polyglucosides (such as ceteraryl glucoside), glycerol ethers, oxyethylenated ethers, oxypropylenated ethers, and ethylene glycol polymers. A more exhaustive but nonlimiting list of nonionic emulsifiers is included under the heading "Nonionic Emulsifiers."

The total amount of the one or more nonionic emulsifiers in the cosmetic compositions will vary but in certain embodiments is from about 0.1 wt.% to about 10 wt.%, based on the total weight of the cosmetic composition. In further embodiments, the total amount of the one or more nonionic emulsifiers in the cosmetic composition is from about 0.1 to

about 8 wt.%, from about 0.1 to about 5 wt.%, from about 0.1 to about 3 wt.%, about 0.2 to about 10 wt.%, about 0.2 to about 8 wt.%, about 0.2 to about 5 wt.%, about 0.2 to about 3 wt.%, about 0.3 to about 10 wt.%, about 0.3 to about 8 wt.%, about 0.3 to about 5 wt.%, or about 0.3 to about 3 wt.%, 0.5 to about 10 wt.%, about 0.5 to about 8 wt.%, about 0.5 to about 5 wt.%, or about 0.5 to about 3 wt.%, 1.0 to about 10 wt.%, about 1.0 to about 8 wt.%, about 1.0 to about 5 wt.%, or about 1.0 to about 3 wt.%, based on the total weight of the cosmetic composition.

The amount of water in the cosmetic compositions will vary depending on the amount of the other components in the cosmetic compositions. Nonetheless, in certain embodiments, the amount of water is from about 35 wt.% to about 85 wt.%. In further embodiments, the amount of the water is from about 35 to about 80 wt.%, about 35 to about 75 wt.%, about 35 to about 70 wt.%, about 40 to about 85 wt.%, about 40 to about 80 wt.%, about 40 to about 75 wt.%, about 40 to about 70 wt.%, about 50 to about 85 wt.%, about 50 to about 80 wt.%, about 50 to about 75 wt.%, about 50 to about 70 wt.%, based on the total weight of the cosmetic composition.

Nonlimiting examples of water-soluble solvents include glycerin, mono-alcohols, polyols (such as polyhydric alcohols), glycols, and a mixture thereof. A more exhaustive but nonlimiting list of water-soluble solvents is included under the heading "Water-Soluble Solvents."

The total amount of the one or more water-soluble solvents will vary but in certain embodiments is from about 0.1 to about 20 wt.%, based on the total weight of the cosmetic composition. In further embodiments, the total amount of the one or more water-soluble solvents is from about 0.1 to about 15 wt.%, about 0.1 to about 10 wt.%, about 0.5 to about 20 wt.%, about 0.5 to about 15 wt.%, about 0.5 to about 10 wt.%, about 1 to about 20 wt.%, about 1 to about 15 wt.%, about 1 to about 10 wt.%, based on the total weight of the cosmetic composition.

In certain embodiments, the cosmetic compositions may optionally include one more thickening polymer(s), which are different from the two or more taurate copolymers (c). A nonlimiting list of useful thickening polymers is included under the heading "Thickening Polymers."

The amount of the one or more thickening polymer(s), when present, will vary but in certain embodiments is from about 0.01 to about 5 wt.%, based on the total weight of the composition. In further embodiments, the total amount of the one or more thickening polymer(s) is from about 0.01 to about 4 wt.%, about 0.01 to about 3 wt.%, about 0.1 to about 5 wt.%, about 0.1 to about 4 wt.%, about 0.1 to about 3 wt.%, about 0.5 to about 5 wt.%, about 0.5 to about 4 wt.%, or about 0.5 to about 3 wt.%, based on the total weight of the composition.

Nonlimiting examples of skin active agents include madecassoside, a moisturizing agent, a depigmenting agent, an anti-wrinkle agent, a skin active agent for oily skin, an antioxidant, a flavonoid, a vitamin, a skin whitening agent, and a mixture thereof. A more exhaustive list of useful miscellaneous ingredients is included under the heading "Miscellaneous Ingredients."

Miscellaneous ingredients can be included in the cosmetic composition, for example, in an amount of about 0.01 to about 10 wt.%, based on the total weight of the cosmetic composition. The total amount of the one or more miscellaneous ingredients may be about 0.01 to about 8 wt.%, about 0.01 to about 5 wt.%, about 0.01 to about 3 wt.%, about 0.1 to about 10 wt.%, about 0.1 to about 8 wt.%, about 0.1 to about 5 wt.%, about 0.1 to about 3 wt.%, about 1 to about 10 wt.%, about 1 to about 8 wt.%, about 1 to about 5 wt.%, or about 1 to about 3 wt.%, based on the total weight of the cosmetic composition.

Silicones can optionally be included in the cosmetic compositions but preferably the compositions are free or essentially free from silicones. Silicones are synthetic polymers made up of repeating units of siloxane, elemental silicon and oxygen, combined with other elements, most often carbon and hydrogen. Thus, silicones are also called polysiloxanes. In some instances, the cosmetic compositions of the instant case can be free or essentially free from dimethicones, amomdimethicones, dimethiconols, cyclosiloxanes, siloxanes, etc.

In certain embodiments, the compositions in the form of oil in water emulsions include:

(a) about 1 to about 5 wt.% of acetyl trifluoromethylphenyl valylglycine;

(b) about 10 to about 40 wt.% of hydroxypropyl tetrahydropyrantriol;

(c) about 2 to about 15 wt.% of two or more taurate copolymers;

(d) about 0.5 to about 10 wt.% of one or more fatty alcohols;

(e) about 5 to about 25 wt.% of one or more fatty compounds;

(f) about 0.1 to about 10 wt.% of one or more nonionic emulsifiers; and

(g) water;

wherein the composition is an oil in water emulsion, preferably a gel emulsion, and all percentages by weight are

based on the total weight of the cosmetic composition.

In certain embodiments, the cosmetic compositions in the form of oil in water emulsions comprise or consist of:

(a) about 1 to about 5 wt.%, preferably about 2 to about 4 wt.%, more preferably about 2 to about 4 wt/% of acetyl trifluoromethylphenyl valylglycine;

(b) about 10 to about 40 wt.%, more preferably about 10 to about 20 wt.%, and more preferably about 12 to about 18 wt.% of hydroxypropyl tetrahydropyrantriol;

(c) about 2 to about 15 wt.%, preferably about 2 to about 8 wt.%, more preferably about 2 to about 5 wt.% of two or more, preferably three or more taurate copolymers, for example, chosen from acrylamide/sodium acryloyl dimethyl taurate copolymer, hydroxyethyl acrylate/sodium acryloyl dimethyl taurate copolymer, ammonium acryloyldimethyl taurate/VP copolymer, sodium acrylate/sodium acryloyl dimethyl taurate copolymer, hydroxyethyl acrylate/ sodium acryloyldimethyl taurate copolymer, and a mixture thereof, preferably all three of acrylamide/sodium acryloyl dimethyl taurate copolymer, hydroxyethyl acrylate/sodium acryloyl dimethyl taurate copolymer, ammonium acryloyldimethyl taurateNP copolymer;

(d) about 0.5 to about 10 wt.%, preferably about 0.5 to about 8 wt.%, more preferably about 1 to about 5 wt.% of one or more fatty alcohols, preferably having from 10 to 30 carbon atoms, more preferably chosen from decyl alcohol, undecyl alcohol, dodecyl, myristyl, cetyl alcohol, stearyl alcohol, cetearyl alcohol, isostearyl alcohol, isocetyl alcohol, behenyl alcohol, oleyl alcohol, myricyl alcohol and a mixture thereof;

(e) about 5 to about 25 wt.%, preferably about 5 to about 20 wt.%, more preferably about 5 to about 15 wt.% of one or more fatty compounds, for example, one or more fatty compounds chosen from fatty esters (such as isononyl isononanoate), polyolefins (such as petrolatum), waxes, squalane, squalene, hydrogenated polyisobutene, hydrogenated polydecene, polybutene, mineral oil, pentahydrosqualene, plant and/or vegetable oil, hydrocarbon-based oils (such as isohexadecane), and a mixture thereof, more preferably chosen from isohexadecane, isononyl isononanoate, squalene, soybean oil, and a mixture thereof;

(f) about 0.1 to about 10 wt.%, preferably about 0.1 to about 5, even more preferably about 0.1 to about 3 wt.% of one or more nonionic emulsifiers, for example, chosen from alkanolamides, sorbitan fatty esters (e.g., sorbitan isostearate and sorbitan oleate), ethoxylated sorbitan fatty esters (e.g., polysorbate-80), polyol esters, glyceryl esters, polyglucosides (e.g., cetearyl glucoside), glycerol ethers, oxyethylenated ethers, oxypropylenated ethers, and ethylene glycol polymers, preferably chosen from polysorbate 80, cetearyl glucoside, sorbitan isostearate, sorbitan oleate, mixtures thereof;

(g) about 40 to about 80 wt.%, preferably about 40 to about 70 wt.%, more preferably about 45 to about 65 wt.% of water;

(h) optionally, one or more water-soluble solvents, for example, one or more water-soluble solvents chosen glycerin, alcohols (for example, C1-30, C1-15, C1-10, or C1-4 alcohols), organic solvents, polyols (polyhydric alcohols), glycols (e.g., propylene glycol, butylene glycol, caprylyl glycol, etc.), and a mixture thereof, preferably one or more glycols, in particular propylene glycol, wherein if present, the one or more water-soluble solvents comprise about 0.1 to about 20 wt.%, preferably about 0.1 to about 15 wt.%, more preferably about 1 to about 15 wt.% of the cosmetic composition;

(i) optionally, one or more thickening polymers that are different from the one or more taurate copolymers of (c), for example, chosen from polyacrylate, polymethacrylate, polyethylacrylate, polyacrylamide, poly C10-30 alkyl acrylate, and mixtures thereof, preferably poly C10-30 alkyl acrylate, wherein if present, the one or more thickening polymers comprise about 0.01 to about 5 wt.%, preferably about 0.05 to about 4 wt.%, more preferably about 0.1 to about 3 wt.% of the cosmetic composition;

(j) optionally, one or more miscellaneous ingredients, for example, one or more miscellaneous ingredients chosen from miscellaneous emulsifiers/surfactants other than the nonionic emulsifiers of (f), preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, preferably wherein at least one of the one or more miscellaneous ingredients is a further skin active agent such as madecassoside, wherein if present, the one or more miscellaneous

ingredients comprise about 0.01 to about 0.1 to about 10 wt.%, preferably about 0.1 to about 8 wt.%, more preferably about 1 to about 8 wt.% of the cosmetic composition;
wherein the composition is an oil in water emulsion, preferably a gel emulsion, and all percentages by weight are based on the total weight of the cosmetic composition.

As noted above, in certain embodiments, it is preferable to include three or more taurate copolymers in the cosmetic composition. The three or more taurate copolymers may be ammonium acryloyldimethyl taurateNP copolymer, acrylamide/sodium acryloyl dimethyl taurate copolymer, hydroxyethyl acrylate/ sodium acryloyldimethyl taurate copolymer, preferably in the following amounts, based on the total weight of the cosmetic compositions:

- 0.1 to 4 wt.%, preferably 0.5 to 3 wt.%, more preferably 0.6 to 2 wt.% of ammonium acryloyldimethyl taurateNP copolymer,

- 0.7 to 4 wt.%, preferably 0.7 to 3 wt.%, more preferably, 0.8 to 2 wt.% of acrylamide/sodium acryloyl dimethyl taurate copolymer, and

- 0.1 to about 4 wt.%, preferably 0.5 to 3 wt.%, more preferably 0.6 to 2 wt.% of hydroxyethyl acrylate/ sodium acryloyldimethyl taurate copolymer.

In a particularly preferred embodiment, the cosmetic composition comprises:

- 0.6 to 2 wt.% of ammonium acryloyldimethyl taurateNP copolymer,

- 0.8 to 2 wt.% of acrylamide/sodium acryloyl dimethyl taurate copolymer, and

- 0.6 to 2 wt.% of hydroxyethyl acrylate/ sodium acryloyldimethyl taurate copolymer.

In a preferred embodiment, the cosmetic composition in the form of an oil in water emulsion comprises or consists of:

(a) about 1 to about 5 wt.%, preferably about 2 to about 4 wt.%, more preferably about 2 to about 4 wt/% of acetyl trifluoromethylphenyl valylglycine;

(b) about 10 to about 40 wt.%, more preferably about 10 to about 20 wt.%, and more preferably about 12 to about 18 wt.% of hydroxypropyl tetrahydropyrantriol;

(c) about 2 to about 15 wt.%, preferably about 2 to about 8 wt.%, more preferably about 2 to about 5 wt.% of two or more, preferably three or more taurate copolymers, for example, chosen from acrylamide/sodium acryloyl dimethyl taurate copolymer, hydroxyethyl acrylate/sodium acryloyl dimethyl taurate copolymer, ammonium acryloyldimethyl taurate/VP copolymer, sodium acrylate/sodium acryloyl dimethyl taurate copolymer, hydroxyethyl acrylate/ sodium acryloyldimethyl taurate copolymer, and a mixture thereof, preferably acrylamide/sodium acryloyl dimethyl taurate copolymer, hydroxyethyl acrylate/sodium acryloyl dimethyl taurate copolymer, and ammonium acryloyldimethyl taurateNP copolymer;

(d) about 0.5 to about 10 wt.%, preferably about 0.5 to about 8 wt.%, more preferably about 1 to about 5 wt.% of one or more fatty alcohols having from 10 to 30 carbon atoms, for example, one or more fatty alcohols chosen from decyl alcohol, undecyl alcohol, dodecyl, myristyl, cetyl alcohol, stearyl alcohol, cetearyl alcohol, isostearyl alcohol, isocetyl alcohol, behenyl alcohol, oleyl alcohol, myricyl alcohol and a mixture thereof, preferably comprising behenyl alcohol and cetearyl alcohol;

(e) about 5 to about 25 wt.%, preferably about 5 to about 20 wt.%, more preferably about 5 to about 15 wt.% of one or more fatty compounds, for example, one or more fatty compounds chosen from fatty esters (such as isononyl isononanoate), polyolefins (such as petrolatum), waxes, squalane, squalene, hydrogenated polyisobutene, hydrogenated polydecene, polybutene, mineral oil, pentahydrosqualene, plant and/or vegetable oil, hydrocarbon-based oils (such as isohexadecane), and a mixture thereof, more preferably chosen from isohexadecane, isononyl isononanoate, squalene, soybean oil, and a mixture thereof;

(f) about 0.1 to about 10 wt.%, preferably about 0.1 to about 5, even more preferably about 0.1 to about 3 wt.% of one or

more nonionic emulsifiers, for example, chosen from sorbitan fatty esters (e.g., sorbitan isostearate and sorbitan oleate), ethoxylated sorbitan fatty esters (e.g., polysorbate-80), polyol esters, glyceryl esters, polyglucosides (e.g., cetearyl glucoside), preferably chosen from polysorbate 80, cetearyl glucoside, sorbitan isostearate, sorbitan oleate, mixtures thereof;

(g) about 40 to about 80 wt.%, preferably about 40 to about 70 wt.%, more preferably about 45 to about 65 wt.% of water;

(h) about 0.1 to about 20 wt.%, preferably about 0.1 to about 15 wt.%, more preferably about 1 to about 15 wt.% of one or more water-soluble solvents, for example, one or more water-soluble solvents chosen glycerin, alcohols (for example, C1-30, C1-15, C1-10, or C1-4 alcohols), organic solvents, polyols (polyhydric alcohols), glycols (e.g., propylene glycol, butylene glycol, caprylyl glycol, etc.), and a mixture thereof, preferably one or more glycols, preferably propylene glycol;

(i) optionally, one or more thickening polymers that are different from the one or more taurate copolymers of (c), for example, chosen from polyacrylate, polymethacrylate, polyethylacrylate, polyacrylamide, poly C10-30 alkyl acrylate, and mixtures thereof, preferably poly C10-30 alkyl acrylate, wherein if present, the one or more thickening polymers comprise about 0.01 to about 5 wt.%, preferably about 0.05 to about 4 wt.%, more preferably about 0.1 to about 3 wt.% of the cosmetic composition;

(j) about 0.01 to about 0.1 to about 10 wt.%, preferably about 0.1 to about 8 wt.%, more preferably about 1 to about 8 wt.% of one or more miscellaneous ingredients, for example, one or more miscellaneous ingredients chosen from miscellaneous emulsifiers/surfactants other than the nonionic emulsifiers of (f), preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, preferably wherein at least one of the one or more miscellaneous ingredients is a further skin active agent, which is preferably madecassoside;
wherein the composition is an oil in water emulsion, preferably a gel emulsion and all percentages by weight are based on the total weight of the cosmetic composition.

As noted above, in some instance, three or more taurate copolymers may be included in the cosmetic composition. The three or more taurate copolymers may be ammonium acryloyldimethyl taurate/VP copolymer, acrylamide/sodium acryloyl dimethyl taurate copolymer, hydroxyethyl acrylate/ sodium acryloyldimethyl taurate copolymer, preferably in the following amounts, based on the total weight of the cosmetic compositions:

- 0.1 to 4 wt.%, preferably 0.5 to 3 wt.%, more preferably 0.6 to 2 wt.% of ammonium acryloyldimethyl taurateNP copolymer,
- 0.7 to 4 wt.%, preferably 0.7 to 3 wt.%, more preferably, 0.8 to 2 wt.% of acrylamide/sodium acryloyl dimethyl taurate copolymer, and
- 0.1 to about 4 wt.%, preferably 0.5 to 3 wt.%, more preferably 0.6 to 2 wt.% of hydroxyethyl acrylate/ sodium acryloyldimethyl taurate copolymer.

In a preferred embodiment, the cosmetic composition comprises:

- 0.6 to 2 wt.% of ammonium acryloyldimethyl taurateNP copolymer,
- 0.8 to 2 wt.% of acrylamide/sodium acryloyl dimethyl taurate copolymer, and
- 0.6 to 2 wt.% of hydroxyethyl acrylate/ sodium acryloyldimethyl taurate copolymer.

The composition preferably has a pH of about 5.5 to about 8, preferably about 5.5 to about 7.5, more preferably about 5.5 to about 7.

The cosmetic compositions of the instant disclosure are stable. For example, the cosmetic compositions do not visually phase separate or form visibly observable particulates for at least 2 weeks, 4 weeks, and/or 8 weeks in storage at 4°C, 25°C, 37°C, and/or 45°C.

In various embodiments, the cosmetic compositions do not visually phase separate or form visibly observable

particulates for at least 10 cycles of freeze-thaw testing, wherein the freeze-thaw testing comprises placing the cosmetic composition in a stability chamber and subjecting it to temperature fluctuation at 12-hour intervals, for a first interval of 12 hours at -20°C followed by a second interval of 12 hours at 25°C.

In various embodiments, the viscosity of the cosmetic compositions does not change by more than 20%, 15%, 10%, or 5%, for at least 2 weeks, 4 weeks, and/or 8 weeks in storage at 4°C, 25°C, 37°C, and/or 45°C.

The cosmetic compositions preferably have a viscosity of about 5,000 to about 200,000 Pa.s at 25°C, and shear rate of 1 $s^{-1}$ at 25°C. However, the cosmetic compositions may have a viscosity of about 10,000 to about 200,000 Pa.s, about 10,000 to about 180,000 Pa.s, about 10,000 to about 150,000 Pa.s, about 10,000 to about 120,000 Pa.s, about 10,000 to about 100,000 Pa.s, about 10,000 to about 80,000 Pa.s, about 15,000 to about 200,000 Pa.s, about 15,000 to about 180,000 Pa.s, about 15,000 to about 150,000 Pa.s, about 15,000 to about 120,000 Pa.s, about 15,000 to about 100,000 Pa.s, about 15,000 to about 80,000 Pa.s, about 20,000 to about 200,000 Pa.s, about 20,000 to about 180,000 Pa.s, about 20,000 to about 150,000 Pa.s, about 20,000 to about 120,000 Pa.s, about 20,000 to about 100,000 Pa.s, about 20,000 to about 80,000 Pa.s, about 30,000 to about 200,000 Pa.s, about 30,000 to about 180,000 Pa.s, about 30,000 to about 150,000 Pa.s, about 30,000 to about 120,000 Pa.s, about 30,000 to about 100,000 Pa.s,about 30,000 to about 80,000 Pa.s, about 35,000 to about 200,000 Pa.s, about 35,000 to about 180,000 Pa.s, about 35,000 to about 150,000 Pa.s, about 35,000 to about 120,000 Pa.s, about 35,000 to about 100,000 Pa.s, about 35,000 to about 80,000 Pa.s, about 40,000 to about 200,000 Pa.s, about 40,000 to about 180,000 Pa.s, about 40,000 to bout 150,000 Pa.s, about 40,000 to about 120,000 Pa.s, about 40,000 to about 100,000 Pa.s, about 40,000 to about 80,000 Pa.s at 25°C, and shear rate of 1 $s^{-1}$ at 25°C.

**Example 6**

**(Oil in Water Emulsions with Acetyl Trifluoromethylphenyl Valylglycine)**

| | | | Inventive | | | | | | Comparative | | |
|---|---|---|---|---|---|---|---|---|---|---|---|
| | | | **A** | **B** | **C** | **D** | **E** | **F** | **C-1** | **C-2** | **C-3** |
| (a) | Active | ACETYL TRIFLUOROMETHY L-PHENYLVALYLGLY CINE | 3. 0 | 3. 0 | 3. 0 | 3. 0 | 3. 0 | 3. 0 | 3.0 | 3.0 | 3.0 |
| (b) | Active | HYDROXYPROPYL TETRAHYDROPYRANTRIOL | 15 | 15 | 15 | 15 | 15 | 15 | 15 | 15 | 15 |
| (j) | Active (misc.) | MADECASSOSIDE | 0. 9 | 0. 9 | 0. 9 | 0. 9 | 0. 9 | 0. 9 | 0.9 | 0.9 | 0.9 |
| (c) | Taurate Co-polym er | AMMONIUM ACRYLOYLDI-METH YL-TAURATE/ VP CO-POLYMER | 1. 2 | 0. 7 | 0. 7 | 1. 0 | 1. 2 | 0. 7 | 1.2 | 0.7 | 0.7 |
| | | ACRYLAMIDE/SODI UM ACRYLOYLDIMETH YLTAURATE COPOLYMER | 1. 0 | 1. 0 | 0. 8 | 1. 0 | 1. 0 | 1. 0 | 0.5 | 1.0 | 0.6 |
| | | HYDROXYETHYL ACRYLA-TE/SODIUM ACRYLOYLDI-METH YL TAURATE COPO-LYMER | 0. 7 | 0. 7 | 0. 7 | 0. 7 | 0. 7 | 0. 7 | 0.7 | - | 0.7 |
| (d) | Fatty Alco-hol | BEHENYL ALCOHOL and CE-TEARYL ALCOHOL | 1. 8 | 1. 8 | 1. 8 | 1. 8 | 1. 8 | 1. 8 | 1.8 | 1.8 | 1.8 |
| (i) | Thickeni ng Polymer | POLY C10-30 ALKYL ACRYLATE | 0. 5 | 0. 5 | - | 0. 5 | 0. 5 | 0. 5 | 0.5 | 0.5 | - |
| (e) | Fatty Com-pou nd | ISOHEXADECANE, ISONO-NYL ISONONANOATE, SQUALANE, AND/OR GLYCINE SOJA (SOYBEAN) OIL | 9. 5 | 9. 5 | 9. 5 | 9. 5 | 9. 5 | 9. 5 | 9.5 | 9.5 | 9.5 |

(continued)

| | | | Inventive | | | | | | Comparative | | |
|---|---|---|---|---|---|---|---|---|---|---|---|
| | | | A | B | C | D | E | F | C-1 | C-2 | C-3 |
| (f) | Nonionic Emulsifie r | POLYSORBATE 80, CETEARYL GLUCOSIDE, SORBITAN ISOSTEARATE, AND/OR SORBITAN OLEATE | 0. 5 | 0. 5 | 0. 4 | 0. 5 | 0. 5 | 0. 5 | 0.4 | 0.5 | 0.4 |
| (h) | Solvent | PROPYLENE GLYCOL | 11 | 1. 7 | 1. 7 | 1. 7 | 1. 7 | 1. 7 | 11 | 1.7 | 1.7 |
| (j) | Miscellaneous emulsifers/surfactants, salts, preservatives, pH adjusters, fragrances, colorants, chelants, and/or extracts, etc. | | ≤ 4 | ≤ 4 | ≤ 4 | ≤ 4 | ≤ 4 | ≤ 4 | ≤4 | ≤4 | ≤4 |
| (g) | WATER | | 54 | 64 | 64 | 63 | 63 | 63 | 55 | 64 | 64 |
| **Stable** | | | **Y** | **Y** | **Y** | **Y** | Y | **Y** | N | N | N |

**Example 7**

**(Comparative Compositions)**

| | | | C-4 | C-5 | C-6 |
|---|---|---|---|---|---|
| (a) | Active | ACETYL TRIFLUOROMETHYLPHENYL VALYLGLYCINE | 3.0 | 3.0 | 3.0 |
| (b) | Active | HYDROXYPROPYL TETRAHYDROPYRANTRIOL | | 15.0 | 15.0 |
| (c) | Taurate Copolymer | AMMONIUM ACRYLOYLDIMETHYLTAURATE/ VP COPOLYMER | | 1.5 | |
| (d) | Fatty Alcohol | BEHENYL ALCOHOL and CETEARYL ALCOHOL | | | 0.8 |
| | Thickening Agent | SODIUM POLYACRYLATE | 1.3 | 0.4 | 0.4 |
| (e) | Fatty Compound | ISONONYL ISONONANOATE, CETEARYL ETHYLHEXANOATE, AND/OR CAPRYLIC/CAPRIC TRIGLYCERIDE | 3 | 2.7 | 2.7<br>2.7 |
| | Silicone | DIMETHICONE | | 0.5 | 0.5 |
| (f) | Nonionic Emulsifier | CETEARYL GLUCOSIDE | | | 0.2 |
| (h) | WS Solvent | PROPYLENE GLYCOL AND/OR GLYCERIN | 8 | 10.7 | 10.7 |
| (j) | Miscellaneous emulsifers/surfactants, Emolliants, Salts, Preservatives, pH Adjusters, Fragrances, Colorants, Chelants, and/or Extracts, Etc. | | ≤4 | ≤4 | ≤4 |
| (g) | Water | | 77 | 57 | 58 |
| **Stable** | | | **N** | **N** | **N** |

**Example 8**

**(Stability Testing)**

The compositions of Example 1 and Example 2 were subjected to stability studies and visually evaluated for phase separation and assessed under a microscope for particulate formation. The compositions were assessed upon initial manufacture of the composition ($T_0$). The compositions were again evaluated after 10 days of freeze-thaw testing. The compositions were placed in a stability chamber and subjected to temperature fluctuation at 12-hour intervals. For 12 hours, the compositions were held at -20°C. For the next 12 hours, the compositions were held at 25°C. The cycle was

repeated 10 times (for 10 days). Separately, the compositions of Example 1 and Example 2 were evaluated after 2 weeks in storage at 4°C, 25°C, 37°C, and 45°C, 4 weeks (1 month) in storage at 4°C, 25°C, 37°C, and 45°C, and again at 8 weeks (2 months) in storage at 4°C, 25°C, 37°C, and 45°C and visually evaluated for phase separation and assessed under a microscope for particulate formation.

The inventive compositions were deemed stable ("Y") (yes) because they did not visually phase separate and did not form particulates. The Comparative Compositions (C-1 through C-6), however, phase separated and formed particulates. Therefore, the Comparative Compositions were deemed not stable ("N") (no).

The data shows, among other things, the importance of including at least two taurate copolymers in an amount of at least 2 wt.%, based on the total weight of the composition.

***Oil in Water Emulsions with Ceramide-NP***

Ceramide NP represents "(9Z)-N-[(2S,3S,4R)-1,3,4-trihydroxyoctadecan-2-yl]octadec-9-enamide." Ceramide NP consists of a phytosphingosine backbone N-acylated with a saturated fatty acid (stearic acid). Ceramide NP provides numerous benefits to the skin such as, rehydration of dry skin, reduction of itching, chronic dryness, relieving itching, reduction of peeling and scaling.

Generally, the amount of ceramide NP in the cosmetic compositions ranges from about 0.1 wt.% to about 5 wt.% based on the total weight of the composition. In various embodiments, the amounts of ceramide NP ranges from about 0.1 wt.% to about 4.0 wt.%, from about 0.1 to about 3 wt.%, from about 0.2 wt.% to about 5 wt.%, about 0.2 to about 4 wt.%, about 0.2 to about 2 wt.%, about 0.2 to about 3 wt.%, about 0.3 to about 5 wt.%, about 0.3 to about 4 wt.%, about 0.3 to about 3 wt.%, about 0.3 to about 2 wt.%, about 0.4 to about 5 wt.%, about 0.4 to about 4 wt.%, about 0.4 to about 3 wt.%, about 0.4 to about 2 wt.%, about 0.5 wt.% to about 5 wt.%, about 0.5 wt.%, about 4 wt.%, about 0.5 wt.% to about 3 wt.%, about 0.5 to about 2 wt.%, or 0.5 to about 1 wt.%, based on the total weight of the composition. In even further embodiments, the amount of ceramide NP is at least 0.1 wt.%, at least 0.2 wt.%, at least 0.3 wt.%, at least 0.4 wt.%, at least 0.5 wt.%, or at least 0.6 wt.% and can have a maximum of about 1, 2, 3, 4, or 5 wt.%, based on the total weight of the composition.

In an aspect, the present disclosure is directed to, among other things, a cosmetic composition in the form of an oil in water emulsion. In an embodiment, the cosmetic composition includes:

(a) ceramide NP;

(b) water;

(c) one or more first emulsifiers chosen from polyglycerol-based emulsifiers;

(d) one or more second emulsifiers chosen from glyceryl esters having an HLB (hydrophilic-lipophilic balance) of about 3 to about 6;

(e) one or more third emulsifiers chosen from ethoxylated fatty acids;

(f) one or more fatty alcohols; and

(g) one or more non-triglyceride and non-aromatic fatty compounds;
wherein the composition is an oil-in-water emulsion and all percentages by weight are based on the total weight of the composition.

In certain embodiments, the cosmetic compositions include one or more of the following weight ratios for (a) and (c)-(g):

a weight ratio of the one or more first emulsifiers chosen from polyglycerol-based emulsifiers to the ceramide NP of about 1.5:1 to about 8:1 ((c):(a)); and/or

a weight ratio of the one or more second emulsifiers chosen from glyceryl esters having an HLB of about 3 to about 6 to the ceramide NP of about 0.8:1 to about 4:1 ((d):(a)); and/or

a weight ratio of the one or more third emulsifiers chosen from ethoxylated fatty acids to the ceramide NP of about 0.7:1 to about 4:1 ((e):(a)); and/or

a weight ratio of the one or more fatty alcohols to the ceramide NP of about 0.7:1 to about 4:1 ((f):(a)); and/or

a weight ratio of the one or more non-triglyceride and non-aromatic fatty compounds to the ceramide NP of about 4:1 to about 20:1 ((g):(a)).

The amount of water in the composition of the instant disclosure can and will vary depending on the amount of the other components in the cosmetic composition. Nonetheless, in certain embodiments, the amount of water in the composition is from about 50 to about 90 wt.%, based on the total weight of the composition. In various embodiments, the amount of water in the cosmetic compositions is from about 55 to about 90 wt.%, about 60 to about 90 wt.%, about 65 to about 90 wt.%, about 70 to about 90 wt.%, about 60 to about 85 wt.%, about 65 to about 85 wt.%, about 70 to about 85 wt.%, about 60 to about 80 wt.%, about 65 to about 80 wt.%, or about 70 to about 80 wt.%, based on the total weight of the composition.

Nonlimiting examples of polyglycerol-based emulsifiers include polyglyceryl 10-stearate, polyglyceryl-3-caprate, polyglyceryl-3-diisostearate, polyglyceryl-3 methylglucose distearate, or a mixture thereof. A more exhaustive but nonlimiting list of polyglycerol-based emulsifiers is included under the heading "Polyglycerol-Based Emulsifiers."

The amount of the one or more first emulsifier chosen from polyglycerol-based emulsifiers varies but, in some embodiments, is from about 0.5 wt.% to about 5 wt.% based on the total weight of the composition. In further embodiments, the amount of the one or more first emulsifier chosen from polyglycerol-based emulsifiers is from about 0.5 wt.% to about 4 wt.%, about 0.5 to about 3 wt.%, about 0.5 to about 2 wt.%, about 0.6 to about 5 wt.%, about 0.6 to about 4 wt.%, about 0.6 to about 3 wt.%, about 0.6 to about 2 wt.%, about 0.8 to about 4 wt.%, about 0.8 wt.% about 3 wt.%, or about 0.8 to about 2 wt.%, based on the total weight of the composition.

Nonlimiting examples of glyceryl esters having an HLB of about 3 to about 8 include glyceryl behenate, glyceryl erucate, glyceryl hydroxystearate, glyceryl isostearate, glyceryl lanolate, glyceryl laurate, glyceryl linoleate, glyceryl myristate, glyceryl palmitate lactate, ***glyceryl stearate,*** glyceryl distearate, glyceryl laurate, or a mixture thereof. In at least one instance the glyceryl ester comprises glyceryl stearate, glyceryl ricinoleate, and mixtures thereof. A more exhaustive but nonlimiting list of glyceryl esters is provided under the heading "Glyceryl Fatty Esters."

In general, the amount of the one or more of a second emulsifier(s) chosen from glyceryl ester having a HLB of about 3 to about 6 may range from about 0.5 wt.% to about 5 wt.%, based on the total weight of the composition. In various embodiments, the amount of the one or more of a second emulsifier chosen from glyceryl ester having a HLB of about 3 to about 6 may range from about 0.2 to about 5 wt.%, about 0.2 to about 4 wt.%, about 0.2 to about 3 wt.%, about 0.5 wt.% to about 4 wt.%, about 0.5 to about 3 wt.%, about 0.5 to about 2 wt.%, about 0.6 to about 5 wt.%, about 0.6 to about 4 wt.%, about 0.6 to about 3 wt.%, about 0.6 to about 2 wt.%, about 0.8 to about 4 wt.%, about 0.8 wt.% about 3 wt.%, or about 0.8 to about 2 wt.%, about 1.0 to about 4 wt.%, about 1.0 wt.% about 3 wt.%, or about 1.0 to about 2 wt.%, based on the total weight of the composition.

Nonlimiting examples of ethoxylated fatty acids having from 40 to 100 propylene oxide groups and the fatty acid chain ranging from 12 to 24 carbons include lauric acid, tridecylic acid, myristic acid, pentadecylic acid, palmitic acid, margaric acid, stearic acid, nonadecylic aciud, arachidic acid, heniicosylic acid, behenic acid, tricosylic cid, and lignoceric acid, especially containing 40 to 100 propylene oxide groups. A more exhaustive but nonlimiting list of ethoxylated fatty acids is provided under the heading "Ethoxylated Fatty Acids."

The amount of the one or more third emulsifiers chosen from ethoxylated fatty acids will vary. Nonetheless, in various embodiment is from about 0.5 wt.% to about 5 wt.%, based on the total weight of the composition. In various embodiments, the total amount of the one or more third emulsifiers chosen from ethoxylated fatty acids is from about 0.2 to about 5 wt.%, about 0.2 to about 4 wt.%, about 0.2 to about 3 wt.%, about 0.5 wt.% to about 4 wt.%, about 0.5 to about 3 wt.%, about 0.5 to about 2 wt.%, about 0.6 to about 5 wt.%, about 0.6 to about 4 wt.%, about 0.6 to about 3 wt.%, about 0.6 to about 2 wt.%, about 0.8 to about 4 wt.%, about 0.8 wt.% about 3 wt.%, or about 0.8 to about 2 wt.%, about 1.0 to about 4 wt.%, about 1.0 wt.% about 3 wt.%, or about 1.0 to about 2 wt.%, based on the total weight of the composition.

Nonlimiting example of fatty alcohols include those having from 12 to 24 carbon atoms. For example, fatty alcohols chosen from cetyl alcohol, stearyl alcohol, cetearyl alcohol, behenyl alcohol, lauryl alcohol, myristic or myristyl alcohol, arachidyl alcohol, lignoceryl alcohol, and mixtures thereof. A more exhaustive but nonlimiting list of fatty alcohols is provided under the heading "Fatty Alcohols."

The amount of one or more fatty alcohols in the cosmetic compositions may vary but in various embodiments ranges from about 0.2 wt.% to about 5 wt.%, based on the total weight of the composition. In various embodiments, the amount of the one or more fatty alcohols in the composition ranges from about 0.2 to about 5 wt.%, about 0.2 to about 4 wt.%, about 0.2 to about 3 wt.%, about 0.2 to about 2 wt.%, about 0.3 to about 5 wt.%, about 0.3 to about 4 wt.%, about 0.3 to about 3 wt.%, about 0.3 to about 2 wt.%, about 0.5 to about 4 wt.%, about 0.5 to about 3 wt.%, about 0.5 to about 2 wt.%, about 0.7 to about 4 wt.%, about 0.7 to about 3 wt.%, about 0.7 to about 2 wt.%, based on the total weight of the composition.

Nonlimiting examples of non-triglyceride and non-aromatic fatty compounds include fatty esters (isopropyl myristate, sorbitan isostearate), sarcosinates, for instance an acyl sarcosinate, plant and/or vegetable oils, and mixtures thereof. Non-limiting examples of sarcosinates can be selected from the group consisting of sodium lauroyl sarcosinate,

sodium cocoyl sarcosinate, sodium myristoyl sarcosinate, sodium caproyl sarcosinate, TEA-cocoyl sarcosinate, ammonium cocoyl sarcosinate, ammonium lauroyl sarcosinate, dimer dilinoleyl bis-lauroylglutamate/lauroylsarcosinate, disodium lauroamphodiacetate lauroyl sarcosinate, isopropyl lauroyl sarcosinate, potassium cocoyl sarcosinate, potassium lauroyl sarcosinate, sodium cocoyl sarcosinate, sodium lauroyl sarcosinate, sodium myristoyl sarcosinate, sodium oleoyl sarcosinate, sodium palmitoyl sarcosinate, TEA-cocoyl sarcosinate, TEA-lauroyl sarcosinate, TEA-oleoyl sarcosinate, TEA-palm kernel sarcosinate, and combinations thereof. Further nonlimiting examples of non-triglyceride and non-aromatic fatty compounds is included under the heading "Fatty Compounds."

The total amount of the one or more non-triglyceride and non-aromatic fatty compounds will vary. Nonetheless, in certain embodiments the total amount of the non-triglyceride and non-aromatic fatty compounds is from about 4 to about 20 wt.%, based on the total weight of the composition. In further embodiments, total amount of the one or more non-triglyceride and non-aromatic fatty compounds is from about 1 to about 20 wt.%, about 1 to about 15 wt.%, about 1 to about 10 wt.%, about 2 to about 20 wt.%, about 2 to about 15 wt.%, about 2 to about 10 wt.%, about 3 to about 20 wt.%, about 3 to about 15 wt.%, about 3 to about 10 wt.%, about 4 to about 20 wt.%, about 4 to about 15 wt.%, or about 4 to about 10 wt.%, based on the total weight of the composition.

In various embodiments, the cosmetic compositions may optionally include one or more thickening polymers. A list of useful thickening polymers is provided under the heading "Thickening Polymers."

The amount of the one or more thickening polymers, when present, will vary. Nonetheless, in various embodiments the total amount of the one or more thickening polymers is from about 0.01 to about 5 wt.%, based on the total weight of the composition. In further embodiments, the total amount of the one or more thickening polymers is from about 0.01 to about 4 wt.%, about 0.01 to about 3 wt.%, about 0.1 to about 5 wt.%, about 0.1 to about 4 wt.%, about 0.1 to about 3 wt.%, about 0.5 to about 5 wt.%, about 0.5 to about 4 wt.%, or about 0.5 to about 3 wt.%, about 1 to about 5 wt.%, about 1 to about 4 wt.%, or about 1 to about 3 wt.%, about 1.5 to about 5 wt.%, about 1.5 to about 4 wt.%, or about 1.5 to about 3 wt.%, based on the total weight of the composition.

In various embodiments, the cosmetic compositions may optionally include one or more water soluble solvents. Non-limiting examples of water-soluble solvents include, for example, glycerin, alcohols (for example, $C_{1-30}$, $C_{1-15}$, $C_{1-10}$, or $C_{1-4}$ alcohols), organic solvents, polyols, glycols, and a mixture thereof. A more exhaustive but nonlimiting list of water-soluble solvents is included under the heading "Water-Soluble Solvents."

In various embodiments, silicones can optionally be included in the cosmetic compositions but preferably the compositions are free or essentially free from silicones. Silicones are synthetic polymers made up of repeating units of siloxane, elemental silicon and oxygen, combined with other elements, most often carbon and hydrogen. Thus, silicones are also called polysiloxanes. In some instances, the cosmetic compositions of the instant case can be free or essentially free from dimethicones, amomdimethicones, dimethiconols, cyclosiloxanes, siloxanes, etc.

The total amount of the one or more water-soluble solvents can and will vary but is typically about 1 to about 20 wt.%, based on the total weight of the cosmetic composition. In some cases, the total amount of the one or more water-soluble solvents may be about 1 to about 15 wt.%, about 1 to about 10 wt.%, about 1 to about 5 wt.%, about 2 to about 20 wt.%, about 2 to about 15 wt.%, about 2 to about 10 wt.%, about 2 to about 5 wt.%, about 5 to about 20 wt.%, about 5 to about 15 wt.%, or about 5 to about 10 wt.%, based on the total weight of the cosmetic composition.

In an embodiment, the cosmetic compositions include one or more miscellaneous ingredients. Nonlimiting examples of miscellaneous ingredients include miscellaneous emulsifiers/surfactants other than the dimethicone copolyol emulsifiers of (d), preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, and mixtures thereof. In an embodiment, the cosmetic composition includes at least one further skin active agent, for example, madecassoside. A more exhaustive but nonlimiting list of miscellaneous ingredients is provided under the heading "Miscellaneous Ingredients"

Miscellaneous ingredients, when present, can be in an amount from about 0.01 to about 10 wt.%, based on the total weight of the cosmetic composition. The total amount of the one or more miscellaneous ingredients may be about 0.01 to about 8 wt.%, about 0.01 to about 5 wt.%, about 0.01 to about 3 wt.%, about 0.1 to about 10 wt.%, about 0.1 to about 8 wt.%, about 0.1 to about 5 wt.%, about 0.1 to about 3 wt.%, about 1 to about 10 wt.%, about 1 to about 8 wt.%, about 1 to about 5 wt.%, or about 1 to about 3 wt.%, based on the total weight of the cosmetic composition.

In certain embodiments the cosmetic compositions in the form of oil in water emulsions include:

(a) about 0.1 to about 5 wt.% of ceramide NP;

(b) water;

(c) about 0.5 to about 5 wt.% of one or more first emulsifiers chosen from polyglycerol-based emulsifiers;

(d) about 0.5 to about 5 wt.% of one or more second emulsifiers chosen from glyceryl esters having an HLB of about 3 to about 6;

(e) about 0.5 to about 5 wt.% of one or more third emulsifiers chosen from ethoxylated fatty acids;

(f) about 0.2 to about 5 wt.% of one or more fatty alcohols; and

(g) about 4 to about 20 wt.% of one or more non-triglyceride and non-aromatic fatty compounds;
wherein the composition is an oil in water emulsion and all percentages by weight are based on the total weight of the composition, provided that one or more of the following ratios apply:

a weight ratio of the one or more first emulsifiers chosen from polyglycerol-based emulsifiers to the ceramide NP of about 1.5:1 to about 8:1 ((c):(a)); and/or

a weight ratio of the one or more second emulsifiers chosen from glyceryl esters having an HLB of about 3 to about 6 to the ceramide NP of about 0.8:1 to about 4:1 ((d):(a)); and/or

a weight ratio of the one or more third emulsifiers chosen from ethoxylated fatty acids to the ceramide NP of about 0.7:1 to about 4:1 ((e):(a)); and/or

a weight ratio of the one or more fatty alcohols to the ceramide NP of about 0.7:1 to about 4:1 ((f):(a));

a weight ratio of the one or more non-triglyceride and non-aromatic fatty compounds to the ceramide NP of about 4:1 to about 20:1 ((g):(a)).

In various embodiments, it is preferably that the cosmetic compositions include one or more ratios relating to components (a) and (c)-(g).

In an embodiment, the weight ratio of the one or more first emulsifiers chosen from polyglycerol-based emulsifiers to the ceramide NP is from about 1.5:1 to about 8:1 ((c):(a)), preferably about 1.5:1 to about 5:1, more preferably about 1.5:1 to about 3:1.

In an embodiment, the weight ratio of the one or more second emulsifiers chosen from glyceryl esters having an HLB of about 3 to about 6 to the ceramide NP to is from about 0.8:1 to about 4:1 ((d):(a)), preferably 1:1 to about 3:1, more preferably about 1.2:1 to about 2.5:1.

In an embodiment, the weight ratio of the one or more third emulsifiers chosen from ethoxylated fatty acids to the ceramide NP is from about 0.7:1 to about 4:1 ((e):(a)), preferably about 0.8:1 to about 3:1, more preferably about 0.8:1 to about 2.5:1.

In an embodiment, the weight ratio of the one or more fatty alcohols to the ceramide NP is from about 0.7:1 to about 4:1 ((f):(a)), preferably about 0.8:1 to about 3:1, more preferably about 0.8:1 to about 2.5:1.

In an embodiment, the weight ratio of the one or more non-triglyceride and non-aromatic fatty compounds to the ceramide NP is from about 4:1 to about 20:1 ((g):(a)), preferably about 5:1 to 15:1, more preferably about 6:1 to 10:1.

In a preferred embodiment, the cosmetic compositions include the following ratio relating to components (a) and (c)-(g):

the weight ratio of the one or more first emulsifiers chosen from polyglycerol-based emulsifiers to the ceramide NP is from about 1.5:1 to about 8:1 ((c):(a)), preferably about 1.5:1 to about 5:1, more preferably about 1.5:1 to about 3:1;

the weight ratio of the one or more second emulsifiers chosen from glyceryl esters having an HLB of about 3 to about 6 to the ceramide NP to is from about 0.8:1 to about 4:1 ((d):(a)), preferably 1:1 to about 3:1, more preferably about 1.2:1 to about 2.5:1;

the weight ratio of the one or more third emulsifiers chosen from ethoxylated fatty acids to the ceramide NP is from about 0.7:1 to about 4:1 ((e):(a)), preferably about 0.8:1 to about 3:1, more preferably about 0.8:1 to about 2.5:1;

the weight ratio of the one or more fatty alcohols to the ceramide NP is from about 0.7:1 to about 4:1 ((f):(a)), preferably about 0.8:1 to about 3:1, more preferably about 0.8:1 to about 2.5:1; and

the weight ratio of the one or more non-triglyceride and non-aromatic fatty compounds to the ceramide NP is from about 5:1 to about 20:1 ((g):(a)), preferably about 5:1 to 15:1, more preferably about 6:1 to 10:1.

In certain embodiments the cosmetic compositions in the form of oil in water emuisions comprise or consist of:

(a) about 0.1 to about 5 wt.%, preferably about 0.1 to about 3 wt.%, more preferably about 0.2 to about 2 wt.%, even more preferably, about 0.3 to about 1.5 wt.% of ceramide NP;

(b) about 50 to about 90 wt.%, preferably about 55 to about 80 wt.%, more preferably about 55 to about 75 wt.% of water;

(c) about 0.5 to about 5 wt.%, more preferably about 0.5 to about 4 wt.%, even more preferably about 0.8 to about 3 wt.% of one or more first emulsifiers chosen from polyglycerol-based emulsifiers, preferably:

- one or more polyglyceryl esters of C12-22 saturated, unsaturated and branched chain fatty acids, such as polyglyceryl-4 isostearate, polyglyceryl-3 oleate, polyglyceryl-2-sesquioleate, triglyceryl diisostearate, diglyceryl monooleate, tetraglyceryl monooleate, and mixtures thereof; and or
- one or more polyglyceryl methylglucose surfactants, such as polyglyceryl-3 methylglucose distearate, polyglyceryl-6 methylglucose distearate, polyglyceryl-10 methyl glucose distearate;

(d) about 0.5 to about 5 wt.%, more preferably about 0.5 to about 4 wt.%, even more preferably about 0.8 to about 3 wt.% of one or more second emulsifiers chosen from glyceryl esters having an HLB of about 3 to about 6, preferably chosen from bis-diglyceryl polyacyladipate-2, glyceryl behenate, glyceryl caprate, glyceryl cocoate, glyceryl erucate, glyceryl hydroxystearate, glyceryl isostearate, glyceryl lanolate, glyceryl laurate, glyceryl linoleate, glyceryl myristate, glyceryl oleate, glyceryl palmitate lactate, glyceryl sesquioleate, glyceryl stearate, glyceryl stearate citrate, glyceryl stearate lactate, glyceryl dioleate, glyceryl distearate, glyceryl laurate, and mixtures thereof, more preferably chosen from glyceryl stearate, bis-diglyceryl polyacyladipate, or a mixture thereof;

(e) about 0.5 to about 5 wt.%, preferably about 0.5 to about 4 wt.%, more preferably about 0.5 to about 3 wt.% of one or more third emulsifiers chosen from ethoxylated fatty acids, preferably derived from a fatty acid containing 8 to 24 carbon atoms and having from 2 to 200 molecules of ethylene oxide, more preferably chosen from polethoxylated stearic acid esters, for example, PEG-9 stearate, PEG-8 distearate, PEG-20 stearate, PEG-8 stearate, PEG-8 oleate, PEG-20 stearate, PEG-30 stearate, PEG-40 stearate, PEG-50 stearate, PEG-100 stearate, PEG-150 laurate, and mixtures therefore;

(f) about 0.2 to about 5 wt.%, preferably about 0.3 to about 4 wt.%, more preferably about 0.5 to about 3 wt.% of one or more fatty alcohols, preferably having from 10 to 30 carbon atoms, more preferably chosen from decyl alcohol, undecyl alcohol, dodecyl, myristyl, cetyl alcohol, stearyl alcohol, cetearyl alcohol, isostearyl alcohol, isocetyl alcohol, behenyl alcohol, linalool, oleyl alcohol, myricyl alcohol and a mixture thereof;

(g) about 4 to about 20 wt.%, preferably about 4 to about 15 wt.%, more preferably about 5 to about 10 wt.% of one or more non-triglyceride and non-aromatic fatty compounds, preferably chosen from fatty esters (e.g., isopropyl myristate, sorbitan isostearate), acyl sarcosinates, oils, alkanes (paraffins), fatty acids, fatty alcohol derivatives, fatty acid derivatives, waxes, lanolin, and mixtures thereof; preferably chosen from fatty esters acyl sarcosinates, and mixtures thereof;

(h) optionally, hydroxypropyl tetrahydropyrantriol, wherein if present, is preferably in an amount of about 10 to about 40 wt.%, more preferably about 10 to about 20 wt.%, and more preferably about 12 to about 18 wt.%;

(i) optionally, one or more thickening polymers, wherein if present, may be in an amount of about 0.01 to about 5 wt.%, preferably about 0.1 to about 4 wt.%, more preferably about 0.1 to about 3 wt.%, for example, polyacrylates (e.g., sodium polyacrylate), hydroxyethyl acrylate/sodium acryloyldimethyl taurate copolymer, ammonium acryloyldimethyltaurateNP copolymer, acrylamide/sodium acryloyldimethyltaurate copolymer, polyacrylate crosspolymer-6, polyacrylamide, acrylatesc10-30 alkyl acrylate crosspolymer and mixtures thereof;

(j) optionally, one or more water-soluble solvents, wherein if present may be in an amount of about 1 to about 20 wt.%, preferably about 1 to about 15 wt.%, more preferably about 5 to about 15 wt.%, for example, glycerin, $C_2$-$C_6$ monoalcohols, polyols (polyhydric alcohols), glycols, and a mixture thereof; and

(k) optionally, one or more miscellaneous ingredients, wherein if present, may be in an amount of about 0.01 to about

10 wt.%, preferably about 0.1 to about 8 wt.%, more preferably about 1 to about 6 wt.%, for example, miscellaneous ingredients chosen from miscellaneous emulsifiers/surfactants, preservatives, fragrances, pH adjusters, salts, antioxidants, vitamins, botanical extracts, UV filtering agents, proteins, protein hydrolysates and/or isolates, hydrotropes, pearlescent agents, fillers, colorants, mattifying agents, further skin active agents, buffers, and mixtures thereof;
wherein the composition is an oil-in-water emulsion and all percentages by weight are based on the total weight of the composition.

In further embodiments the cosmetic compositions in the form of oil in water emulsions comprise or consist of:

(a) about 0.5 to about 5 wt.%, preferably about 0.5 to about 3 wt.% of ceramide NP;

(b) about 50 to about 80 wt.%, preferably about 60 to 80 wt.% of water;

(c) about 0.1 to about 5 wt.%, preferably about 0.5 to about 4 wt.% of one or more first emulsifiers chosen from polyglyceryl 10-stearate, polyglyceryl-3-caprate, polyglyceryl-3-diisostearate, polyglyceryl-3 methylglucose distearate, and a mixture thereof;

(d) about 0.5 to about 5 wt.%, preferably about 0.5 to about 4 wt.%, more preferably about 0.5 to about 3 wt.% of one or more second emulsifiers chosen from glyceryl esters having an HLB of about 3 to about 10, for example, chosen from bis-diglyceryl polyacyladipate-2, glyceryl behenate, glyceryl caprate, glyceryl cocoate, glyceryl erucate, glyceryl hydroxystearate, glyceryl isostearate, glyceryl lanolate, glyceryl laurate, glyceryl linoleate, glyceryl myristate, glyceryl oleate, glyceryl palmitate lactate, glyceryl sesquioleate, glyceryl stearate, glyceryl stearate citrate, glyceryl stearate lactate, glyceryl dioleate, glyceryl distearate, glyceryl laurate, or a mixture thereof. In at least one instance the glyceryl ester comprises glyceryl stearate, bis-diglyceryl polyacyladipate, glyceryl ricinoleate, or a mixture thereof;

(e) about 0.5 to about 5 wt.%, preferably about 0.5 to about 4 wt.%, more preferably about 0.5 to about 3 wt.% of one or more third emulsifiers chosen from ethoxylated fatty acids having propylene oxide groups ranging from 40 to 100 and fatty chain of 8 to 24 carbon atoms, preferably polethoxylated stearic acid esters, for example, PEG-9 stearate, PEG-8 distearate, PEG-20 stearate, PEG-8 stearate, PEG-8 oleate, PEG-20 stearate, PEG-30 stearate, PEG-40 stearate, PEG-50 stearate, PEG-100 stearate, PEG-150 laurate, and mixtures therefore;

(f) about 0.2 to about 5 wt.%, preferably about 0.3 to about 4 wt.%, more preferably about 0.5 to about 3 wt.% of one or more fatty alcohols having from 8 to 24 carbon atoms, preferably chosen from decyl alcohol, undecyl alcohol, dodecyl, myristyl, cetyl alcohol, stearyl alcohol, cetearyl alcohol, isostearyl alcohol, isocetyl alcohol, behenyl alcohol, and a mixture thereof;

(g) about 4 to about 20 wt.%, preferably about 4 to about 15 wt.%, more preferably about 5 to about 10 wt.% of one or more non-triglyceride and non-aromatic fatty compounds chosen from fatty esters, acyl sarcosinates, or a mixture thereof;

(h) optionally, hydroxypropyl tetrahydropyrantriol, wherein if present, is preferably in an amount of about 10 to about 40 wt.%, more preferably about 10 to about 20 wt.%, and more preferably about 12 to about 18 wt.%;

(i) optionally, one or more thickening polymers, wherein if present, may be in an amount of about 0.01 to about 5 wt.%, preferably about 0.1 to about 4 wt.%, more preferably about 0.1 to about 3 wt.%, for example, polyacrylates (e.g., sodium polyacrylate), hydroxyethyl acrylate/sodium acryloyldimethyl taurate copolymer, ammonium acryloyldimethyltaurateNP copolymer, acrylamide/sodium acryloyldimethyltaurate copolymer, polyacrylate crosspolymer-6, polyacrylamide, acrylatesc10-30 alkyl acrylate crosspolymer and mixtures thereof;

(j) about 1 to about 15 wt.% of one or more water-soluble solvents chosen from glycerin, $C_2$-$C_6$ mono-alcohols, polyols (polyhydric alcohols), glycols, and a mixture thereof; and

(k) optionally, one or more miscellaneous ingredients, wherein if present, may be in an amount of about 0.01 to about 10 wt.%, preferably about 0.1 to about 8 wt.%, more preferably about 1 to about 6 wt.%, for example miscellaneous ingredients chosen from miscellaneous emulsifiers/surfactants, preservatives, fragrances, pH adjusters, salts, antioxidants, vitamins, botanical extracts, UV filtering agents, proteins, protein hydrolysates and/or isolates, hydrotropes, pearlescent agents, fillers, colorants, mattifying agents, further skin active agents, buffers, and mixtures

thereof;
wherein the composition is an oil-in-water emulsion and all percentages by weight are based on the total weight of the composition.

In addition to the various amounts for components (a) and (c)-(g) set forth above, in various embodiments it is preferably that the cosmetic compositions include one or more ratios relating to components (a) and (c)-(g) chosen from:

a weight ratio of the one or more first emulsifiers chosen from polyglycerol-based emulsifiers to the ceramide NP from about 1.5:1 to about 8:1 ((c):(a)), preferably about 1.5:1 to about 5:1, more preferably about 1.5:1 to about 3:1;

a weight ratio of the one or more second emulsifiers chosen from glyceryl esters having an HLB of about 3 to about 6 to the ceramide NP from about 0.8:1 to about 4:1 ((d):(a)), preferably 1:1 to about 3:1, more preferably about 1.2:1 to about 2.5:1;

a weight ratio of the one or more third emulsifiers chosen from ethoxylated fatty acids to the ceramide NP from about 0.7:1 to about 4:1 ((e):(a)), preferably about 0.8:1 to about 3:1, more preferably about 0.8:1 to about 2.5:1;

a weight ratio of the one or more fatty alcohols to the ceramide NP from about 0.7:1 to about 4:1 ((f):(a)), preferably about 0.8:1 to about 3:1, more preferably about 0.8:1 to about 2.5:1;

a weight ratio of the one or more non-triglyceride and non-aromatic fatty compounds to the ceramide NP from about 4:1 to about 20:1 ((g):(a)), preferably about 5:1 to 15:1, more preferably about 6:1 to 10:1.

In a preferred embodiment, the cosmetic compositions include the following ratios relating to all of components (a) and (c)-(g):

the weight ratio of the one or more first emulsifiers chosen from polyglycerol-based emulsifiers to the ceramide NP is from about 1.5:1 to about 8:1 ((c):(a)), preferably about 1.5:1 to about 5:1, more preferably about 1.5:1 to about 3:1;

the weight ratio of the one or more second emulsifiers chosen from glyceryl esters having an HLB of about 3 to about 6 to the ceramide NP to is from about 0.8:1 to about 4:1 ((d):(a)), preferably 1:1 to about 3:1, more preferably about 1.2:1 to about 2.5:1;

the weight ratio of the one or more third emulsifiers chosen from ethoxylated fatty acids to the ceramide NP is from about 0.7:1 to about 4:1 ((e):(a)), preferably about 0.8:1 to about 3:1, more preferably about 0.8:1 to about 2.5:1;

the weight ratio of the one or more fatty alcohols to the ceramide NP is from about 0.7:1 to about 4:1 ((f):(a)), preferably about 0.8:1 to about 3:1, more preferably about 0.8:1 to about 2.5:1; and

the weight ratio of the one or more non-triglyceride and non-aromatic fatty compounds to the ceramide NP is from about 1:1 to about 20:1 ((g):(a)), preferably about 2:1 to 20:1, more preferably about 2:1 to 10:1.

The cosmetic compositions of the instant disclosure are preferably stable, and ceramide NP is preferably solubilized. With respect to stability, in certain embodiments, the cosmetic compositions do not visually phase separate or form visibly observable particulates for at least 2 weeks, 4 weeks, and/or 8 weeks in storage at 4°C, 25°C, 37°C, and/or 45°C.

In another embodiment, the cosmetic compositions do not visually phase separate or form visibly observable particulates for at least 10 cycles of freeze-thaw testing, wherein the freeze-thaw testing comprises placing the cosmetic composition in a stability chamber and subjecting it to temperature fluctuation at 12-hour intervals, for a first interval of 12 hours at -20°C followed by a second interval of 12 hours at 25°C.

In another embodiment, the viscosity of the cosmetic compositions does not change by more than 20%, 15%, 10%, or 5%, for at least 2 weeks, 4 weeks, and/or 8 weeks in storage at 4°C, 25°C, 37°C, and/or 45°C.

The cosmetic compositions preferably have a viscosity of about 5,000 to about 200,000 Pa.s at 25°C, and shear rate of 1 $s^{-1}$ at 25°C. However, the cosmetic compositions may have a viscosity of about 10,000 to about 200,000 Pa.s, about 10,000 to about 180,000 Pa.s, about 10,000 to about 150,000 Pa.s, about 10,000 to about 120,000 Pa.s, about 15,000 to about 200,000 Pa.s, about 15,000 to about 180,000 Pa.s, about 15,000 to about 150,000 Pa.s, about 15,000 to about 120,000 Pa.s, about 20,000 to about 200,000 Pa.s, about 20,000 to about 180,000 Pa.s, about 20,000 to about 150,000 Pa.s, about 20,000 to about 120,000, about 50,000 to about 200,000 Pa.s, about 50,000 to about 180,000 Pa.s,

about 50,000 to about 150,000 Pa.s, about 50,000 to about 120,000 Pa.s, about 70,000 to about 200,000 Pa.s, about 70,000 to about 180,000 Pa.s, about 70,000 to about 150,000 Pa.s, about 70,000 to about 120,000 Pa.s, or about 70,000 to about 100,000 Pa.s at 25°C, and shear rate of 1 $s^{-1}$ at 25°C.

**Example 9**

**(Cosmetic Compositions)**

| | | | **Inventive** | | **Comparative** | | | |
|---|---|---|---|---|---|---|---|---|
| | | | **A** | **B** | **C-1** | **C-2** | **C-3** | **C-4** |
| (a) | Active | CERAMIDE NP (Ceramide 3) | 0.6 | 0.6 | 0.6 | 0.6 | 0.6 | 0.6 |
| (b) | Water | WATER | QS | QS | QS | QS | QS | QS |
| (c) | Polyglyceryl-Based Emulsifier | POLYGLYCERYL-3 METHYLGLUCOSE DISTEARATE | 1.0 | 1.0 | | 1.0 | 1.0 | 1.0 |
| (d) | Glyceryl Ester Emulsifier | GLYCERYL STEARATE | 1.0 | 1.0 | 1.0 | | 1.0 | 1.0 |
| (e) | Ethoxylated Fatty Acid Emulsifier | PEG-100 STEARATE | 0.5 | 0.5 | 0.5 | 0.5 | | 0.5 |
| (f) | Fatty Alcohol | STEARYL ALCOHOL | 0.5 | 0.5 | 0.5 | 0.5 | 0.5 | |
| (g) | Fatty Compouds | ISOPROPYL MYRISTATE, SORBITAN ISOSTEARATE, AND/OR ISOPROPYL LAUROYL SARCOSINATE | 4.3 | 4.3 | 4.3 | 4.3 | 4.3 | 4.3 |
| (h) | Active | HYDROXYPROPYL TETRAHYDROPYRANTRIOL | 15.0 | 15.0 | 15.0 | 15.0 | 15.0 | 15.0 |
| (i) | Thickening Polymer | SODIUM POLYACRYLATE, AMMONIUM ACRYLOYLDIMETHYLTAURATE/VP COPOLYMER, AND/OR HYDROXYETHYL ACRYLATE/SODIUM ACRYLOYLDI METHYL TAURATE COPOLYMER | 1.1 | 1.4 | 1.4 | 1.4 | 1.4 | 1.4 |
| (j) | Water-Soluble Solvent | PROPYLENE GLYCOL, AND/OR T-BUTYL ALCOHOL | 10.7 | 10.7 | 10.7 | 10.7 | 10.7 | 10.7 |
| (k) | Miscellaneous emulsifers/surfactants, salts, preservatives, pH adjusters, fragrances, colorants, chelants, and/or extracts, etc. | | ≤4 | ≤4 | ≤4 | ≤4 | ≤4 | ≤4 |
| **Stable** | | | **Y** | **Y** | **NA** | **NA** | **NA** | **NA** |
| **Soluble (ceramide NP) below 95°C** | | | **Y** | **Y** | **N** | **N** | **N** | **N** |

**Example 10**

**(Stability Studies)**

Studies were carried out to determine the ceramide NP was solubilized below 95°C. To determine ceramide NP solubility, the components (a), (c), (d), (e), (f), and (g) were combined and heated to 95°C with constant agitation. If the mixture became transparent below 95°C it was solubilized initially. The mixtures were then added to a hot water phase (85-90°C) (component (b)), homogenized, stirred, and allowed to cool. Solubility was monitored via microscope and macroscopic changes (e.g., grittiness). Inventive Compositions A and B solubilized the ceramide NP and are therefore

designed with a "Y" (yes) for solubility in the table in Example 1. Comparative Compositions C-1 through C-4 did not solubilize the ceramide NP and are therefore designed with a "N" (no) for solubility in the table in Example 1. Because Comparative Compositions C-1 through C-4 did not solubilize the ceramide NP, these compositions were not subjected to further stability testing.

Inventive Compositions A and B of Example 1 were subjected to physical stability studies and visually evaluated for phase separation and assessed under a microscope for particulate formation. The compositions were analyzed upon initial manufacture of the composition ($T_0$). The compositions were again analyzed after 10 days of freeze-thaw testing. For freeze-thaw testing, the compositions were placed in a stability chamber and subjected to temperature fluctuation at 12-hour intervals. For 12 hours, the compositions were held at -20°C. For the next 12 hours, the compositions were held at 25°C. The cycle was repeated 10 times (for 10 days). Separately, the compositions of Example 1 were evaluated after 4 weeks (1 month) in storage at 4°C, 25°C, 37°C, and 45°C and again at 8 weeks (2 months) at 4°C, 25°C, 37°C, and 45°C and visually evaluated for phase separation and assessed under a microscope for particulate formation.

The inventive compositions were deemed stable ("Y") (yes) because they did not visually phase separate and did not form particulates.

**WATER IN SILICONES EMULSIONS**

The water in silicone emulsions of the instant case are characterized in that the emulsions include at least 70 wt.% of an aqueous phase (internal phase), based on the total weight of the silicone emulsion. Furthermore, the water in silicone emulsions include one or more active agents, preferably one or more active agents that are stabilized and/or solubilized in the water or silicone phase of the emulsion. In particularly preferred embodiment, at least one of the one or more active agents is hydroxypropyl tetrahydropyrantriol.

***Water in Silicone Emulsions Comprising Hydroxypropyl Tetrahydropyrantriol***

The amount of hydroxypropyl tetrahydropyrantriol in the cosmetic compositions will vary. Nonetheless, in an embodiment, the amount of hydroxypropyl tetrahydropyrantriol is from about 10 wt.% to about 50 wt.% based on the total weight of the composition. In further embodiments, the amount of hydroxypropyl tetrahydropyrantriol in the composition is from about 12, to about 50 wt.%, about 12 to about 45 wt.%, about 12 to about 40 wt.%, about 12 to about 35 wt.%, about 12 to about 30 wt.%, about 15 to about 50 wt.%, about 15 to about 45 wt%, about 15 to about 40 wt.%, about 15 to about 35 wt.%, about 15 to about 30 wt.%, about 20 to about 50 wt.%, about 20 to about 45 wt.%, about 20 to about 40 wt.%, about 20 to about 35 wt.%, about 20 to about 30 wt.%, about 25 to about 50 wt.%, about 25 to about 45 wt.%, about 25 to about 40 wt.%, about 25 to about 35 wt.%, about 30 to about 50 wt.%, about 30 to about 45 wt.%, about 30 to about 40 wt.%, based on the total weight of the cosmetic composition.

In an embodiment, the present disclosure is directed to, among other things, water in silicone emulsions, wherein the silicone emulsion is a cosmetic composition comprising:

(a) hydroxypropyl tetrahydropyrantriol, preferably in amounts set forth above;

(b) one or more silicone oils;

(c) one or more non-emulsifying silicone elastomers;

(d) one or more dimethicone copolyol emulsifiers comprising at least one oxyethylene group and at least one oxypropylene group;

(e) water; and

(f) one or more water-soluble solvents.

Nonlimiting examples of silicone oils include dimethicone, dimethiconol, cyclopentasiloxane, cyclomethicone, cyclotetrasiloxane, cyclohexasiloxane, cycloheptasiloxane, decamethylcyclopentasiloxane, cyclotetrasiloxane, cyclotrisiloxane, capryldimethicone, caprylyl trimethicone, caprylyl methicone, cetearylmethicone, hexadecylmethicone, hexylmethicone, lauryl methicone, myristyl methicone, phenyl methicone, stearyl methicone, stearyl dimethicone, behenyl dimethicone, trifluoropropyl methicone, cetyl dimethicone, polyphenylmethylsiloxane, dimethylpolysiloxane, methylphenylpolysiloxane, methyltrimethicone, diphenylsiloxyphenyl trimethicone, and phenyl trimethicone, and mixtures thereof. In some embodiments, dimethicone is a particularly useful silicone oil. A more exhaustive but nonlimiting list of useful silicone oils is included under the heading "Silicone Oils."

The total amount of the one or more silicone oils in the cosmetic compositions will vary but in various embodiment is from about 5 to about 50 wt.%, based on the total weight of the cosmetic composition. In further embodiments, the total amount of the one or more silicone oils is from about 5 to about 45 wt.%, about 5 to about 40 wt.%, about 5 to about 35 wt.%, about 10 to about 45 wt.%, about 10 to about 40 wt.%, about 10 to about 35 wt.%, about 15 to about 50 wt.%, about 15 to about 45 wt.%, about 15 to about 40 wt.%, about 15 to about 35 wt.%, about 20 to about 50 wt.%, about 20 to about 45 wt.%, about 20 to about 40 wt.%, about 20 to about 35 wt.%, about 25 to about 50 wt.%, about 25 to about 45 wt.%, about 25 to about 40 wt.%, or about 25 to about 35 wt.%, based on the total weight of the cosmetic composition.

Nonlimiting examples of non-emulsifying silicone elastomers include dimethicone crosspolymer, vinyl dimethicone crosspolymer, dimethicone/vinyl dimethicone crosspolymer, dimethicone crosspolymer-3, polysilicone-11, and mixtures thereof. In some embodiments, dimethicone crosspolymers is a particularly useful silicone elastomer. A more exhaustive but nonlimiting list of useful non-emulsifying silicone elastomers is included under the heading "Non-Emulsifying Silicone Elastomers."

The total amount of the one or more non-emulsifying silicone elastomers may be from about 0.1 to about 10 wt.%, based on the total weight of the cosmetic composition. In some embodiments, the total amount of the one or more non-emulsifying silicone elastomers may be in an amount of about 0.1 to about 8 wt.%, about 0.1 to about 5 wt.%, about 0.5 to about 10 wt.%, about 0.5 to about 8 wt.%, about 0.5 to about 5 wt.%, about 1 to about 10 wt.%, about 1 to about 8 wt.%, about 1 to about 5 wt.%, or about 1 to about 3 wt.%, 1.5 to about 10 wt.%, about 1.5 to about 8 wt.%, about 1.5 to about 5 wt.%, or about 1.5 to about 3 wt.%, based on the total weight of the cosmetic composition.

Nonlimiting examples of dimethicone copolyol emulsifiers comprising at least one oxyethylene group and at least one oxypropylene group include bis-PEG/PPG-14/14 dimethicone, bis-isobutyl PEG/PPG-10/7 dimethicone copolymer, bis-PEG/PPG-18/6 dimethicone; bis-PEG/PPG-20/20 dimethicone, bis-PEG/PPG-16/16 dimethicone, cetyl PEG/PPG-15/16 butyl ether dimethicone, cetyl PEG/PPG-15/15 butyl ether dimethicone, cetyl PEG/PPG-7/3 dimethicone, cetyl PEG/PPG-10/1 dimethicone, dimethicone PEG/PPG-7/4 phosphate, dimethicone PEG/PPG-12/4 phosphate, PEG/PPG-28/21 acetate dimethicone, PEG/PPG-22/22 butyl ether dimethicone, PEG/PPG-23/23 butyl ether dimethicone, PEG/PPG-24/18 butyl ether dimethicone, PEG/PPG-3/10 dimethicone, PEG/PPG-4/12 dimethicone, PEG/PPG-6/11 dimethicone, PEG/PPG-8/14 dimethicone, PEG/PPG-12/16 dimethicone, PEG/PPG-12/18 dimethicone, PEG/PPG-14/4 dimethicone, PEG/PPG-15/5 dimethicone, PEG/PPG-15/15 dimethicone, PEG/PPG-16/2 dimethicone, PEG/PPG-16/8 dimethicone, PEG/PPG-17/18 dimethicone, PEG/PPG-18/12 dimethicone, PEG/PPG-19/19 dimethicone, PEG/PPG-20/6 dimethicone, PEG/PPG-20/15 dimethicone, PEG/PPG-20/20 dimethicone, PEG/PPG-20/29 dimethicone, PEG/PPG-22/23 dimethicone, PEG/PPG-22/24 dimethicone, PEG/PPG-25/25 dimethicone, PEG/PPG-27/27 dimethicone, PEG/PPG-30/10 dimethicone, PEG/PPG-10/3 oleyl ether dimethicone, and mixtures thereof. A more exhaustive but nonlimiting list of dimethicone copolyol emulsifiers is provided under the heading "Dimethicone Copolyol Emulsifiers."

The total amount of the one or more dimethicone copolyol emulsifiers will vary but in various embodiment is from about 0.1 to about 10 wt.%, based on the total weight of the cosmetic composition. In further embodiments, the total amount of the one or more dimethicone copolyol emulsifiers is from about 0.1 to about 8 wt.%, about 0.1 to about 5 wt.%, about 0.5 to about 10 wt.%, about 0.5 to about 8 wt.%, about 0.5 to about 5 wt.%, about 1 to about 10 wt.%, about 1 to about 8 wt.%, about 1 to about 5 wt.%, or about 1 to about 3 wt.%, 1.5 to about 10 wt.%, about 1.5 to about 8 wt.%, about 1.5 to about 5 wt.%, or about 1.5 to about 3 wt.%, based on the total weight of the cosmetic composition.

The weight ratio of the one or more non-emulsifying silicone elastomers (c) to the one or more dimethicone copolyol emulsifiers (d) may vary. In an embodiment, the ratio of the one or more non-emulsifying silicone elastomers (c) to the one or more dimethicone copolyol emulsifiers (d) is from about 1:10 to about 10:1, about 1:8 to 8:1, about 1:6 to about 6:1, about 1:5 to about 5:1, about 1:4 to 4:1, about 1:3 to about 3 to about 1. In further embodiments, the ratio of the one or more non-emulsifying silicone elastomers (c) to the one or more dimethicone copolyol emulsifiers (d) is from about 1:5 to about 5:1, about 1:4 to about 4:1, about 1:3 to about 3:1, or about 1:2 to about 2:1.

The amount of water in the cosmetic compositions can and will vary depending on the amount of the other components in the cosmetic compositions. In an embodiment, the total amount of water in the compositions is from about 0.1 to about 70 wt.%, based on the total weight of the cosmetic composition. In further embodiment, the total amount of water is from 0.1 to about 60 wt.%, about 0.1 to about 50 wt.%, about 0.1 to about 40 wt.%, about 0.1 to about 30 wt.%, about 0.1 to about 20 wt.%, about 0.1 to about 10 wt.%, or about 0.1 to about 5 wt.%, based on the total weight of the cosmetic composition. In further preferred embodiments, the amount of water in the cosmetic composition is from 5 to about 50 wt.%, about 10 to about 45 wt.%, about 10 to about 40 wt.%, about 10 to about 35 wt.%, about 15 to about 50 wt.%, about 15 to about 45 wt.%, about 15 to about 40 wt.%, about 15 to about 35 wt.%, about 20 to about 50 wt.%, about 20 to about 45 wt.%, about 20 to about 40 wt.%, about 20 to about 35 wt.%, about 25 to about 50 wt.%, about 25 to about 45 wt.%, about 25 to about 40 wt.%, or about 25 to about 35 wt.%, based on the total weight of the cosmetic composition.

Nonlimiting examples of water-soluble solvents include glycerin, mono-alcohols, polyols (polyhydric alcohols), glycols, and a mixture thereof. In an embodiments, the one or more water-soluble solvents may be chosen from propylene glycol, butylene glycol, pentylene glycole, dipropylene glycol, ethanol, isopropanol, t-butyl alcohol, and a mixture thereof.

A more exhaustive but nonlimiting list of useful water-soluble solvents are provided under the heading "Water-Soluble Solvents."

The total amount of the one or more water-soluble solvents will vary but in certain embodiments is from about 0.1 to about 50 wt.%, based on the total weight of the cosmetic composition. In further embodiments, the total amount of the one or more water-soluble solvents may be from about 0.1 to about 40 wt.%, about 0.1 to about 30 wt.%, about 0.1 to about 20 wt., 0.1 to about 15 wt.%, about 0.1 to about 10 wt.%, about 0.1 to about 5 wt.%, about 1 to about 20 wt.%, about 1 to about 15 wt.%, about 1 to about 10 wt.%, or about 1 to about 5 wt.%, based on the total weight of the cosmetic composition.

In an embodiment, the cosmetic compositions may optionally include one or more miscellaneous ingredients. Nonlimiting examples of miscellaneous ingredients include miscellaneous emulsifiers/surfactants other than the dimethicone copolyol emulsifiers of (d), preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, and mixtures thereof. In an embodiment, the cosmetic composition include at least one further skin active agent, for example, madecassoside. A more exhaustive but nonlimiting list of miscellaneous ingredients is provided under the heading "Miscellaneous Ingredients"

Miscellaneous ingredients can be included in the cosmetic composition, for example, in an amount of about 0.01 to about 10 wt.%, based on the total weight of the cosmetic composition. The total amount of the one or more miscellaneous ingredients may be about 0.01 to about 8 wt.%, about 0.01 to about 5 wt.%, about 0.01 to about 3 wt.%, about 0.1 to about 10 wt.%, about 0.1 to about 8 wt.%, about 0.1 to about 5 wt.%, about 0.1 to about 3 wt.%, about 1 to about 10 wt.%, about 1 to about 8 wt.%, about 1 to about 5 wt.%, or about 1 to about 3 wt.%, based on the total weight of the cosmetic composition.

In an embodiment, the cosmetic compositions in the form of water in silicone emulsions include:

(a) about 10 to about 50 wt.% of hydroxypropyl tetrahydropyrantriol;

(b) about 5 to about 50 wt.% of one or more silicone oils;

(c) about 0.1 to about 10 wt.% of one or more non-emulsifying silicone elastomers;

(d) about 0.1 to about 10 wt.% of one or more dimethicone copolyol emulsifiers comprising at least one oxyethylene group and at least one oxypropylene group;

(e) water; and

(f) about 0.1 to about 40 wt.% of one or more water-soluble solvents;
wherein the composition is in the form of a water in silicone emulsion and all weight percentages are based on the total weight of the composition.

In certain embodiments, the cosmetic compositions in the form of water in silicone emlusions comprise or consist of:

(a) about 10 to about 50 wt.%, preferably about 15 to about 40 wt.%, more preferably about 25 to about 40 wt.% of hydroxypropyl tetrahydropyrantriol;
(b) about 10 to about 50 wt.%, preferably about 15 to about 40 wt.%, more preferably about 20 to about 30 wt.% of one or more silicone oils, preferably one or more silicone oils chosen from dimethicone, dimethiconol, cyclopentasiloxane, cyclomethicone, cyclotetrasiloxane, cyclohexasiloxane, cycloheptasiloxane, decamethylcyclopentasiloxane, cyclotetrasiloxane, cyclotrisiloxane, capryldimethicone, caprylyl trimethicone, caprylyl methicone, cetearylmethicone, hexadecylmethicone, hexylmethicone, lauryl methicone, myristyl methicone, phenyl methicone, stearyl methicone, stearyl dimethicone, behenyl dimethicone, trifluoropropyl methicone, cetyl dimethicone, polyphenylmethylsiloxane, dimethylpolysiloxane, methylphenylpolysiloxane, methyltrimethicone, diphenylsiloxyphenyl trimethicone, phenyl trimethicone, and mixtures thereof, more preferably dimethicone;
(c) about 0.1 to about 10 wt.%, preferably about 0.1 to about 5 wt.%, more preferably about 1 to about 5 wt.% of one or more non-emulsifying silicone elastomers, preferably chosen from dimethicone crosspolymers, (dimethicone/phenyl divinyl methicone) crosspolymers, (dimethicone/vinyl dimethicone/methicone) crosspolymers, (dimethicone/vinyl dimethicone) crosspolymers, and (diphenyl dimethicone/vinyl diphenyl dimethicone/silsesquioxane) crosspolymers, and mixtures thereof, more preferably chosen from dimethicone crosspolymer, vinyl dimethicone crosspolymer, dimethicone/vinyl dimethicone crosspolymer, dimethicone crosspolymer-3, polysilicone-11, and mixtures thereof;
(d) about 0.1 to about 10 wt.%, preferably about 0.1 to about 5 wt.%, of one or more dimethicone copolyol emulsifiers

comprising at least one oxyethylene group and at least one oxypropylene group, preferably one or more C8-C22 alkyl dimethicone copolyol emulsfiers comprising at least one oxyethylene group and at least one oxypropylene group, for example, bis-PEG/PPG-14/14 dimethicone, bis-isobutyl PEG/PPG-10/7 dimethicone copolymer, bis-PEG/PPG-18/6 dimethicone; bis-PEG/PPG-20/20 dimethicone, bis-PEG/PPG-16/16 dimethicone, cetyl PEG/PPG-15/16 butyl ether dimethicone, cetyl PEG/PPG-15/15 butyl ether dimethicone, cetyl PEG/PPG-7/3 dimethicone, cetyl PEG/PPG-10/1 dimethicone, dimethicone PEG/PPG-7/4 phosphate, dimethicone PEG/PPG-12/4 phosphate, PEG/PPG-28/21 acetate dimethicone, PEG/PPG-22/22 butyl ether dimethicone, PEG/PPG-23/23 butyl ether dimethicone, PEG/PPG-24/18 butyl ether dimethicone, PEG/PPG-3/10 dimethicone, PEG/PPG-4/12 dimethicone, PEG/PPG-6/11 dimethicone, PEG/PPG-8/14 dimethicone, PEG/PPG-12/16 dimethicone, PEG/PPG-12/18 dimethicone, PEG/PPG-14/4 dimethicone, PEG/PPG-15/5 dimethicone, PEG/PPG-15/15 dimethicone, PEG/PPG-16/2 dimethicone, PEG/PPG-16/8 dimethicone, PEG/PPG-17/18 dimethicone, PEG/PPG-18/12 dimethicone, PEG/PPG-19/19 dimethicone, PEG/PPG-20/6 dimethicone, PEG/PPG-20/15 dimethicone, PEG/PPG-20/20 dimethicone, PEG/PPG-20/29 dimethicone, PEG/PPG-22/23 dimethicone, PEG/PPG-22/24 dimethicone, PEG/PPG-25/25 dimethicone, PEG/PPG-27/27 dimethicone, PEG/PPG-30/10 dimethicone, PEG/PPG-10/3 oleyl ether dimethicone, and mixtures thereof, preferably one of the one or more dimethicone copolyol emulsifiers is dimethicone/PEG-10/15 crosspolymer;

wherein the weight ratio of (c) to (d) is from about 5:1 to about 1:5, preferably from about 4:1 to about 1:4, more preferably from about 3:1 to about 1:3, and even more preferably from about 2:1 to about 1:2;

(e) about 10 to about 50 wt.%, preferably about 15 to about 45 wt.% of one or more preferably about 25 to about 40 wt.% of water; and

(f) about 0.1 to about 20 wt.%, preferably about 0.1 to about 15 wt.%, more preferably about 1 to about 10 wt.% of one or more water-soluble solvents, preferably one or more water-soluble solvents chosen from glycerin, $C_1$-$C_5$ monoalcohols, polyols (polyhydric alcohols), glycols, and a mixture thereof, more preferably chosen from propylene glycol, butylene glycol, pentylene glycol, dipropylene glycol, ethanol, isopropanol, tert-butyl alcohol, and mixtures thereof;

(g) optionally, one or more miscellaneous ingredients, for example, miscellaneous ingredients chosen from miscellaneous emulsifiers/surfactants other than the dimethicone copolyol emulsifiers of (d), preservatives, fragrances, pH adjusters, salts, antioxidants, vitamins, botanical extracts, UV filtering agents, proteins, protein hydrolysates and/or isolates, hydrotropes, pearlescent agents, fillers, colorants, mattifying agents, further skin active agents, buffers, and mixtures thereof, preferably the one or more miscellaneous ingredient comprise one or more further skin active agents, wherein if present, the one or more miscellaneous ingredients may be in an amount of about 0.01 to about 10 wt.%, preferably about 0.1 to about 8 wt.%, more preferably about 1 to about 6 wt.%;

wherein the composition is in the form of a water in silicone emulsion and all weight percentages are based on the total weight of the composition.

In further embodiments, the cosmetic compositions in the form of water in silicone emulsions comprise or consist of:

(a) about 10 to about 50 wt.%, preferably about 15 to about 40 wt.%, more preferably about 25 to about 40 wt.% of hydroxypropyl tetrahydropyrantriol;

(b) about 10 to about 50 wt.%, preferably about 15 to about 40 wt.%, more preferably about 20 to about 30 wt.% of one or more silicone oils, preferably one or more silicone oils chosen from dimethicone, dimethiconol, cyclopentasiloxane, cyclomethicone, phenyl trimethicone, and mixtures thereof, more preferably dimethicone;

(c) about 0.1 to about 10 wt.%, preferably about 0.1 to about 5 wt.%, more preferably about 1 to about 5 wt.% of one or more non-emulsifying silicone elastomers, wherein the one or more non-emulsifying silicone elastomers are chosen from dimethicone crosspolymer, vinyl dimethicone crosspolymer, dimethicone/vinyl dimethicone crosspolymer, dimethicone crosspolymer-3, polysilicone-11, and mixtures thereof, preferably dimethicone crosspolymer;

(d) about 0.1 to about 10 wt.%, preferably about 0.1 to about 5 wt.%, of one or more dimethicone copolyol emulsifiers comprising at least one oxyethylene group and at least one oxypropylene group, preferably C8-C22 alkyl dimethicone copolyol emulsfiers comprising at least one oxyethylene group and at least one oxypropylene group, for example, bis-PEG/PPG-14/14 dimethicone, bis-isobutyl PEG/PPG-10/7 dimethicone copolymer, bis-PEG/PPG-18/6 dimethicone; bis-PEG/PPG-20/20 dimethicone, bis-PEG/PPG-16/16 dimethicone, cetyl PEG/PPG-15/16 butyl ether dimethicone, cetyl PEG/PPG-15/15 butyl ether dimethicone, cetyl PEG/PPG-7/3 dimethicone, cetyl PEG/PPG-10/1 dimethicone, dimethicone PEG/PPG-7/4 phosphate, dimethicone PEG/PPG-12/4 phosphate, PEG/PPG-28/21 acetate dimethicone, PEG/PPG-22/22 butyl ether dimethicone, PEG/PPG-23/23 butyl ether dimethicone, PEG/PPG-24/18 butyl ether dimethicone, PEG/PPG-3/10 dimethicone, PEG/PPG-4/12 dimethicone, PEG/PPG-6/11 dimethicone, PEG/PPG-8/14 dimethicone, PEG/PPG-12/16 dimethicone, PEG/PPG-12/18 dimethicone, PEG/PPG-14/4 dimethicone, PEG/PPG-15/5 dimethicone, PEG/PPG-15/15 dimethicone, PEG/PPG-16/2 dimethicone, PEG/PPG-16/8 dimethicone, PEG/PPG-17/18 dimethicone, PEG/PPG-18/12 dimethicone, PEG/PPG-19/19 dimethicone,

PEG/PPG-20/6 dimethicone, PEG/PPG-20/15 dimethicone, PEG/PPG-20/20 dimethicone, PEG/PPG-20/29 dimethicone, PEG/PPG-22/23 dimethicone, PEG/PPG-22/24 dimethicone, PEG/PPG-25/25 dimethicone, PEG/PPG-27/27 dimethicone, PEG/PPG-30/10 dimethicone, PEG/PPG-10/3 oleyl ether dimethicone, and mixtures thereof, preferably one of the one or more dimethicone copolyol emulsifiers is dimethicone/PEG-10/15 crosspolymer; wherein the weight ratio of (c) to (d) is from about 5:1 to about 1:5, preferably from about 4:1 to about 1:4, more preferably from about 3:1 to about 1:3, and even more preferably from about 2:1 to about 1:2;

(e) about 10 to about 50 wt.%, preferably about 15 to about 45 wt.%, of one or more preferably about 25 to about 40 wt.% of water; and

(f) about 0.1 to about 20 wt.%, preferably about 0.1 to about 15 wt.%, more preferably about 1 to about 10 wt.% of one or more water-soluble solvents, preferably one or more water-soluble solvents chosen from glycerin, $C_1$-$C_5$ monoalcohols, polyols (polyhydric alcohols), glycols, and a mixture thereof, more preferably chosen from propylene glycol, butylene glycol, pentylene glycol, dipropylene glycol, ethanol, isopropanol, tert-butyl alcohol, and mixtures thereof;

(g) about 0.01 to about 10 wt.%, preferably about 0.1 to about 8 wt.%, more preferably about 1 to about 6 wt.% of one or more miscellaneous ingredients, wherein at least one of the one or more miscellaneous ingredients is a further skin active agent, preferably, madecassoside;

wherein the composition is preferably a water in silicone emulsion and all percentages by weight are based on the total weight of the composition.

The cosmetic compositions are stable. For example, in an embodiment the cosmetic compositions do not visually phase separate or form visibly observable particulates for at least 2 weeks, 4 weeks, and/or 8 weeks in storage at 4°C, 25°C, 37°C, and/or 45°C.

In another embodiment, the cosmetic compositions do not visually phase separate or form visibly observable particulates for at least 10 cycles of freeze-thaw testing, wherein the freeze-thaw testing comprises placing the cosmetic composition in a stability chamber and subjecting it to temperature fluctuation at 12-hour intervals, for a first interval of 12 hours at -20°C followed by a second interval of 12 hours at 25°C.

In an embodiment, the viscosity of the cosmetic compositions does not change by more than 20%, 15%, 10%, or 5%, for at least 2 weeks, 4 weeks, and/or 8 weeks in storage at 4°C, 25°C, 37°C, and/or 45°C.

The cosmetic compositions preferably have a viscosity of about 5,000 to about 200,000 Pa.s at 25°C, and shear rate of 1 $s^{-1}$ at 25°C. However, in further embodiments, the compositions have a viscosity of about 10,000 to about 200,000 Pa.s, about 10,000 to about 180,000 Pa.s, about 10,000 to about 150,000 Pa.s, about 10,000 to about 120,000 Pa.s, about 10,000 to about 100,000 Pa.s, about 10,000 to about 80,000 Pa.s, about 15,000 to about 200,000 Pa.s, about 15,000 to about 180,000 Pa.s, about 15,000 to about 150,000 Pa.s, about 15,000 to about 120,000 Pa.s, about 15,000 to about 100,000 Pa.s, about 15,000 to about 80,000 Pa.s, about 20,000 to about 200,000 Pa.s, about 20,000 to about 180,000 Pa.s, about 20,000 to about 150,000 Pa.s, about 20,000 to about 120,000 Pa.s, about 20,000 to about 100,000 Pa.s, about 20,000 to about 80,000 Pa.s, about 30,000 to about 200,000 Pa.s, about 30,000 to about 180,000 Pa.s, about 30,000 to about 150,000 Pa.s, about 30,000 to about 120,000 Pa.s, about 30,000 to about 100,000 Pa.s,about 30,000 to about 80,000 Pa.s, about 35,000 to about 200,000 Pa.s, about 35,000 to about 180,000 Pa.s, about 35,000 to about 150,000 Pa.s, about 35,000 to about 120,000 Pa.s, about 35,000 to about 100,000 Pa.s, or about 35,000 to about 80,000 Pa.s at 25°C, and shear rate of 1 $s^{-1}$ at 25°C.

**Example 11**

**(Water in Silicone Emulsion with Hydroxypropyl Tetrahydropyrantriol)**

| | | | Inventive | | | Comparative | |
|---|---|---|---|---|---|---|---|
| | | | A | B | C | C-1 | C-2 |
| (a) | Active | HYDROXYPROPYL TETRAHYDROPYRANTRIOL | 30 | 30 | 30 | 30 | 30 |
| (b) | Silicone Oil | DIMETHICONE | 25.1 | 25.1 | 22.5 | 25.1 | 25.1 |
| (c) | Non-Emulsifying Silicone Elastomer | DIMETHICONE CROSSPOLYMER | 2 | 2 | 2 | | 2 |
| (d) | Dimethicone Copolyol Emulsifier | CETYL PEG/PPG-10/1 DIMETHICONE | 1.5 | 1.9 | 1.9 | 1.5 | |

(continued)

| Ratio (c):(d) | | | 1.3:1 | 1.1:1 | 1.1:1 | NA | NA |
|---|---|---|---|---|---|---|---|
| (f) | Water-Soluble Solvent | PROPYLENE GLYCOL | 3.4 | 3.4 | 3.4 | 3.4 | 3.4 |
| (9) | MADECASSOSIDE | | | | 0.9 | | |
| | LAUROYL LYSINE | | | | 1 | | |
| | Miscellaneous emulsifers/surfactants, salts, preservatives, pH adjusters, fragrances, colorants, antioxidants, chelants, and/or extracts, etc. | | ≤4 | ≤4 | ≤4 | ≤4 | ≤ 4 |
| (e) | WATER | | 35.4 | 35 | 35 | 37.3 | 36.9 |
| Stability | | | Y | Y | Y | N* | N* |
| * Comparative Compositions C-1 and C-2 did not form emulsions. | | | | | | | |

**Example 12**

**(Stability Testing)**

The compositions of Example 9 were subjected to stability studies and visually evaluated for phase separation and assessed under a microscope for particulate formation. The visual color change of the compositions was also evaluated. The compositions were assessed upon initial manufacture of the composition ($T_0$). The compositions were again evaluated after 10 days of freeze-thaw testing. The compositions were placed in a stability chamber and subjected to temperature fluctuation at 12-hour intervals. For 12 hours, the compositions were held at -20°C. For the next 12 hours, the compositions were held at 25°C. The cycle was repeated 10 times (for 10 days).

Separately, the compositions of Example 9 were evaluated after 4 weeks (1 month) in storage at 4°C, 25°C, 37°C, and 45°C and again at 8 weeks (2 months) at 4°C, 25°C, 37°C, and 45°C and visually evaluated for phase separation and assessed under a microscope for particulate formation.

Inventive Compositions A and B were deemed stable ("Y") (yes) because they did not visually phase separate, retained their color, and did not form particulates. Comparative Compositions C-1 and C-2 were not subjected to stability test because they failed to form emulsions that could be further tested.

***Water in Silicone Emulsions Comprising Hydroxypropyl Tetrahydropyrantriol***

In certain embodiments, the cosmetic compositions in the form of water in silicone emulsion include hydroxypropyl tetrahydropyrantriol. In an embodiment, the amount of hydroxypropyl tetrahydropyrantriol in the cosmetic compositions is from about 15 wt.% to about 60 wt.% based on the total weight of the composition. In various embodiments, the amount of hydroxypropyl tetrahydropyrantriol in the composition is from about 15 to about 55 wt.%, about 15 to about 50 wt.%, about 15 to about 45 wt%, about 15 to about 40 wt.%, about 15 to about 35 wt.%, about 15 to about 30 wt.%, about 15 to about 25 wt.%, about 20 to about 60 wt.%, about 20 to about 55 wt.%, about 20 to about 50 wt.%, about 20 to about 45 wt.%, about 20 to about 40 wt.%, about 20 to about 35 wt.%, about 20 to about 30 wt.%, about 25 to about 60 wt.%, about 25 to about 55 wt.%, about 25 to about 50 wt.%, about 25 to about 45 wt.%, about 25 to about 40 wt.%, about 25 to about 35 wt.%, about 30 to about 60 wt.%, about 30 to about 55 wt.%, about 30 to about 50 wt.%, about 30 to about 45 wt.%, about 30 to about 40 wt.%, about 35 to about 55 wt.%, about 35 to about 60 wt.%, about 35 to about 50 wt.%, about 35 to about 45 wt.%, about 40 to about 60 wt.%, about 40 to about 55 wt.%, about 40 to about 50 wt.%, about 45 to about 55 wt.%, or about 50 to 60 wt.%, based on the total weight of the cosmetic composition.

In certain embodiments, the cosmetic compositions in the form of water in silicone emulsions include:

(a) hydroxypropyl tetrahydropyrantriol, preferably in the amounts set forth above;

(b) water;

(d) one or more silicone oils;

(d) one or more crosslinked emulsifying silicone elastomers; and

(e) one or more co-emulsifier silicones chosen from dimethicone copolyols;

wherein all percentages by weight are based on the total weight of the composition.

The amount of water in the cosmetic compositions will vary depending on the amount of the other components in the cosmetic compositions. Nonetheless, in certain embodiments, the amount of water in the composition can is from about 10 to about 60 wt.%, based on the total weight of the cosmetic composition. In further embodiments, the amount of water in the cosmetic composition is from about 10 to about 55 wt.%, about 10 to about 50 wt.%, about 15 to about 60 wt.%, about 15 to about 55 wt.%, about 15 to about 50 wt.%, about 15 to about 45 wt.%, about 15 to about 40 wt.%, about 15 to about 30 wt.%, about 15 to about 25 wt.%, about 20 to about 60 wt.%, about 20 to about 55 wt.%, about 20 to about 50 wt.%, about 20 to about 45 wt.%, about 20 to about 40 wt.%, about 30 to about 50 wt.%, based on the total weight of the cosmetic composition.

Nonlimiting examples of silicone oils include dimethicone, dimethiconol, cyclopentasiloxane, cyclomethicone, cyclotetrasiloxane, cyclohexasiloxane, cycloheptasiloxane, decamethylcyclopentasiloxane, cyclotetrasiloxane, cyclotrisiloxane, capryldimethicone, caprylyl trimethicone, caprylyl methicone, cetearylmethicone, hexadecylmethicone, hexylmethicone, lauryl methicone, myristyl methicone, phenyl methicone, stearyl methicone, stearyl dimethicone, behenyl dimethicone, trifluoropropyl methicone, cetyl dimethicone, polyphenylmethylsiloxane, dimethylpolysiloxane, methylphenylpolysiloxane, methyltrimethicone, diphenylsiloxyphenyl trimethicone, and phenyl trimethicone, and mixtures thereof. A more exhaustive but nonlimiting list of useful silicone oils is included under the heading "Silicone Oils."

The total amount of silicone oils in the cosmetic compositions will vary but in certain embodiments is from about 5 to about 25 wt.%, based on the total weight of the cosmetic composition. In further embodiments, the total amount of the one or more silicone oils is from about 5 to about 20 wt.%, about 5 to about 15 wt.%, about 5 to about 12 wt.%, about 6 to about 25 wt.%, about 6 to about 20 wt.%, about 6 to about 15 wt.%, or about 6 to about 12 wt.%, based on the total weight of the cosmetic composition.

Nonlimiting examples of crosslinked emulsifying silicone elastomers include polyoxyalkylenated emulsifying silicone elastomers, polyglycerolated emulsifying silicone elastomers, and mixtures thereof. Further, nonlimiting examples of polyoxyalkylenated emulsifying silicone elastomers include dimethicone/PEG-10/15 crosspolymer, PEG-15 lauryl dimethicone crosspolymer, PEG-15 lauryl dimethicone crosspolymer, PEG-15 lauryl dimethicone crosspolymer, PEG-10 lauryl dimethicone crosspolymer, PEG-15 lauryl dimethicone crosspolymer, PEG-12 dimethicone crosspolymers, PEG- 3 to PEG-32 dimethicones (e.g., PEG-10 dimethicone), dimethicone/dimethicone PEG/PPG 15 crosspolymer, dimethicone PEG-10 crosspolymer, dimethicone PEG-10/15 crosspolymer, dimethicone PEG-15 crosspolymer, dimethicone polyglycerin-3 crosspolymer, dimethicone PPG-20 crosspolymer, lauryl dimethicone PEG-15 crosspolymer, lauryl dimethicone polyglycerin-3 crosspolymer, PEG-8 dimethicone polysorbate-20 crosspolymer, PEG-10 dimethicone/vinyl dimethicone crosspolymer, PEG-10 lauryl dimethicone crosspolymer, PEG-15/lauryl dimethicone crosspolymer, PEG-15 laurylpolydimethylsiloxy ethyl crosspolymer, and mixtures thereof. In an embodiment, the cosmetic compositions may include two or more polyoxyalkylenated emulsifying silicone elastomers. A more exhaustive but nonlimiting list of crosslinked emulsifying silicone elastomers is provided under the heading "Crosslinked Emulsifying Silicone Elastomers."

The total amount of the one or more crosslinked emulsifying silicone elastomers will vary but in certain embodiments is about 0.1 to about 10 wt.%, based on the total weight of the cosmetic composition. In further embodiments, the total amount of the one or more crosslinked emulsifying silicone elastomer is from about 0.1 to about 8 wt.%, about 0.1 to about 5 wt.%, about 0.1 to about 4 wt.%, about 0.1 to about 3 wt.%, about 0.5 to about 10 wt.%, about 0.5 to about 8 wt.%, about 0.5 to about 5 wt.%, about 0.5 to about 3 wt.%, about 1 to about 10 wt.%, about 1 to about 8 wt.%, about 1 to about 5 wt.%, or about 1 to about 3 wt.%, based on the total weight of the cosmetic composition.

Nonlimiting examples of co-emulsifier silicones chosen that are dimethicone copolyols include dimethicone PEG-8 benzoate, dimethicone PEG-7 Phosphate, dimethicone PEG-8 phosphate, dimethicone PEG-10 phosphate, PEG-7 dimethicone, PEG-8 dimethicone, PEG-9 dimethicone, PEG-10 dimethicone, PEG-12 dimethicone, PEG-14 dimethicone, PEG-17 dimethicone, PEG/PPG-3/10 dimethicone, PEG/PPG-4/12 dimethicone, PEG/PPG-17/18 dimethicone, cetyl PEG/PPG-10/1 dimethicone, and mixtures thereof. PEG-10 dimethicone is particularly preferred. A more exhaustive but nonlimiting list of co-emulsifier silicones is provided under the heading "Co-Emulsifying Silicones."

The total amount of the optional one or more co-emulsifying silicones, if present, will vary but in certain embodiments is from about 0.01 to about 5 wt.%, based on the total weight of the cosmetic composition. In further embodiments, the total amount of the one or more co-emulsifying silicones is about 0.01 to about 4 wt.%, about 0.01 to about 3 wt.%, about 0.01 to about 2 wt.%, about 0.1 to about 5 wt.%, about 0.1 to about 4 wt.%, about 0.1 to about 3 wt.%, or about 0.1 to about 2 wt.%, based on the total weight of the cosmetic composition.

In an embodiment, the cosmetic compositions include one or more water soluble solvents. Nonlimiting examples of water-soluble solvents include glycerin, mono-alcohols, polyols (polyhydric alcohols), glycols, and a mixture thereof. In an embodiment, the one or more water-soluble solvents may be chosen from propylene glycol, butylene glycol, pentylene glycole, dipropylene glycol, ethanol, isopropanol, t-butyl alcohol, and a mixture thereof. A more exhaustive but nonlimiting list of useful water-soluble solvents are provided under the heading "Water-Soluble Solvents."

The total amount of the one or more water-soluble solvents can and will vary but in certain embodiments is from

about 1 to about 45 wt.%, based on the total weight of the cosmetic composition. In various embodiments, the total amount of the one or more water-soluble solvents is from about 1 to about 40 wt.%, about 1 to about 35 wt.%, about 1 to about 30 wt.%, about 3 to about 45 wt.%, about 3 to about 40 wt.%, about 3 to about 35 wt.%, about 3 to about 30 wt.%, about 3 to about 25 wt.%, about 5 to about 45 wt.%, about 5 to about 40 wt.%, about 5 to about 35 wt.%, based on the total weight of the cosmetic composition.

In an embodiment, the cosmetic compositions include one or more silicone powders. Nonlimiting examples include are chosen from (dimethicone/vinyl dimethicone) crosspolymer, (dimethicone/phenyl vinyl dimethicone) crosspolymer, (vinyl dimethicone/lauryl dimethicone) crosspolymer, (vinyl dimethicone/methicone silsesquioxane) crosspolymer, (diphenyl dimethicone/vinyl diphenyl dimethicone/silsesquioxane) crosspolymer, polysilicone-1 crosspolymer, polysilicone-22, polyalkylsilsesquioxane (for example, polymethylsilsesquioxane), polysilicone-11, and polyarylsilsesquioxane, and mixtures thereof.

The silicone powders can be added to the cosmetic compositions but do not typically remain as powders after incorporation into the cosmetic compositions. The silicone powders will generally be solubilized after incorporation into the cosmetic compositions. Nonlimiting examples of silicone powders include (dimethicone/vinyl dimethicone) crosspolymer, (dimethicone/phenyl vinyl dimethicone) crosspolymer, (vinyl dimethicone/lauryl dimethicone) crosspolymer, (vinyl dimethicone/methicone silsesquioxane) crosspolymer, (diphenyl dimethicone/vinyl diphenyl dimethicone/silsesquioxane) crosspolymer, polysilicone-1 crosspolymer, polysilicone-22, polyalkylsilsesquioxane (for example, polymethylsilsesquioxane). Preferred silicone powders include polyalkylsilsesquioxane (for example, polymethylsilsesquioxane), vinyl dimethicone/methicone silsesquioxane, polysilicone-11, and mixtures thereof.

The total amount of the one or more silicone powders in the cosmetic compositions will vary but in certain embodiments is from about 0.1 to about 5 wt.%, based on the total weight of the cosmetic composition. In further embodiments, the total amount of the one or more silicone powders is from about 0.1 to about 4 wt.%, about 0.1 to about 3 wt.%, about 0.1 to about 2 wt.%, about 0.5 to about 5 wt.%, about 0.5 to about 4 wt.%, about 0.5 to about 3 wt.%, about 0.5 to about 2 wt.%, about 1 to about 5 wt.%, about 1 to about 4 wt.%, about 1 to about 3 wt.%, or about 1 to about 2 wt.%, based on the total weight of the cosmetic composition.

In an embodiment, the cosmetic compositions may optionally include one or more miscellaneous ingredients. Nonlimiting examples of miscellaneous ingredients include miscellaneous emulsifiers/surfactants other than the dimethicone copolyol emulsifiers of (d), preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, and mixtures thereof. In an embodiment, the cosmetic composition include at least one further skin active agent, for example, madecassoside. A more exhaustive but nonlimiting list of miscellaneous ingredients is provided under the heading "Miscellaneous Ingredients"

Miscellaneous ingredients can be included in the cosmetic composition, for example, in an amount of about 0.01 to about 10 wt.%, based on the total weight of the cosmetic composition. The total amount of the one or more miscellaneous ingredients may be about 0.01 to about 8 wt.%, about 0.01 to about 5 wt.%, about 0.01 to about 3 wt.%, about 0.1 to about 10 wt.%, about 0.1 to about 8 wt.%, about 0.1 to about 5 wt.%, about 0.1 to about 3 wt.%, about 1 to about 10 wt.%, about 1 to about 8 wt.%, about 1 to about 5 wt.%, or about 1 to about 3 wt.%, based on the total weight of the cosmetic composition.

In an embodiment, the cosmetic compositions in the form of water in silicone emulsions comprise or consist of:

(a) about 10 to about 55 wt.%, preferably about 20 to about 55 wt.%, more preferably about 30 to about 50 wt.% of hydroxypropyl tetrahydropyrantriol;

(b) about 15 to about 50 wt.%, preferably about 20 to about 50 wt.%, more preferably about 25 to about 50 wt.% of water;

(c) about 5 to about 25 wt.% of one or more silicone oils, preferably one or more non-volatile silicone oils, more preferably one or more silicone oils chosen from dimethicone, dimethiconol, cyclomethicone, polysilicone-11, phenyl trimethicone, amodimethicone, and mixtures thereof, preferably one of the one or more silicone oils is dimethicone;

(d) about 0.1 to about 10 wt.%, preferably about 0.5 to about 5 wt.%, more preferably about 1 to about 4 wt.% of one or more crosslinked emulsifying silicone elastomers, preferably one or more polyoxyalkylenated emulsifying silicone elastomers, more preferably selected from dimethicone/PEG-10/15 crosspolymer, PEG-15 lauryl dimethicone crosspolymer, PEG-15 lauryl dimethicone crosspolymer, PEG-15 lauryl dimethicone crosspolymer, PEG-10 lauryl dimethicone crosspolymer, PEG-15 lauryl dimethicone crosspolymer, PEG-12 dimethicone crosspolymers, dimethicone/dimethicone PEG/PPG 15 crosspolymer, dimethicone PEG-10 crosspolymer, dimethicone PEG-10/15 crosspolymer, dimethicone PEG-15 crosspolymer, dimethicone polyglycerin-3 crosspolymer, dimethicone PPG-20 crosspolymer, lauryl dimethicone PEG-15 crosspolymer, lauryl dimethicone polyglycerin-3 crosspolymer, PEG-8 dimethicone polysorbate-20 crosspolymer, PEG-10 dimethicone/vinyl dimethicone crosspolymer, PEG-10 lauryl dimethicone crosspolymer, PEG-15/lauryl dimethicone crosspolymer, PEG-15 laurylpolydimethylsiloxy ethyl crosspolymer, and mixtures thereof, even more preferably dimethicone/PEG-10/15 crosspolymer;

(e) about 0.01 to about 5 wt.%, preferably about 0.01 to about 3 wt.%, more preferably about 0.1 to about 2 wt.% of one or more co-emulsifying silicones chosen from dimethicone copolyols, for example, dimethicone copolyols chosen from dimethicone PEG-8 Benzoate, dimethicone PEG-7 Phosphate, dimethicone PEG-8 phosphate, dimethicone PEG-10 phosphate, PEG-7 dimethicone, PEG-8 dimethicone, PEG-9 dimethicone, PEG-10 dimethicone, PEG-12 dimethicone, PEG-14 dimethicone, PEG-17 dimethicone, PEG/PPG-3/10 dimethicone, PEG/PPG-4/12 dimethicone, PEG/PPG-17/18 dimethicone, cetyl PEG/PPG-10/1 dimethicone, and mixtures thereof, in particular PEG-10 Dimethicone;

(f) optionally, one or more water-soluble solvents, for example, glycerin, $C_2$-$C_6$ mono-alcohols, polyols (polyhydric alcohols), glycols, and a mixture thereof, wherein if present, the one or more water-soluble solvents may optionally be in an amount of about 5 to about 45 wt.%, preferably about 10 to about 40 wt.%, more preferably about 15 to about 35 wt.%;

(g) optionally, of one or more silicone powders, for example, (dimethicone/vinyl dimethicone) crosspolymer, (dimethicone/phenyl vinyl dimethicone) crosspolymer, (vinyl dimethicone/lauryl dimethicone) crosspolymer, (vinyl dimethicone/methicone silsesquioxane) crosspolymer, (diphenyl dimethicone/vinyl diphenyl dimethicone/silsesquioxane) crosspolymer, polysilicone-1 crosspolymer, polysilicone-22, polyalkylsilsesquioxane (for example, polymethylsilsesquioxane). Preferred silicone powders include polyalkylsilsesquioxane (for example, polymethylsilsesquioxane), vinyl dimethicone/methicone silsesquioxane, polysilicone-11, and mixtures thereof, wherein if present, may be in an amount of about 0.1 to about 5 wt.%, preferably about 0.1 to about 4 wt.%, more preferably about 0.5 to about 3 wt.%;

(h) optionally, one or more miscellaneous ingredients, for example, miscellaneous ingredients chosen from miscellaneous emulsifiers/surfactants different from the crosslinked emulsifying silicone elastomers of (d), preservatives, fragrances, pH adjusters, salts, antioxidants, vitamins, botanical extracts, UV filtering agents, proteins, protein hydrolysates and/or isolates, hydrotropes, pearlescent agents, fillers, colorants, mattifying agents, further skin active agents, buffers, and mixtures thereof, wherein if present, may be in an amount of about 0.01 to about 10 wt.%, preferably about 0.1 to about 8 wt.%, more preferably about 1 to about 6 wt.%;
wherein the composition is preferably water in silicone emulsion and all percentages by weight are based on the total weight of the composition.

In an embodiment, the cosmetic compositions in the form of water in silicone emulsions comprise or consist of:

(a) about 10 to about 55 wt.%, preferably about 20 to about 55 wt.%, more preferably about 30 to about 50 wt.% of hydroxypropyl tetrahydropyrantriol;

(b) about 15 to about 50 wt.%, preferably about 20 to about 50 wt.%, more preferably about 25 to about 50 wt.% of water;

(c) about 5 to about 25 wt.% of one or more silicone oils, preferably one or more non-volatile silicone oils, more preferably one or more silicone oils chosen from dimethicone, dimethiconol, cyclomethicone, polysilicone-11, phenyl trimethicone, and amodimethicone, in particular dimethicone;

(d) about 0.1 to about 10 wt.%, preferably about 0.5 to about 5 wt.%, more preferably about 1 to about 4 wt.% of one or more crosslinked emulsifying silicone elastomers, preferably one or more polyoxyalkylenated emulsifying silicone elastomers, more preferably selected from dimethicone/PEG-10/15 crosspolymer, PEG-15 lauryl dimethicone crosspolymer, PEG-15 lauryl dimethicone crosspolymer, PEG-15 lauryl dimethicone crosspolymer, PEG-10 lauryl dimethicone crosspolymer, PEG-15 lauryl dimethicone crosspolymer, PEG-12 dimethicone crosspolymers, dimethicone/dimethicone PEG/PPG 15 crosspolymer, dimethicone PEG-10 crosspolymer, dimethicone PEG-10/15 crosspolymer, dimethicone PEG-15 crosspolymer, dimethicone polyglycerin-3 crosspolymer, dimethicone PPG-20 crosspolymer, lauryl dimethicone PEG-15 crosspolymer, lauryl dimethicone polyglycerin-3 crosspolymer, PEG-8 dimethicone polysorbate-20 crosspolymer, PEG-10 dimethicone/vinyl dimethicone crosspolymer, PEG-10 lauryl dimethicone crosspolymer, PEG-15/lauryl dimethicone crosspolymer, PEG-15 laurylpolydimethylsiloxy ethyl crosspolymer,

and mixtures thereof, even more preferably dimethicone/PEG-10/15 crosspolymer;

(e) about 0.01 to about 5 wt.%, preferably about 0.01 to about 3 wt.%, more preferably about 0.1 to about 2 wt.% of one or more co-emulsifying silicones chosen from dimethicone copolyols, for example, dimethicone copolyols chosen from dimethicone PEG-8 Benzoate, dimethicone PEG-7 Phosphate, dimethicone PEG-8 phosphate, dimethicone PEG-10 phosphate, PEG-7 dimethicone, PEG-8 dimethicone, PEG-9 dimethicone, PEG-10 dimethicone, PEG-12 dimethicone, PEG-14 dimethicone, PEG-17 dimethicone, PEG/PPG-3/10 dimethicone, PEG/PPG-4/12 dimethicone, PEG/PPG-17/18 dimethicone, cetyl PEG/PPG-10/1 dimethicone, and mixtures thereof, in particular PEG-10 Dimethicone;

(f) about 5 to about 45 wt.%, preferably about 10 to about 40 wt.%, more preferably about 15 to about 35 wt.% of one or more water-soluble solvents, for example, glycerin, $C_2$-$C_6$ mono-alcohols, polyols (polyhydric alcohols), glycols, and a mixture thereof;

(g) about 0.1 to about 5 wt.%, preferably about 0.1 to about 4 wt.%, more preferably about 0.5 to about 3 wt.% of one or more silicone powders, for example, (dimethicone/vinyl dimethicone) crosspolymer, (dimethicone/phenyl vinyl dimethicone) crosspolymer, (vinyl dimethicone/lauryl dimethicone) crosspolymer, (vinyl dimethicone/methicone silsesquioxane) crosspolymer, (diphenyl dimethicone/vinyl diphenyl dimethicone/silsesquioxane) crosspolymer, polysilicone-1 crosspolymer, polysilicone-22, polyalkylsilsesquioxane (for example, polymethylsilsesquioxane), polysilicone-11, and mixtures thereof, preferably, polyalkylsilsesquioxane (for example, polymethylsilsesquioxane), vinyl dimethicone/methicone silsesquioxane, polysilicone-11, and mixtures thereof, more preferably polymethylsilsesquioxane;

(h) about 0.01 to about 10 wt.%, preferably about 0.1 to about 8 wt.%, more preferably about 1 to about 6 wt.%, of one or more miscellaneous ingredients, for example, miscellaneous ingredients chosen from miscellaneous emulsifiers/surfactants different from the crosslinked emulsifying silicone elastomers of (d), preservatives, fragrances, pH adjusters, salts, antioxidants, vitamins, botanical extracts, UV filtering agents, proteins, protein hydrolysates and/or isolates, hydrotropes, pearlescent agents, fillers, colorants, mattifying agents, further skin active agents, buffers, and mixtures thereof;
wherein the composition is preferably a water in silicone emulsion and all percentages by weight are based on the total weight of the composition.

The cosmetic compositions are stable. For example, in an embodiment the cosmetic compositions do not visually phase separate or form visibly observable particulates for at least 2 weeks, 4 weeks, and/or 8 weeks in storage at 4°C, 25°C, 37°C, and/or 45°C.
In another embodiment, the cosmetic compositions do not visually phase separate or form visibly observable particulates for at least 10 cycles of freeze-thaw testing, wherein the freeze-thaw testing comprises placing the cosmetic composition in a stability chamber and subjecting it to temperature fluctuation at 12-hour intervals, for a first interval of 12 hours at -20°C followed by a second interval of 12 hours at 25°C.
In an embodiment, the viscosity of the cosmetic compositions does not change by more than 20%, 15%, 10%, or 5%, for at least 2 weeks, 4 weeks, and/or 8 weeks in storage at 4°C, 25°C, 37°C, and/or 45°C.
The cosmetic compositions preferably have a viscosity of about 5,000 to about 200,000 Pa.s at 25°C, and shear rate of 1 $s^{-1}$ at 25°C. However, in further embodiments, the compositions have a viscosity of about 10,000 to about 200,000 Pa.s, about 10,000 to about 180,000 Pa.s, about 10,000 to about 150,000 Pa.s, about 10,000 to about 120,000 Pa.s, about 10,000 to about 100,000 Pa.s, about 10,000 to about 80,000 Pa.s, about 15,000 to about 200,000 Pa.s, about 15,000 to about 180,000 Pa.s, about 15,000 to about 150,000 Pa.s, about 15,000 to about 120,000 Pa.s, about 15,000 to about 100,000 Pa.s, about 15,000 to about 80,000 Pa.s, about 20,000 to about 200,000 Pa.s, about 20,000 to about 180,000 Pa.s, about 20,000 to about 150,000 Pa.s, about 20,000 to about 120,000 Pa.s, about 20,000 to about 100,000 Pa.s, about 20,000 to about 80,000 Pa.s, about 30,000 to about 200,000 Pa.s, about 30,000 to about 180,000 Pa.s, about 30,000 to about 150,000 Pa.s, about 30,000 to about 120,000 Pa.s, about 30,000 to about 100,000 Pa.s,about 30,000 to about 80,000 Pa.s, about 35,000 to about 200,000 Pa.s, about 35,000 to about 180,000 Pa.s, about 35,000 to about 150,000 Pa.s, about 35,000 to about 120,000 Pa.s, about 35,000 to about 100,000 Pa.s, or about 35,000 to about 80,000 Pa.s at 25°C, and shear rate of 1 $s^{-1}$ at 25°C.

## Example 13

**(Water in Silicone Emulsions with Hydroxypropyl Tetrahydropyrantriol)**

| | | | A | B | C | D | E | F | G | C-1 |
|---|---|---|---|---|---|---|---|---|---|---|
| (a) | Active | HYDROXYPROPYL TETRAHYDROPYRANTRIOL | 10 | 20 | 30.4 | 47.4 | 50.1 | 50.1 | 50.1 | 15 |
| (c) | Silicone Oil | DIMETHICONE | 10.7 | 10.7 | 10.7 | 10.7 | 10.9 | 6.5 | 8.0 | 71.8 |
| (d) | Crosslinked Emulsifying Silicone Elastomer | DIMETHICONE/PEG-10/15 CROSSPOLYMER | 1.3 | 1.3 | 1.3 | 1.3 | 1.3 | 1.3 | 2.0 | 1.3 |
| (e) | Co-Emulsifier | PEG-10 DIMETHICONE | 0.1 | 0.1 | 0.1 | 0.1 | 0.1 | 0.1 | 0.1 | 0.1 |
| (f) | Water-Soluble Solvent | GLYCERIN, PROPANEDIOL, PROPYLENE GLYCOL, DIPROPYLENE GLYCOL, AND/OR ETHANOL | 27.1 | 29.3 | 23.5 | 20.4 | 5.7 | 5.7 | 10.7 | 1.7 |
| (g) | Silicone Powder | POLYMETHYLSILSESQUIOXANE | 1 | 0.5 | 1 | 0.5 | 0.5 | 0.5 | 0.5 | 0.5 |
| (h) | Miscellaneous emulsifers/surfactants, Emolliants, Salts, Preservatives, pH Adjusters, Fragrances, Colorants, Chelants, and/or Extracts, Etc. | | ≤4 | ≤4 | ≤4 | ≤4 | ≤4 | ≤4 | ≤4 | ≤ 4 |
| (b) | WATER | | 48 | 36 | 31 | 18 | 29 | 34 | 27 | 8 |
| **Stable** | | | **Y** | **Y** | **Y** | **Y** | **Y** | **Y** | **Y** | **N*** |
| *N - The composition (C-1) phase separated within 2 hours | | | | | | | | | | |

**Example 14**

**(Stability Testing)**

The compositions of Example 13 were subjected to stability studies and visually evaluated for phase separation and assessed under a microscope for particulate formation. The compositions were analyzed upon initial manufacture of the composition ($T_0$). The compositions were again analyzed after 10 days of freeze-thaw testing. For freeze-thaw testing, the compositions were placed in a stability chamber and subjected to temperature fluctuation at 12-hour intervals. For 12 hours, the compositions were held at -20°C. For the next 12 hours, the compositions were held at 25°C. The cycle was repeated 10 times (for 10 days). Separately, the compositions of Example 1 were evaluated after 4 weeks (1 month) in storage at 4°C, 25°C, 37°C, and 45°C and again at 8 weeks (2 months) at 4°C, 25°C, 37°C, and 45°C and visually evaluated for phase separation and assessed under a microscope for particulate formation. Viscosity was also evaluated.

The inventive compositions were deemed stable ("Y") (yes) because they did not visually phase separate, did not form particulates, and retained viscosity within at least 10% (viscosity change was not more than ± 10%). Comparative Composition C-1 was deemed not stable ("N") (no) because it phase separated within 2 hours after formation.

**ANHYDROUS COMPOSITIONS**

The anhydrous compositions of the instant case are substantially free from water, i.e., they contain less than 2 wt.% of water. In some instances, however, the anhydrous compositions contain less than 1 wt.% of water, less than 0.5 wt.% of water, less than 0.1 wt.% of water, or less than 0.05 wt.% of water. The anhydrous compositions are further characterized by having at least 90 wt.% of ingredients with a log P value of 2 or less, for example, 1.5 or less, 1 or less, 0.75 or less, 0.5 or less, 0.25 less, or 0. The log P value for a compound is the logarithm (base 10) of the partition coefficient (P), which is defined as the ratio of the compound's organic (oil)-to-aqueous phase concentrations:

**Partition Coefficient (P) = [Organic] / [Aqueous], where [ ] is the compound's concentration in that solvent phase log P = Log 10 (P)**

The log P of a compound is constant for a given specific pair of aqueous and organic solvents, and its value can be determined empirically by one of several phase-partitioning methods known in the art. However, the calculated log P value for a compound in water vs. a simple organic compound like octanol or hexane can provide a guideline for predicting its solubility characteristics in other aqueous and organic solvents. For example, a compound with a measured log P equal to 1 indicates that its concentration is at a 10:1 ratio in organic to aqueous phase. This compound is hydrophobic and requires dissolution in an organic solvent. In contrast, a compound with a log P equal to -1 indicates that the concentration is at a 1:10 ratio in organic to aqueous phase. This compound is hydrophilic and can be dissolved directly in an aqueous buffer. Finally, a compound with a log P equal to 0 partitions at a 1:1 ratio in organic to aqueous phase; hence, the compound is likely soluble in both organic and aqueous solvent.

The anhydrous compositions include one or more active agents, preferably one or more active agents that are soluble in the anhydrous composition. Nonlimiting examples of useful active agents include ascorbic acid, ferulic acid, or a mixture thereof.

In an embodiment the anhydrous compositions include less than 2 wt.% water, preferably less than 1 wt.% water, and more preferably less than 0.1 wt.% of water, and:

(a) one or more active agents, preferably one or more active agents that are soluble in the anhydrous compositions;

(b) one or more water-soluble solvents;

(c) one or more thickening polymers; and

(d) optionally, one or mor miscellaneous ingredients.

The total amount of the one or more active agents in the anhydrous composition may vary but is typically from about 1 to about 25 wt.%, based on the total weight of the anhydrous composition. In various embodiments, the total amount of the one or more active agents in the anhydrous compositions is from about 1 to about 20 wt.%, about 1 to about 18 wt.%, about 1 to about 15 wt.%, about 2 to about 20 wt.%, about 2 to about 18 wt.%, about 2 to about 15 wt.%, about 5 to about 20 wt.%, about 5 to about 18 wt.%, or about 5 to about 15 wt.%, based on the total weight of the anhydrous composition.

In a preferred embodiment, the anhydrous composition includes ascorbic acid. The total amount of ascorbic acid in the anhydrous composition can vary but is typically from about 1 to about 20 wt.%, based on the total weight of the anhydrous composition. In various embodiments, the total amount of ascorbic acid in the anhydrous composition is from about 1 to about 18 wt.%, about 1 to about 15 wt.%, about 2 to about 20 wt.%, about 2 to about 18 wt.%, about 2 to about 15 wt.%, about 5 to about 20 wt.%, about 5 to about 18 wt.%, or about 5 to about 15 wt.%, based on the total weight of the anhydrous composition.

In a preferred embodiment, the anhydrous composition includes ferulic acid. The total amount of ferulic acid in the anhydrous composition can vary but is typically from about 0.1 to about 10 wt.%, based on the total weight of the anhydrous composition. In various embodiments, the total amount of ferulic acid is from about 0.1 to about 8 wt.%, about 0.1 to about 5 wt.%, about 0.5 to about 10 wt.%, about 0.5 to about 8 wt.%, about 0.5 to about 5 wt.%, about 1 to about 10 wt.%, about 1 to about 8 wt.%, or about 1 to about 5 wt.%, based on the total weight of the anhydrous composition.

Nonlimiting examples of water-soluble solvents include glycerin, mono-alcohols, polyols (polyhydric alcohols), glycols, and a mixture thereof. In an embodiments, the one or more water-soluble solvents may be chosen from propylene glycol, butylene glycol, pentylene glycole, dipropylene glycol, ethanol, isopropanol, t-butyl alcohol, and a mixture thereof. A more exhaustive but nonlimiting list of useful water-soluble solvents are provided under the heading "Water-Soluble Solvents."

The total amount of the one or more water-soluble solvents will vary but in various embodiment is from about 50 to about 95 wt.%, based on the total weight of the anhydrous composition. In further embodiments, the total amount of the one or more water-soluble solvents is from about 60 to about 95 wt.%, about 70 to about 95 wt.%, about 75 to about 95 wt.%, about 60 to 90 wt.%, about 70 to about 90 wt.%, about 80 to about 90 wt.%.

Useful thickening polymers include, among others, taurate copolymers. Nonlimiting examples include acrylamide/sodium acryloyl dimethyl taurate copolymer, hydroxyethyl acrylate/sodium acryloyl dimethyl taurate copolymer, ammonium acryloyldimethyl taurateNP copolymer, sodium acrylate/sodium acryloyl dimethyl taurate copolymer, hydroxyethyl acrylate/ sodium acryloyldimethyl taurate copolymer, and mixtures thereof. A more exhaustive but nonlimiting list of useful thickening polymers are included under the heading "Thickening Polymers."

Generally, the amount of the one or more thickening polymers will vary depending on the type of thickening polymers used; and depending on the desired viscosity of the cosmetic composition. Therefore, in an embodiment, the total amount of the one or more thickening agents is sufficient to achieve the viscosities set forth throughout the instant

disclosure. Nonetheless, in various embodiments, the total amount of the one or more thickening polymers may range from about 0.01 to about 8 wt.%, based on the total weight of the cosmetic composition. In various embodiments, the amount of the one or more thickening polymers may range from about 0.01 to about 6 wt%, from about 0.01 to about 5 wt%, from about 0.01 to about 3 wt%, from 0.1 to about 8 wt.%, about 0.1 to about 5 wt.%, about 0.1 to about 3 wt.%, or about 0.1 to about 2 wt.%, based on the total weight of the anhydrous composition.

In certian embodiments, the anhydrous compositions include one or more miscellaneous ingredients. Non-limiting examples include e preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle agents, etc. A more exhaustive but nonlimiting list of miscellaneous ingredients that may be included in the anhydrous composition is provided under the heading "Miscellaneous Ingredients."

The total amount of the one or more miscellaneous ingredients, if present, will vary but in certain embodiments is in an amount of about 0.01 to about 10 wt.%, based on the total weight of the anhydrous composition. The total amount of the one or more miscellaneous ingredients may be about 0.01 to about 8 wt.%, about 0.01 to about 5 wt.%, about 0.01 to about 3 wt.%, about 0.1 to about 10 wt.%, about 0.1 to about 8 wt.%, about 0.1 to about 5 wt.%, about 0.1 to about 3 wt.%, about 1 to about 10 wt.%, about 1 to about 8 wt.%, about 1 to about 5 wt.%, or about 1 to about 3 wt.%, based on the total weight of the anhydrous composition.

**Example 15**

**(Anhydrous Compositions with Ascorbic Acid and Ferulic Acid)**

| | | A | B |
|---|---|---|---|
| Active | ASCORBIC ACID | 12 | 12 |
| Active | FERULIC ACID | 1.5 | 3.0 |
| Thickening Polymer | AMMONIUM POLYACRYLOYLDIMETHYL TAURATE | 0.3 | 0.2 |
| Water-Soluble Solvent | GLYCERIN | 23.2 | 43.2 |
| | PROPYLENE GLYCOL | 48 | 14.7 |
| | DIPROPYLENE GLYCOL | 13.5 | 24.5 |
| | BUTYLENE GLYCOL | | 2.5 |
| Extract | POLYGONUM CUSPIDATUM ROOT EXTRACT | 1.5 | |
| WATER | | ≤ 0.01 | ≤ 0.01 |

**Water-Soluble Solvents**

The term "water-soluble solvent" is interchangeable with the term "water-miscible solvent" and means a compound that is liquid at 25°C and at atmospheric pressure (760 mmHg), and it has a solubility of at least 50% in water under these conditions. In an embodiment, the water-soluble solvents have a solubility of at least 60%, 70%, 80%, or 90%. Non-limiting examples of one or more water-soluble solvents are chosen from glycerin, mono-alcohols, polyols (polyhydric alcohols), glycols, and a mixture thereof.

As nonlimiting examples of organic solvents, mention can be made of monoalcohols and polyols such as ethyl alcohol, isopropyl alcohol, propyl alcohol, isopropyl alcohol, benzyl alcohol, 4-tert-butylcyclohexanol, and phenylethyl alcohol, or glycols or glycol ethers such as, for example, monomethyl, monoethyl and monobutyl ethers of ethylene glycol, propylene glycol or ethers thereof such as, for example, monomethyl ether of propylene glycol, butylene glycol, hexylene glycol, dipropylene glycol as well as alkyl ethers of diethylene glycol, for example monoethyl ether or monobutyl ether of diethylene glycol. Other suitable examples of organic solvents are ethylene glycol, propylene glycol, butylene glycol, hexylene glycol, propane diol, and glycerin. The organic solvents can be volatile or non-volatile compounds.

Further non-limiting examples of water-soluble solvents include alkanediols (polyhydric alcohols) such as glycerin, 1,2,6-hexanetriol, trimethylolpropane, ethylene glycol, propylene glycol, diethylene glycol, triethylene glycol, tetraethylene glycol, pentaethylene glycol, dipropylene glycol, 2-butene-1,4-diol, 2-ethyl-1,3-hexanediol, 2-methyl-2,4-pentanediol, (caprylyl glycol), 1,2-hexanediol, 1,2-pentanediol, and 4-methyl-1,2-pentanediol; alkyl alcohols having 1 to 4

carbon atoms such as ethanol, methanol, butanol, propanol, and isopropanol; glycol ethers such as ethylene glycol monomethyl ether, ethylene glycol monoethyl ether, ethylene glycol monobutyl ether, ethylene glycol monomethyl ether acetate, diethylene glycol monomethyl ether, diethylene glycol monoethyl ether, diethylene glycol mono-n-propyl ether, ethylene glycol mono-iso-propyl ether, diethylene glycol mono-iso-propyl ether, ethylene glycol mono-n-butyl ether, ethylene glycol mono-t-butyl ether, diethylene glycol mono-t-butyl ether, 1-methyl-1-methoxybutanol, propylene glycol monomethyl ether, propylene glycol monoethyl ether, propylene glycol mono-t-butyl ether, propylene glycol mono-n-propyl ether, propylene glycol mono-iso-propyl ether, dipropylene glycol monomethyl ether, dipropylene glycol monoethyl ether, dipropylene glycol mono-n-propyl ether, and dipropylene glycol mono-iso-propyl ether; 2-pyrrolidone, N-methyl-2-pyrrolidone, 1,3-dimethyl-2-imidazolidinone, formamide, acetamide, dimethyl sulfoxide, sorbit, sorbitan, acetine, diacetine, triacetine, sulfolane, and a mixture thereof.

Polyhydric alcohols are also useful. Examples of polyhydric alcohols include glycerin, ethylene glycol, diethylene glycol, triethylene glycol, propylene glycol, dipropylene glycol, tripropylene glycol, 1,3-butanediol, 2,3-butanediol, 1,4-butanediol, 3-methyl-1,3-butanediol, 1,5-pentanediol, tetraethylene glycol, 1,6-hexanediol, 2-methyl-2,4-pentanediol, polyethylene glycol, 1,2,4-butanetriol, 1,2,6-hexanetriol, and a mixture thereof. Polyol compounds may also be used. Non-limiting examples include the aliphatic diols, such as 2-ethyl-2-methyl-1,3-propanediol, 3,3-dimethyl-1,2-butanediol, 2,2-diethyl-1,3-propanediol, 2-methyl-2-propyl-1,3-propanediol, 2,4-dimethyl-2,4-pentanediol, 2,5-dimethyl-2,5-hexanediol, 5-hexene-1,2-diol, and 2-ethyl-1,3-hexanediol, and a mixture thereof.

In an embodiment, the cosmetic compositions of the instant disclosure include one or more glycols and/or one or more alcohols, for example, one or more water-soluble solvents selected from propylene glycol, butylene glycol, pentylene glycol, ethanol, isopropanol, tert-butyl alcohol, and mixtures thereof.

**Fatty Compounds**

The term "fatty compounds" is interchangeable with the "fatty materials." Fatty compounds are known as compounds that are not soluble (or only sparingly soluble) in water; they are hydrophilic and are often solubilized in organic solvents. They include materials such as oils, fats, waxes, hydrocarbons, fatty alcohols, fatty acids, fatty esters, etc. Silicones are not considered fatty compounds according to the instant disclosure. Non-limiting examples of useful fatty compounds include oils, waxes, alkanes (paraffins), fatty alcohols, fatty acids, fatty esters, triglyceride compounds, lanolin, hydrocarbons, derivatives thereof, and mixtures thereof. Fatty compounds are described by the International Federation Societies of Cosmetic Chemists, for example, in Cosmetic Raw Material Analysis and Quality, Volume I: Hydrocarbons, Glycerides, Waxes and Other Esters (Redwood Books, 1994).

Non-limiting examples of fatty compounds include oils, mineral oil, alkanes (paraffins), fatty acids, fatty alcohol derivatives, fatty acid derivatives, esters of fatty alcohols, hydroxy-substituted fatty acids, waxes, triglyceride compounds, lanolin, and a mixture thereof.

*Fatty Alcohol Derivatives*

The fatty compounds may include one or more fatty alcohol derivatives, which are different from fatty alcohols (component (d)). Fatty alcohol derivatives include fatty esters derived from one or more fatty alcohols. Fatty alcohol derivatives also include alkoxylated fatty alcohols, e.g., having about 1 to about 100 moles of an alkylene oxide per mole of alkoxylated fatty alcohol. For example, the alkoxylated fatty alcohols may be alkoxylated with about 1 to about 80 moles, about 2 to about 50, about 5 to about 45 moles, about 10 to about 40 moles, or 15 to about 35 mores, including all ranges and subranges therebetween, of an alkylene oxide per mole of alkoxylated fatty alcohol.

As examples of alkoxylated fatty alcohols, steareth (for example, steareth-2, steareth-20, and steareth-21), laureth (for example, laureth-4, and laureth-12), ceteth (for example, ceteth-10 and ceteth-20) and ceteareth (for example, ceteareth-2, ceteareth-10, and ceteareth-20) are mentioned. In at least one instance, the one or more alkoxylated fatty alcohols include steareth-20. In some instances, the one or more alkoxylated fatty alcohols may be exclusively steareth-20.

Additional fatty alcohol derivatives that may, optionally be suitable include methyl stearyl ether; 2-ethylhexyl dodecyl ether; stearyl acetate; cetyl propionate; the ceteth series of compounds, such as ceteth-1 through ceteth-45, which are ethylene glycol ethers of cetyl alcohol, wherein the numeric designation indicates the number of ethylene glycol moieties present; the steareth series of compounds such as steareth-1 through 10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, i.e. a mixture of fatty alcohols containing predominantly cetyl and stearyl alcohol, wherein the numeric designation indicates the number of ethylene glycol moieties present; C1-C30 alkyl ethers of the ceteth, steareth, and ceteareth compounds just described; polyoxyethylene ethers of branched alcohols such as octyldodecyl alcohol, dodecylpentadecyl alcohol, hexyldecyl alcohol, and isostearyl alcohol; polyoxyethylene ethers of behenyl alcohol; PPG ethers such as PPG-9-steareth-3, PPG-11 stearyl ether, PPG8-ceteth-1,

and PPG-10 cetyl ether; and a mixture thereof.

*Fatty Acids*

In some instances, the fatty compounds may be chosen from fatty acids, fatty acid derivatives, esters of fatty acids, hydroxyl-substituted fatty acids, and alkoxylated fatty acids. The fatty acids may be straight or branched chain acids and/or may be saturated or unsaturated. Non-limiting examples of fatty acids include diacids, triacids, and other multiple acids as well as salts of these fatty acids. For example, the fatty acid may optionally include or be chosen from lauric acid, palmitic acid, stearic acid, behenic acid, arichidonic acid, oleic acid, isostearic acid, sebacic acid, and a mixture thereof. In some cases, the fatty acids are selected from the group consisting of palmitic acid, stearic acid, and a mixture thereof.

Non-limiting examples of polyglycerol esters of fatty acids include those of the following formula:

$$R^1\text{-}(OCH_2\text{-}\underset{}{\overset{OR^2}{\overset{|}{C}}}H\text{-}CH_2O)_n\text{-}R^3$$

wherein the average value of n is about 3 and $R^1$, $R^2$ and $R^3$ each may independently be a fatty acid moiety or hydrogen, provided that at least one of $R^1$, $R^2$, and $R^3$ is a fatty acid moiety. For instance, $R^1$, $R^2$ and $R^3$ may be saturated or unsaturated, straight or branched, and have a length of $C_1$-$C_{40}$, $C_1$-$C_{30}$, $C_1$-$C_{25}$, or $C_1$-$C_{20}$, $C_1$-$C_{16,\ or}$ $C_1$-$C_{10}$.

The fatty acid derivatives are defined herein to include fatty acid esters of the fatty alcohols as defined above, fatty acid esters of the fatty alcohol derivatives as defined above when such fatty alcohol derivatives have an esterifiable hydroxyl group, fatty acid esters of alcohols other than the fatty alcohols and the fatty alcohol derivatives described above, hydroxy-substituted fatty acids, and a mixture thereof. Non-limiting examples of fatty acid derivatives include ricinoleic acid, glycerol monostearate, 12-hydroxy stearic acid, ethyl stearate, cetyl stearate, cetyl palmitate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, dimethyl sebacate, PEG-15 cocoate, PPG-15 stearate, glyceryl monostearate, glyceryl distearate, glyceryl tristearate, PEG-8 laurate, PPG-2 isostearate, PPG-9 laurate, and a mixture thereof. Preferred for use herein are glycerol monostearate, 12-hydroxy stearic acid, and a mixture thereof.

*Fatty Alcohols*

The term "fatty alcohol" means an alcohol comprising at least one hydroxyl group (OH), and comprising at least 8 carbon atoms, and which is neither oxyalkylenated (in particular neither oxyethylenated nor oxypropylenated) nor glycerolated. The fatty alcohols can be represented by: R-OH, wherein R denotes a saturated (alkyl) or unsaturated (alkenyl) group, linear or branched, comprising from 8 to 40 carbon atoms, preferably 10 to 30 carbon atoms, more preferably 12 to 24 carbon atoms, and even more preferably 14 to 22 carbon atoms.

The fatty alcohol(s) may be liquid or solid. In some instances, it is preferable that the cosmetic compositions include at least one solid fatty alcohol. The solid fatty alcohols that can be used include those that are solid at ambient temperature and at atmospheric pressure (25°C, 780 mmHg), and are insoluble in water, that is to say they have a water solubility of less than 1% by weight, preferably less than 0.5% by weight, at 25°C, 1 atm.

The solid fatty alcohols may be represented by: R-OH, wherein R denotes a linear alkyl group, optionally substituted with one or more hydroxyl groups, comprising from 8 to 40 carbon atoms, preferably 10 to 30 carbon atoms, more preferably 12 to 24 carbon atoms, and even more preferably 14 to 22 carbon atoms.

Non-limiting examples of useful fatty alcohols include lauryl alcohol or lauryl alcohol (1-dodecanol); myristic or myristyl alcohol (1-tetradecanol); cetyl alcohol (1-hexadecanol); stearyl alcohol (1-octadecanol); arachidyl alcohol (1-eicosanol); behenyl alcohol (1-docosanol); lignoceryl alcohol (1-tetracosanol); ceryl alcohol (1-hexacosanol); montanyl alcohol (1-octacosanol); myricylic alcohol (1-triacontanol), and mixtures thereof.

In certain embodiments, the one or more fatty alcohols have from 12 to 24 carbon atoms. Specific nonlimiting examples include cetyl alcohol, stearyl alcohol, cetearyl alcohol, behenyl alcohol, lauryl alcohol, myristic or myristyl alcohol, arachidyl alcohol, lignoceryl alcohol, or mixtures thereof.

Preferably, the cosmetic composition includes one or more solid fatty alcohols, for example, chosen from cetyl alcohol, stearyl alcohol, behenyl alcohol and mixtures thereof, preferably cetyl alcohol, behenyl alcohol, cetearyl alcohol, and mixtures thereof.

The liquid fatty alcohols, in particular those containing C10-C34, preferably have branched carbon chains and/or have one or more, preferably 1 to 3 double bonds. They are preferably branched and/or unsaturated (C=C double bond) and contain from 12 to 40 carbon atoms.

The liquid fatty alcohols may be represented by: R-OH, wherein R denotes a C12-C24 branched alkyl group or an alkenyl group (comprising at least one C12-C24 double bond C=C), R being optionally substituted by a or more hydroxy groups. Preferably, the liquid fatty alcohol is a branched saturated alcohol. Preferably, R does not contain a hydroxyl group. These include oleic alcohol, linoleic alcohol, linolenic alcohol, isocetyl alcohol, isostearyl alcohol, 2-octyl-1-dodecanol, 2-butyloctanol, 2-hexyl- 1-decanol, 2-decyl-1-tetradecanol, 2-tetradecyl-1-cetanol and mixtures thereof. Preferably, the liquid fatty alcohol is 2-octyl-1-dodecanol.

In some instances, the cosmetic compositions include one or more fatty alcohols selected from decyl alcohol, undecyl alcohol, dodecyl, myristyl, cetyl alcohol, stearyl alcohol, cetearyl alcohol, isostearyl alcohol, isocetyl alcohol, behenyl alcohol, linalool, oleyl alcohol, myricyl alcohol and a mixture thereof. In some instances, the cosmetic compositions preferably include cetyl alcohol, behenyl alcohol, and cetearyl alcohol.

*Waxes*

The fatty compounds may, in some instances, include or be chosen from one or more waxes. Non-limiting examples of waxes in this category include for example, synthetic wax, ceresin, paraffin, ozokerite, polyethylene waxes, illipe butter, beeswax, carnauba, microcrystalline, lanolin, lanolin derivatives, candelilla, cocoa butter, shellac wax, spermaceti, bran wax, capok wax, sugar cane wax, montan wax, whale wax, bayberry wax, acacia decurrents flower wax, vegetable waxes (such as sunflower seed (helianthus annuus), carnauba, candelilla, ouricury or japan wax or cork fibre or sugarcane waxes), or a mixture thereof.

*Oils*

In some instances, the fatty compounds may include or be chosen from one or more oil(s). Suitable oils include, but are not limited to, natural oils, such as coconut oil; hydrocarbons, such as mineral oil and hydrogenated polyisobutene; fatty alcohols, such as octyldodecanol; esters, such as $C_{12}$-$C_{15}$ alkyl benzoate; diesters, such as propylene dipelarganate; and triesters, such as glyceryl trioctanoate. Non-limiting examples of oils that may, optionally, be included in the cosmetic compositions include isotridecyl isononanoate, PEG-4 diheptanoate, isostearyl neopentanoate, tridecyl neopentanoate, cetyl octanoate, cetyl palmitate, cetyl ricinoleate, cetyl stearate, cetyl myristate, coco-dicaprylate/caprate, decyl isostearate, isodecyl oleate, isodecyl neopentanoate, isohexyl neopentanoate, octyl palmitate, dioctyl malate, tridecyl octanoate, myristyl myristate, octododecanol, or combinations of octyldodecanol, acetylated lanolin alcohol, cetyl acetate, isododecanol, polyglyceryl-3-diisostearate, castor oil, lanolin and lanolin derivatives, triisocetyl citrate, sorbitan sesquioleate, $C_{10}$-$C_{18}$ triglycerides, caprylic/capric/triglycerides, coconut oil, corn oil, cottonseed oil, glyceryl triacetyl hydroxystearate, glyceryl triacetyl ricinoleate, glyceryl trioctanoate, hydrogenated castor oil, linseed oil, mink oil, olive oil, palm oil, illipe butter, rapeseed oil, soybean oil, sunflower seed oil, tallow, tricaprin, trihydroxystearin, triisostearin, trilaurin, trilinolein, trimyristin, triolein, tripalmitin, tristearin, walnut oil, wheat germ oil, cholesterol, or combinations thereof.

In some embodiments, the cosmetic composition may include one or more fatty compounds chosen from fatty esters (such as isononyl isononanoate), polyolefins (such as petrolatum), waxes, squalane, squalene, hydrogenated polyisobutene, hydrogenated polydecene, polybutene, mineral oil, pentahydrosqualene, plant and/or vegetable oil, hydrocarbon-based oils (such as isohexadecane), or a mixture thereof.

**Thickening Polymers**

Non-limiting examples of various types of thickening polymers include taurate copolymer, polyacrylate, polymethacrylate, polyethylacrylate, polyacrylamide, poly C10-30 alkyl acrylate, acrylic acid/acrylonitrogens copolymer, acrylates/steareth-20 itaconate copolymer, acrylates/ceteth-20 itaconate copolymer, Acrylates/Aminoacrylates/C10-30 Alkyl PEG-20 Itaconate Copolymer, acrylates/aminoacrylates copolymer, acrylates/steareth-20 methacrylate copolymer, acrylates/beheneth-25 methacrylate copolymer, acrylates/steareth-20 methacrylate crosspolymer, acrylates/beheneth-25 methacrylate/HEMA crosspolymer, acrylates/vinyl neodecanoate crosspolymer, acrylates/vinyl isodecanoate crosspolymer, Acrylates/Palmeth-25 Acrylate Copolymer, Acrylic Acid/Acrylamidomethyl Propane Sulfonic Acid Copolymer, and acrylates/C10-C30 alkyl acrylate crosspolymer, carbomers, hydrophobically modified polypolyacrylates; hydrophobically modified polyacrylic acids, hydrophobically modified polyacrylamides; hydrophobically modified polyethers wherein these materials may have a hydrophobe that can be selected from cetyl, stearyl, oleayl, and combinations thereof, acrylamide/ammonium acrylate copolymer, acrylates copolymer, Acrylates Crosspolymer-4, Acrylates Crosspolymer-3, acrylates/beheneth-25 methacrylate copolymer, acrylates/C10-C30 alkyl acrylate crosspolymer, acrylates/-steareth-20 itaconate copolymer, ammonium polyacrylate/lsohexadecane/PEG-40 castor oil; sodium carbomer, crosslinked polyvinylpyrrolidone (PVP), polyacrylamide/C13-14 isoparaffin/laureth-7, polyacrylate 13/polyisobutene/polysorbate 20, polyacrylate crosspolymer-6, polyamide-3, polyquaternium-37, sodium polyacrylate, and a mixture thereof.

Among the nonionic thickening polymers examples include:

(1) Celluloses modified with groups comprising at least one fatty chain; examples that may be mentioned include: hydroxyethylcelluloses modified with groups comprising at least one fatty chain, such as alkyl, arylalkyl or alkylaryl groups, or mixtures thereof, and in which the alkyl groups are preferably C8-C22, for instance the product NATROSOL PLUS GRADE 330 CS ($C_{16}$ alkyls) sold by the company Aqualon, or the product BERMOCOLL EHM 100 sold by the company Berol Nobel; and hydroxyethylcelluloses modified with alkylphenyl polyalkylene glycol ether groups, such as the product AMERCELL POLYMER HM-1500 (polyethylene glycol (15) nonylphenyl ether) sold by the company Amerchol,

(2) Hydroxypropyl guars modified with groups comprising at least one fatty chain, such as the product ESAFLOR HM 22 ($C_{22}$ alkyl chain) sold by the company Lamberti, and the products RE210-18 ($C_{14}$ alkyl chain) and RE205-1 ($C_{20}$ alkyl chain) sold by the company Rhone-Poulenc,

(3) Copolymers of vinylpyrrolidone and of fatty-chain hydrophobic monomers; examples that may be mentioned include: the products ANTARON V216 or GANEX V216 (vinylpyrrolidone/hexadecene copolymer) sold by the company I.S.P. the products ANTARON V220 or GANEX V220 (vinylpyrrolidone/eicosene copolymer) sold by the company I.S.P.,

(4) Copolymers of $C_1$-$C_6$ alkyl methacrylates or acrylates and of amphiphilic monomers comprising at least one fatty chain, for instance the oxyethylenated methyl acrylate/stearyl acrylate copolymer sold by the company Goldschmidt under the name ANTIL 208,

(5) Copolymers of hydrophilic methacrylates or acrylates and of hydrophobic monomers comprising at least one fatty chain, for instance the polyethylene glycol methacrylate/lauryl methacrylate copolymer,

(6) Polyurethane polyethers comprising in their chain both hydrophilic blocks usually of polyoxyethylenated nature and hydrophobic blocks, which may be aliphatic sequences alone and/or cycloaliphatic and/or aromatic sequences.

In a preferred embodiment, the cosmetic composition includes one or more taurate copolymers. These copolymers can act as gelling agents, thickeners, and provide emulsification properties. In particular, the inventors discovered that taurate copolymers are particularly effective for stabilizing the cosmetic compositions of the instant disclosure.

Nonlimiting examples taurate copolymers include acrylamide/sodium acryloyl dimethyl taurate copolymer, hydroxyethyl acrylate/sodium acryloyl dimethyl taurate copolymer, ammonium acryloyldimethyl taurateNP copolymer, sodium acrylate/sodium acryloyl dimethyl taurate copolymer, hydroxyethyl acrylate/ sodium acryloyldimethyl taurate copolymer, and a mixture thereof

The taurate copolymers may be hydrophilic and may contain an acrylate component. The at least one taurate copolymer may include, for example, acrylamide/sodium acryloyl dimethyl taurate copolymer, hydroxyethyl acrylate/-sodium acryloyl dimethyl taurate copolymer, and/or sodium acrylate/sodium acryloyl dimethyl taurate copolymer. In some instances, at least one taurate copolymer is obtainable from ethylenically unsaturated, sulpho-functional monomers and ethylenically unsaturated hydrophilic monomers, for example from crosslinked anionic copolymers of acrylamide or methacrylamide and of 2-acrylamido-2-methylpropanesulfonic acid.

In some instances, the one or more taurate copolymers may be chosen from acrylamide/sodium acryloyl dimethyl taurate copolymer, hydroxyethyl acrylate/sodium acryloyl dimethyl taurate copolymer, ammonium acryloyldimethyl taurate/VP copolymer, and mixtures thereof. Furthermore, in some instances, the cosmetic compositions may include both hydroxyethyl acrylate/sodium acryloyl dimethyl taurate copolymer and ammonium acryloyldimethyl taurateNP copolymer, and optionally further include poly C10-30 alkyl acrylate.

**Nonionic Emulsifiers**

Nonlimiting examples of nonionic emulsifiers include ethoxylated sorbitan fatty esters (such as polysorbate 80), polyol esters, glyceryl esters, alkyl polyglucosides (such as ceteraryl glucoside), glycerol ethers, oxyethylenated ethers, oxypropylenated ethers, and ethylene glycol polymers. In addition, the nonionic emulsifiers may be chosen from alkyl polyglucosides; alcohols, alpha-diols, alkylphenols and esters of fatty acids, being ethoxylated, propoxylated or glycerolated (polyglyceryl-2 isostearate); ethoxylated fatty esters; glyceryl esters of fatty acids; fatty alcohol ethoxylates; alkyl phenol ethoxylates; fatty acid alkoxylates; and mixtures thereof.

The nonionic emulsifiers may be chosen from alcohols and alpha-diols, these compounds being polyethoxylated and/or polypropoxylated and/or polyglycerolated, the number of ethylene oxide and/or propylene oxide groups possibly ranging from 2 to 100, and the number of glycerol groups possibly ranging from 2 to 30; these compounds comprising at

least one fatty chain comprising from 8 to 30 carbon atoms and especially from 16 to 30 carbon atoms.

Mention is also be made of polyethoxylated fatty amides preferably having from 2 to 30 ethylene oxide units, polyglycerolated fatty amides including on average from 1 to 5, and in particular from 1.5 to 4, glycerol groups; polyoxyethylenated fatty acid esters of sorbitan having preferably from 2 to 40 units of ethylene oxide, fatty acid esters of sucrose, polyoxyalkylenated and preferably polyoxyethylenated fatty acid esters containing from 2 to 150 mol of ethylene oxide, such as oxyethylenated plant oils.

Useful nonionic surfactants include those of the alkyl(poly)glycoside type, represented especially by the following general formula: $R_1O-(R_2O)_t-(G)_v$ in which: $R_1$ represents a linear or branched alkyl or alkenyl substituent comprising 6 to 24 carbon atoms and especially 8 to 18 carbon atoms, or an alkylphenyl substituent whose linear or branched alkyl substituent comprises 6 to 24 carbon atoms and especially 8 to 18 carbon atoms; $R_2$ represents an alkylene substituent comprising 2 to 4 carbon atoms; G represents a sugar unit comprising 5 to 6 carbon atoms; t denotes a value ranging from 0 to 10 and preferably 0 to 4; and v denotes a value ranging from 1 to 15 and preferably 1 to 4. Preferably, the alkyl(poly)glycoside surfactants are compounds of the formula described above in which: $R_1$ denotes a linear or branched, saturated or unsaturated alkyl substituent comprising from 8 to 18 carbon atoms; $R_2$ represents an alkylene substituent comprising 2 to 4 carbon atoms; t denotes a value ranging from 0 to 3 and preferably equal to 0; and G denotes glucose, fructose or galactose, preferably glucose; the degree of polymerization, *i.e.* the value of v, possibly ranging from 1 to 15 and preferably from 1 to 4; the mean degree of polymerization more particularly being between 1 and 2. The glucoside bonds between the sugar units are generally of 1-6 or 1-4 type and preferably of 1-4 type. In particular, the alkyl(poly)glycoside surfactant may be an alkyl(poly)glucoside surfactant $C_8/C_{16}$ alkyl(poly)glucosides 1,4, and in particular decyl glucosides and caprylyl/capryl glucosides.

Useful nonionic surfactants may be chosen from polyoxyethylenated C8-C30 fatty acid esters (preferably C12-C18) of sorbitan, polyethoxylated C8-C30 (preferably C12-18) fatty alcohols, polyglycerolated C8-C30 (preferably C12-C18) fatty acid esters, polyoxyethylenated compounds having preferably from 2 to 30 moles of ethylene oxide, polyglycerolated compounds having preferably from 2 to 16 moles of glycerol; and mixtures thereof.

The polyoxyethylenated C8-C30 fatty alcohols may be chosen from C12-C18 fatty alcohols, in particular polyoxyethylenated lauryl alcohol, cetyl alcohol, myristyl alcohol, and stearyl alcohol having from 2 to 30 mol of ethylene oxide, such as: cetyl alcohol polyoxyethylenated with 6 EO (Ceteth-6) (HLB 11.1) cetyl alcohol polyoxyethylenated with 10 EO (Ceteth-10) (HLB 12.9) cetyl alcohol polyoxyethylenated with 20 EO (Ceteth-20) (HLB 15.7) cetyl alcohol polyoxyethylenated with 24 EO (Ceteth-24) (HLB 16.3 lauryl alcohol polyoxyethylenated with 4 EO (Laureth-4) (HLB 9.4) lauryl alcohol polyoxyethylenated with 7 EO (Laureth-7) (HLB 12.3) lauryl alcohol polyoxyethylenated with 9 EO (Laureth-9) (HLB 13.6) lauryl alcohol polyoxyethylenated with 10 EO (Laureth-10) (HLB 13.9) lauryl alcohol polyoxyethylenated with 12 EO (Laureth-12) (HLB 14.6) lauryl alcohol polyoxyethylenated with 21 EO (Laureth-21) (HLB 15.5) lauryl alcohol polyoxyethylenated with 23 EO (Laureth-23) (HLB 16.3) stearyl alcohol polyoxyethylenated with 2 EO (Steareth-2) (HLB 4.9) stearyl alcohol polyoxyethylenated with 10 EO (Steareth-10) (HLB 12.4) stearyl alcohol polyoxyethylenated with 20 EO (Steareth-20) (HLB 15.2) stearyl alcohol polyoxyethylenated with 21 EO (Steareth-21) (HLB 15.5)

The polyoxyethylenated C8-C30 fatty acid esters (preferably C12-C18) of sorbitan may be chosen from polyoxyethylenated esters of C12-C18 fatty acids, in particular lauric, myristic, cetylic or stearic acids, of sorbitan especially containing from 2 to 30 mol of ethylene oxide, such as: polyoxyethylenated sorbitan monolaurate (4 EO) (Polysorbate-21) (HLB 13.3) polyoxyethylenated sorbitan monolaurate (20 EO) (Polysorbate-20) (HLB 16.7) polyoxyethylenated sorbitan monopalmitate (20 EO) (Polysorbate-40) (HLB 15.6) polyoxyethylenated sorbitan monostearate (20 EO) (Polysorbate-60) (HLB 14.9) polyoxyethylenated sorbitan monostearate (4 EO) (Polysorbate-61) (HLB 9.6) polyoxyethylenated sorbitan monooleate (20 EO) (Polysorbate-80) (HLB 15).

In a preferred embodiment, the cosmetic compositions include one or more nonionic surfactants chosen from polyoxyethylenated C8-C30 fatty acid esters (preferably C12-C18) of sorbitan, preferably polyoxyethylenated esters of C12-C18 fatty acids.

The polyglycerolated C8-C30 fatty acid esters, which are particularly preferred, may be chosen from polyglycerolated esters of C12-C18 fatty acids, in particular lauric, myristic, palmitic, stearic or isostearic acid, having from 2 to 16 mol of glycerol, such as: polyglyceryl-2 laurate, polyglyceryl-3 laurate, polyglyceryl-4 laurate, polyglyceryl-5 laurate, polyglyceryl-6 laurate, polyglyceryl-10 laurate; polyglyceryl-2 myristate, polyglyceryl-3 myristate, polyglyceryl-4 myristate, polyglyceryl-5 myristate, polyglyceryl-6 myristate, polyglyceryl-10 myristate; polyglyceryl-2 palmitate, polyglyceryl-3 palmitate, polyglyceryl-6 palmitate, polyglyceryl-10 palmitate; polyglyceryl-2 isostearate, polyglyceryl-3 isostearate, polyglyceryl-4 isostearate, polyglyceryl-5 isostearate, polyglyceryl-6 isostearate, polyglyceryl-10 isostearate; polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, and mixtures thereof.

In some embodiments, the nonionic surfactant may be selected from esters of polyols with fatty acids with a saturated or unsaturated chain containing for example from 8 to 24 carbon atoms, preferably 12 to 22 carbon atoms, and alkoxylated derivatives thereof, preferably with a number of alkyleneoxide of from 10 to 200, and more preferably from 10 to 100, such as glyceryl esters of a $C_8$-$C_{24}$, preferably $C_{12}$-$C_{22}$, fatty acid or acids and alkoxylated derivatives thereof,

preferably with a number of alkyleneoxide of from 10 to 200, and more preferably from 10 to 100; polyethylene glycol esters of a $C_8$-$C_{24}$, preferably $C_{12}$-$C_{22}$, fatty acid or acids and alkoxylated derivatives thereof, preferably with a number of alkyleneoxide of from 10 to 200, and more preferably from 10 to 100; sorbitol esters of a $C_8$-$C_{24}$, preferably $C_{12}$-$C_{22}$, fatty acid or acids and alkoxylated derivatives thereof, preferably with a number of alkyleneoxide of from 10 to 200, and more preferably from 10 to 100; sugar (sucrose, glucose, alkylglycose) esters of a $C_8$-$C_{24}$, preferably $C_{12}$-$C_{22}$, fatty acid or acids and alkoxylated derivatives thereof, preferably with a number of alkyleneoxide of from 10 to 200, and more preferably from 10 to 100; ethers of fatty alcohols; ethers of sugar and a $C_8$-$C_{24}$, preferably $C_{12}$-$C_{22}$, fatty alcohol or alcohols; and mixtures thereof.

Examples of ethoxylated fatty esters that may be mentioned include the adducts of ethylene oxide with esters of lauric acid, palmitic acid, stearic acid or behenic acid, and mixtures thereof, especially those containing from 9 to 100 oxyethylene groups, such as PEG-9 to PEG-50 laurate (as the CTFA names: PEG-9 laurate to PEG-50 laurate); PEG-9 to PEG-50 palmitate (as the CTFA names: PEG-9 palmitate to PEG-50 palmitate); PEG-9 to PEG-50 stearate (as the CTFA names: PEG-9 stearate to PEG-50 stearate); PEG-9 to PEG-50 palmitostearate; PEG-9 to PEG-50 behenate (as the CTFA names: PEG-9 behenate to PEG-50 behenate); polyethylene glycol 100 EO monostearate (CTFA name: PEG-100 stearate); and mixtures thereof.

As glyceryl esters of fatty acids mention is made of glyceryl stearate (glyceryl mono-, di- and/or tristearate) (CTFA name: glyceryl stearate) or glyceryl ricinoleate and mixtures thereof.

As glyceryl esters of $C_8$-$C_{24}$ alkoxylated fatty acids, polyethoxylated glyceryl stearate (glyceryl mono-, di- and/or tristearate) such as PEG-20 glyceryl stearate can for example be cited.

Mixtures of these surfactants, such as for example the product containing glyceryl stearate and PEG-100 stearate, marketed under the name ARLACEL 165 by Uniqema, and the product containing glyceryl stearate (glyceryl mono- and distearate) and potassium stearate marketed under the name TEG1N by Goldschmidt (CTFA name: glyceryl stearate SE), can also be used.

Alkyl polyglucosides are a class of useful nonionic surfactants. Non-limiting examples of alkyl polyglucosides include alkyl polyglucosides having the following formula:

$$\mathbf{R^1\text{-}O\text{-}(R^2O)_n\text{-}Z(x)}$$

wherein $R^1$ is an alkyl group having 8-18 carbon atoms;

$R^2$ is an ethylene or propylene group;

Z is a saccharide group with 5 to 6 carbon atoms;

n is an integer from 0 to 10; and

x is an integer from 1 to 5.

Useful alkyl poly glucosides include lauryl glucoside, octyl glucoside, decyl glucoside, coca glucoside, sucrose laurate, caprylyl/capryl glucoside, and sodium lauryl glucose carboxylate, and mixtures thereof. Typically, the at least one alkyl poly glucoside compound is selected from the group consisting of lauryl glucoside, decyl glucoside and coca glucoside, and more typically lauryl glucoside. In some instances, decyl glucoside is particularly preferred.

*Polyglycerol-Based Emulsifiers*

Polyglycerol-based emulsifiers are a useful type of nonionic emulsifiers. Nonlimiting examples include polyglyceryl 10-stearate, polyglyceryl-3-caprate, polyglyceryl-3-diisostearate, polyglyceryl-3 methylglucose distearate, or a mixture thereof. More generally, the polyglycerol-based emulsifiers may be polyglycerol esters of fatty acids having a structure in accordance with the following formula:

$$R^1\text{-}(OCH_2\text{-}\underset{|}{\overset{OR^2}{CH}}\text{-}CH_2O)_n\text{-}R^3$$

wherein n is from 2 to 20 or from 2 to 10 or from 2 to 5, or is 2, 3, 4, 5, 6, 7, 8, 9, or 10, and $R^1$, $R^2$ and $R^3$ each may independently be a fatty acid moiety or hydrogen, provided that at least one of $R^1$, $R^2$, and $R^3$ is a fatty acid moiety. For instance, $R^1$, $R^2$ and $R^3$ may be saturated or unsaturated, straight or branched, and have a length of $C_1$-$C_{40}$, $C_1$-$C_{30}$,

$C_1$-$C_{25}$, or $C_1$-$C_{20}$, $C_1$-$C_{16}$, or $C_1$-$C_{10}$. Additionally, non-limiting examples of nonionic polyglycerol esters of fatty acids include polyglyceryl-4 caprylate/caprate, polyglyceryl-10 caprylate/caprate, polyglyceryl-4 caprate, polyglyceryl-10 caprate, polyglyceryl-4 laurate, polyglyceryl-5 laurate, polyglyceryl-6 laurate, polyglyceryl-10 laurate, polyglyceryl-10 cocoate, polyglyceryl-10 myristate, polyglyceryl-10 oleate, polyglyceryl-10 stearate, and mixtures thereof.

In certain embodiments, the polyglycerol-based emulsifier may be chosen from polyglyceryl esters of C12-22 saturated, unsaturated and branched chain fatty acids, such as polyglyceryl-4 isostearate, polyglyceryl-3 oleate, polyglyceryl-2-sesquioleate, triglyceryl diisostearate, diglyceryl monooleate, tetraglyceryl monooleate, and mixtures thereof. Non-limiting examples of glyceryl esters can include glyceryl caprylate, glyceryl caprate, glyceryl cocoate, glyceryl laurate, and combinations thereof.

Nonlimiting examples of polyglycerolated fatty acid esters include polyglyceryl-10 laurate; polyglyceryl-10 myristate; polyglyceryl-2 palmitate, polyglyceryl-3 palmitate, polyglyceryl-6 palmitate, polyglyceryl-10 palmitate, polyglyceryl-2 isostearate, polyglyceryl-3 isostearate, polyglyceryl-4 isostearate, polyglyceryl-5 isostearate, polyglyceryl-6 isostearate, polyglyceryl-10 isostearate; polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, and mixtures thereof. In some instances, polyglyceryl-2 isostearate is particularly useful.

In a preferred embodiment, the one or more nonionic emulsifiers are chosen from sorbitan fatty esters (e.g., sorbitan isostearate and sorbitan oleate), ethoxylated sorbitan fatty esters (e.g., polysorbate-80), polyol esters, glyceryl esters, polyglucosides (e.g., cetearyl glucoside), and mixtures thereof

## Ethoxylated Fatty Acids

"Ethoxylated fatty acids" are also known as "ethoxylated fatty acid esters" and polyethoxylated fatty acids." They are formed when a fatty acid is reacted with an alkylene oxide. The resulting product may be a monoester, diester, or mixture thereof.

The ethoxylated fatty acids can be represented by the formula $R-C(O)O(CH_2CH_2O)_n-H$, wherein R represents the aliphatic residue of a fatty acid and n represents the number of molecules of ethylene oxide. In another aspect, n is an integer ranging from 2 to 200, 2 to 150, 2 to 100, 2 to 50, 3 to 200, 3 to 150, 3 to 100, 3 to 50, or 3 to 25 in another aspect, and 3 to 10 in a further aspect. In still another aspect of the invention, R is derived from a fatty acid containing 8 to 24 carbon atoms. Exemplary ethoxylated fatty acids include but are not limited to capric acid ethoxylate, lauric acid ethoxylate, myristic acid ethoxylate, stearic acid ethoxylate, oleic acid ethoxylate, coconut fatty acid ethoxylate, and polyethylene glycol 400 propoxylated monolaurate, wherein the number of ethylene oxide units in each of the foregoing ethoxylates can range from 2 and above in one aspect, and from 2 to about 200 in another aspect, from 2 to 200, 2 to 150, 2 to 100, 2 to 50, 3 to 200, 3 to 150, 3 to 100, 3 to 50, or 3 to 25. More specific examples of ethoxylated fatty acids are PEG-8 distearate (the 8 meaning the number of repeating ethylene oxide units), PEG-8 behenate, PEG-8 caprate, PEG-8 caprylate, PEG-8 caprylate/caprate, PEG cocoates (PEG without a number designation meaning that the number of ethylene oxide units ranges from 2 to 50), PEG-15 dicocoate, PEG-2 diisononanoate, PEG-8 diisostearate, PEG-dilaurates, PEG-dioleates PEG-distearates, PEG Ditallates, PEG-isostearates, PEG-jojoba acids, PEG-laurates, PEG-linolenates, PEG-myristates, PEG-oleates, PEG-palmitates, PEG-ricinoleates, PEG-stearates, PEG-tallates, and the like.

Examples of ethoxylated fatty acids that may be mentioned include the adducts of ethylene oxide with esters of lauric acid, palmitic acid, stearic acid or behenic acid, and mixtures thereof, especially those containing from 9 to 100 oxyethylene groups, such as PEG-9 to PEG-50 laurate (as the CTFA names: PEG-9 laurate to PEG-50 laurate); PEG-9 to PEG-50 palmitate (as the CTFA names: PEG-9 palmitate to PEG-50 palmitate); PEG-9 to PEG-50 stearate (as the CTFA names: PEG-9 stearate to PEG-50 stearate); PEG-9 to PEG-50 palmitostearate; PEG-9 to PEG-50 behenate (as the CTFA names: PEG-9 behenate to PEG-50 behenate); polyethylene glycol 100 EO monostearate (CTFA name: PEG-100 stearate); and mixtures thereof.

In some instance, the one or more ethoxylated fatty acids is chosen from polethoxylated stearic acid esters, for example, PEG-9 stearate, PEG-8 distearate, PEG-20 stearate, PEG-8 stearate, PEG-8 oleate, PEG-20 stearate, PEG-30 stearate, PEG-40 stearate, PEG-50 stearate, PEG-100 stearate, PEG-150 laurate, and combinations therefore.

## Nonionic Emulsifiers having an HLB from 9 to 12

Nonlimiting examples of nonionic emulsifiers having an HLB of about 9 to about 15 include alkylpolyglucosides **(*cetearyl glucoside*),** polyglycerol-based emulsifiers (polygyceryl-3 methylglucose distearate), sorbitan fatty esters (polysorbate 60), sugar esters or ethers, sugar-based esters or ethers, polyol fatty esters or ethers, glyceryl fatty esters or ethers, ethoxylates thereof, or mixtures thereof

In a preferred embodiment, one or more of the nonionic emulsifiers having an HLB of about 9 to about 15 are sugar esters or ethers or sugar-based esters and ethers. "Sugar ester" as used herein means "sugar alcohol fatty acid ester" or "sugar acid fatty alcohol ester" and "sugar ether" as used herein means "sugar alcohol fatty alcohol ether." The

sugar-based esters and ethers of can be esters or ethers of (a) a sugar, a sugar alcohol, or a sugar derivative and (b) a fatty acid or fatty alcohol. In some embodiments, esters may be preferred over ethers, and vice-versa. The esters and ethers may be formed by the combination of a sugar, sugar alcohol, or sugar derivative with a fatty acid, or by the combination of a sugar, sugar alcohol, or sugar derivative with a fatty alcohol. For example, a sucrose laurate ester may be formed by the combination of sucrose with lauric acid or lauryl sucronic acid ester, and unless otherwise specified herein, it is not necessary to the present embodiments that a particular ester (or ether) is the result of esterification (or etherification) with a fatty acid as opposed to a fatty alcohol, or vice-versa.

Preferred sugars of the esters and ethers include monosaccharides, disaccharides, and oligosaccharides, and in a preferred embodiment, the sugar is a mono-, di-, or tri-saccharide, or a mixture thereof. Exemplary sugars include allose, altrose, arabinose, cellobiose, erythrose, erythrulose, fructose, fucose, galactose, gentiobiose, glucose, gulose, idose, isomaltose, lactose, lactulose, lyxose, maltose, maltotriose, mannobiose, mannose, melezitose, raffinose, rhamnose, ribose, ribulose, sorbose, sucrose, talose, threose, trehalose, xylobiose, xylose and xylulose. In a preferred embodiment, the sugar is glucose or sucrose. Exemplary sugar alcohols include allitol, arabitol, ducitol, erythritol, galactitol, glycerol, glycol, iditol, inositol, isomalt, lactitol, mallitol, maltitol, mannitol, sorbitol, and xylitol. Exemplary sugar derivatives such as sulfonated sugars and sugar amines can also be used in the present embodiments. These examples are non-limiting examples, and it should be understood that any saccharide, sugar alcohol, or other sugar derivative that will provide a hydrophilic head group to the ester or ether and is otherwise pharmaceutically acceptable is suitable for use in the present embodiments.

The fatty acids and fatty alcohols used in the esters and ethers may be any fatty acid or fatty acid alcohol capable of providing a hydrophobic tail group such that the ester or ether can exert surface active properties. The fatty acids and fatty alcohols can be short chain (i.e., less than 8 carbons in length), medium chain (i.e., 8 to 14 carbons in length), or long chain (i.e., more than 14 carbons in length). Branched or unbranched fatty acids and fatty alcohols can be used. Non-limiting examples of suitable saturated fatty acids include butyric, caproic, caprylic, capric, lauric, myristic, palmitic, stearic, arachidic, and behenic acids, and non-limiting examples of suitable unsaturated fatty acids include myristoleic, palmitoleic, oleic, linoleic, linolenic, arachidonic, eicosapentaenoic, erucic, and docosahexaenoic acids. Non-limiting examples of suitable linear fatty alcohols include caproyl(caproic), caprylic, capric, lauryl, myristyl, palmityl(cetyl), palmitoleyl, stearyl, oleyl, linoleyl, linolenyl, arachidyl, behenyl, erucyl and lignoceryl alcohols, and non-limiting examples of suitable branched fatty alcohols include isocetyl, isostearyl, and isobehenyl alcohols.

In a preferred embodiment, the fatty component of the sugar-based ester or ether is a $C_{12}$ fatty component (e.g., stearic acid, stearyl alcohol, lauric acid, lauryl alcohol, etc.), or a $C_8$ to $C_{18}$ fatty component. In another embodiment, a mixture of medium and long chain fatty components is preferred, particularly a mixture of saturated and unsaturated $C_8$ to $C_{18}$ fatty components. In yet another embodiment, a mixture of saturated and unsaturated $C_8$ to $C_{18}$ fatty acids is preferred. In a different embodiment, the sugar-based ester or ether is a di- or triester of a fatty acid or alcohol, or a di- or tri-ether of a fatty alcohol.

The sugar-based esters and ethers can be monoesters or monoethers, or can be di-, tri-, or poly-esters and ethers, depending on the sugar or sugar alcohol selected for use. In an embodiment, mono- and di-esters, or mono- and di-ethers are preferred. The esterification and etherification may occur at any free hydroxyl group in the sugar or sugar alcohol.

Mixtures of esters or ethers are also contemplated for use. The mixtures may be varied in a number of ways. For example, a particular ester used in a formulation, such as a sugar monostearate, may comprise a variety of sugar monostearate esters, each esterified at a different hydroxyl group. Or, for example, a sugar stearate may comprise a variety of esters with varying degrees of esterification, for example a monostearate, a distearate, a tristearate, etc. Or, in yet another example, a sugar mono acid or sugar diacid may comprise mono- or di-esterification with stearyl alcohol fatty component. In still another example, a mixture containing a particular ester such as a sugar stearate may contain predominantly esters of stearyl acid (or stearyl alcohol), but also contain esters of other fatty components, such as, e.g., esters of myristic acid (or myristyl alcohol), etc. In yet another example, an "ester" may already be a mixture of fatty acids to form the esters, for example the "ester" sucrose cocoate is actually a mixture of esters including the laurate, palmitic, myristic, stearic and caproic esters of sucrose with smaller quantities of other short and long chain fatty acids as well as mixed di- and tri-esters. These non-limiting examples of mixtures apply equally to sugar-based ethers as well as esters.

The sugar-based esters and ethers may be prepared by any suitable means known in the art, for example by incubating an aqueous mixture of a sugar or sugar-alcohol, a fatty acid and a catalytically active amount of a lipolytic enzyme, and recovering the resulting ester from the mixture. Other methods include admixing the sugar with a fatty acid chloride at about 80° C, with simple removal of the hydrogen chloride formed and recovery of the sugar fatty acid ester. Similarly, a mixture of a methyl fatty acid ester and sugar can be heated at a temperature of about 90°C in the presence of a base catalyst, distilling the methanol formed and recovering the sugar fatty acid ester. Many suitable esters and ethers are also commercially available.

The fatty acid esters of sugars may be chosen in particular from the group comprising esters or mixtures of esters of $C_8$-$C_{22}$ fatty acid and of sucrose, of maltose, of glucose or of fructose, and esters or mixtures of esters of $C_{14}$-$C_{22}$ fatty

acid and of methylglucose.

The $C_8$-$C_{22}$ or $C_{14}$-$C_{22}$ fatty acids forming the fatty unit of the esters which can be used comprise a saturated or unsaturated linear alkyl or alkenyl chain containing, respectively, from 8 to 22 or from 14 to 22 carbon atoms. The fatty unit of the esters may be chosen in particular from stearates, behenates, arachidonates, palmitates, myristates, laurates and caprates, and mixtures thereof. Stearates are preferably used.

As examples of esters or mixtures of esters of fatty acid and of sucrose, of maltose, of glucose or of fructose, mention may be made of sucrose monostearate, sucrose distearate and sucrose tristearate and mixtures thereof; and examples of esters or mixtures of esters of fatty acid and of methylglucose which may be mentioned are polyglyceryl-3 methylglucose distearate. Mention may also be made of glucose or maltose monoesters such as methyl o-hexadecanoyl-6-D-glucoside and o-hexadecanoyl-6-D-maltoside.

In an embodiment, the cosmetic composition preferably includes at least one ester of $C_{14}$-$C_{22}$ fatty acids and of methylglucose, for example, polyglyceryl-3 methylglucose distearate.

The fatty alcohol ethers of sugars, which can be used may be solid at a temperature of less than or equal to 45°C. and may be chosen in particular from the group comprising ethers or mixtures of ethers of $C_8$-$C_{22}$ fatty alcohol and of glucose, of maltose, of sucrose or of fructose, and ethers or mixtures of ethers of a $C_{14}$-$C_{22}$ fatty alcohol and of methylglucose. These are in particular, alkylpolyglucosides, which in various embodiments are preferred. A particularly preferred examples is C12-20 alkyl glucoside. Accordingly, in an embodiment, the cosmetic compositions include at least one alkylpolyglucosides, for example, C12-20 alkyl glucoside.

The $C_8$-$C_{22}$ or $C_{14}$-$C_{22}$ fatty alcohols forming the fatty unit of the ethers which may be used comprise a saturated or unsaturated, linear alkyl or alkenyl chain containing, respectively, from 8 to 22 or from 14 to 22 carbon atoms. The fatty unit of the ethers may be chosen in particular from decyl, cetyl, behenyl, arachidyl, stearyl, palmityl, myristyl, lauryl, capryl and hexadecanoyl units, and mixtures thereof, such as cetearyl.

As examples of fatty alcohol ethers of sugars, mention may be made of alkylpolyglucosides such as decylglucoside and laurylglucoside, cetostearyl glucoside, arachidyl glucoside, and mixtures thereof. Further mention is made of sucrose monostearate, sucrose distearate or sucrose tristearate and mixtures thereof, polyglyceryl-3 methylglucose distearate and alkylpolyglucosides.

In certain embodiments, the nonionic emulsifiers having an HLB from about 9 to about 15 may be chosen from: polyglyceryl fatty acid esters of at least one fatty acid comprising at least one saturated or unsaturated, linear or branched $C_8$-$C_{22}$ hydrocarbon group such as $C_8$-$C_{22}$ alkyl or alkenyl group, preferably $C_8$-$C_{18}$ alkyl or alkenyl group, and more preferably $C_8$-$C_{12}$ alkyl or alkenyl group, and of 2 to 12 glycerols, preferably 2 to 10 glycerols and more preferably 2 to 8 glycerols; polyoxyethylenated alkyl glycerides such as polyethylene glycol derivatives of a mixture of mono-, di- and triglycerides of caprylic and capric acids (preferably 2 to 30 ethylene oxide units, more preferably 2 to 20 ethylene oxide units, and even more preferably 2 to 10 ethylene oxide units); polyoxyethylenated fatty ethers of at least one, preferably one, fatty alcohol comprising at least one saturated or unsaturated, linear or branched $C_8$-$C_{22}$ hydrocarbon group such as $C_8$-$C_{22}$ alkyl or alkenyl group, preferably $C_8$-$C_{18}$ alkyl or alkenyl group, and more preferably $C_8$-$C_{12}$ alkyl or alkenyl group, and of 2 to 60 ethylene oxides, preferably from 2 to 30 ethylene oxides, and more preferably from 2 to 10 ethylene oxides; and mixtures thereof.

It is preferable that the polyglyceryl fatty acid ester have a polyglycerol moiety derived from 2 to 10 glycerols, more preferably from 2 to 8 glycerols, and further more preferably 4 to 6 glycerols. The polyglyceryl fatty acid ester may be chosen from the mono, di and tri esters of saturated or unsaturated acid, preferably saturated acid, including 8 to 22 carbon atoms, preferably 8 to 18 carbon atoms, and more preferably 8 to 12 carbon atoms, such as caprylic acid, capric acid, lauric acid, oleic acid, stearic acid, isostearic acid, and myristic acid.

The polyglyceryl fatty acid ester may be selected from the group consisting of PG2 caprate, PG2 dicaprate, PG2 tricaprate, PG2 caprylate, PG2 dicaprylate, PG2 tricaprylate, PG2 laurate, PG2 dilaurate, PG2 trilaurate, PG2 myristate, PG2 dimyristate, PG2 trimyristate, PG2 stearate, PG2 distearate, PG2 tristearate, PG2 isostearate, PG2 diisostearate, PG2 triisostearate, PG2 oleate, PG2 dioleate, PG2 trioleare, PG3 caprate, PG3 dicaprate, PG3 tricaprate, PG3 caprylate, PG3 dicaprylate, PG3 tricaprylate, PG3 laurate, PG3 dilaurate, PG3 trilaurate, PG3 myristate, PG3 dimyristate, PG3 trimyristate, PG3 stearate, PG3 distearate, PG3 tristearate, PG3 isostearate, PG3 diisostearate, PG3 triisostearate, PG3 oleate, PG3 dioleate, PG3 trioleare, PG4 caprate, PG4 dicaprate, PG4 tricaprate, PG4 caprylate, PG4 dicaprylate, PG4 tricaprylate, PG4 laurate, PG4 dilaurate, PG4 trilaurate, PG4 myristate, PG4 dimyristate, PG4 trimyristate, PG4 stearate, PG4 distearate, PG4 tristearate, PG4 isostearate, PG4 diisostearate, PG4 triisostearate, PG4 oleate, PG4 dioleate, PG4 trioleare, PG5 caprate, PG5 dicaprate, PG5 tricaprate, PG5 caprylate, PG5 dicaprylate, PG5 tricaprylate, PG5 laurate, PG5 dilaurate, PG5 trilaurate, PG5 myristate, PG5 dimyristate, PG5 trimyristate, PG5 stearate, PG5 distearate, PG5 tristearate, PG5 isostearate, PG5 diisostearate, PG5 triisostearate, PG5 oleate, PG5 dioleate, PG5 trioleare, PG6 caprate, PG6 dicaprate, PG6 tricaprate, PG6 caprylate, PG6 dicaprylate, PG6 tricaprylate, PG6 laurate, PG6 dilaurate, PG6 trilaurate, PG6 myristate, PG6 dimyristate, PG6 trimyristate, PG6 stearate, PG6 distearate, PG6 tristearate, PG6 isostearate, PG6 diisostearate, PG6 triisostearate, PG6 oleate, PG6 dioleate, PG6 trioleare, PG10 caprate, PG10 dicaprate, PG10 tricaprate, PG10 caprylate, PG10 dicaprylate, PG10 tricaprylate, PG10 laurate, PG10 dilaurate, PG10

trilaurate, PG10 myristate, PG10 dimyristate, PG10 trimyristate, PG10 stearate, PG10 distearate, PG10 tristearate, PG10 isostearate, PG10 diisostearate, PG10 triisostearate, PG10 oleate, PG10 dioleate, PG10 trioleare, and mixtures thereof.

The polyoxyalkylenated fatty ethers, preferably polyoxyethylenated fatty ethers, may comprise from 2 to 60 ethylene oxide units, preferably from 2 to 30 ethylene oxide units, and more preferably from 2 to 10 ethylene oxide units. The fatty chain of the ethers may be chosen in particular from lauryl, behenyl, arachidyl, stearyl and cetyl units, and mixtures thereof, such as cetearyl. Examples of ethoxylated fatty ethers which may be mentioned are lauryl alcohol ethers comprising 2, 3, 4, and 5 ethylene oxide units (CTFA names: Laureth-2, Laureth-3, Laureth-4, and Laureth-5).

The mixed esters of fatty acids, or of fatty alcohol, of carboxylic acid and of glycerol, which can be used as the above nonionic surfactant, may be chosen in particular from the group comprising mixed esters of fatty acid or of fatty alcohol with an alkyl or alkenyl chain containing from 8 to 22 carbon atoms, preferably from 8 to 18 carbon atoms, and more preferably from 8 to 12 carbon atoms, and of alpha-hydroxy acid and/or of succinic acid, with glycerol. The alpha-hydroxy acid may be, for example, citric acid, lactic acid, glycolic acid or malic acid, and mixtures thereof.

The alkyl chain of the fatty acids or alcohols from which are derived the mixed esters which can be used may be linear or branched, and saturated or unsaturated. They may especially be stearate, isostearate, linoleate, oleate, behenate, arachidonate, palmitate, myristate, laurate, caprate, isostearyl, stearyl, linoleyl, oleyl, behenyl, myristyl, lauryl or capryl chains, and mixtures thereof. nonlimiting examples of mixed esters include the mixed ester of glycerol and of the mixture of citric acid, lactic acid, linoleic acid and oleic acid (CTFA name: Glyceryl citrate/lactate/linoleate/oleate); the mixed ester of succinic acid and of isostearyl alcohol with glycerol (CTFA name: Isostearyl diglyceryl succinate); the mixed ester of citric acid and of stearic acid with glycerol (CTFA name: Glyceryl stearate citrate); the mixed ester of lactic acid and of stearic acid with glycerol (CTFA name: Glyceryl stearate lactate), and mixtures thereof.

The fatty esters of sorbitan and oxyalkylenated fatty esters of sorbitan may be chosen from the group comprising $C_{16}$-$C_{22}$ fatty acid esters of sorbitan and oxyethylenated $C_{16}$-$C_{22}$ fatty acid esters of sorbitan. They may be formed from at least one fatty acid comprising at least one saturated linear alkyl chain containing, respectively, from 16 to 22 carbon atoms, and from sorbitol or from ethoxylated sorbitol. The oxyethylenated esters may generally comprise from 1 to 100 ethylene glycol units and preferably from 2 to 40 ethylene oxide (EO) units. These esters may be chosen in particular from stearates, behenates, arachidates, palmitates, and mixtures thereof. Stearates and palmitates are preferably used. Nonlimiting examples include sorbitan monostearate (CTFA name: sorbitan stearate), sorbitan monopalmitate (CTFA name: sorbitan palmitate), sorbitan tristearate 20 EO (CTFA name: polysorbate 65), and mixtures thereof.

The block copolymers of ethylene oxide (A) and of propylene oxide (B), which may be used may be chosen in particular from block copolymers of formula

$$HO(C_2H_4O)_x(C_3H_6O)_y(C_2H_4O)_zH$$

in which x, y and z are integers such that x+z ranges from 2 to 100 and y ranges from 14 to 60, and mixtures thereof.

The polyoxyethylenated (1-40 EO) and polyoxypropylenated (1-30 PO) alkyl ($C_{16}$-$C_{30}$) ethers, which may be used as the above nonionic surfactant, may be selected from PPG-6 Decyltetradeceth-30; Polyoxyethlene (30) Polyoxypropylene (6) Tetradecyl Ether; PPG-6 Decyltetradeceth-12; Polyoxyethylene (12) Polyoxypropylene (6) Tetradecyl Ether; PPG-13 Decyltetradeceth-24; Polyoxyethylene (24) Polyoxypropylene (13) Decyltetradecyl Ether, PPG-6 Decyltetradeceth-20; Polyoxyethylene (20) Polyoxypropylene (6) Decyltetradecyl Ether, PPG-4 Ceteth-1; Polyoxyethylene (1) Polyoxypropylene (4) Cetyl Ether, PPG-8 Ceteth-1; Polyoxyethylene (1) Polyoxypropylene (8) Cetyl Ether, PPG-4 Ceteth-10; Polyoxyethylene (10) Polyoxypropylene (4) Cetyl Ether, PPG-4 Ceteth-20; Polyoxyethylene (20) Polyoxypropylene (4) Cetyl Ether, PPG-5 Ceteth-20; Polyoxyethylene (20) Polyoxypropylene (5) Cetyl Ether, PPG-8 Ceteth-20; Polyoxyethylene (20) Polyoxypropylene (8) Cetyl Ether, and PPG-23 Steareth-34; Polyoxyethylene Polyoxypropylene Stearyl Ether (34 EO) (23 PO).

In various embodiments, it may be more preferable that the polyoxyethylenated (1-40 EO) and polyoxypropylenated (1-30 PO) alkyl ($C_{16}$-$C_{30}$) ethers are (15-40 EO) and polyoxypropylenated (5-30 PO) alkyl ($C_{16}$-$C_{24}$) ethers, which could be selected from the group consisting of PPG-6 Decyltetradeceth-30, PPG-13 Decyltetradeceth-24, PPG-6 Decyltetradeceth-20, PPG-5 Ceteth-20, PPG-8 Ceteth-20, and PPG-23 Steareth-34. It may be even more preferable that the polyoxyethylenated (1-40 EO) and polyoxypropylenated (1-30 PO) alkyl ($C_{16}$-$C_{30}$) ethers are (15-40 EO) and polyoxypropylenated (5-30 PO) alkyl ($C_{16}$-$C_{24}$) ethers, which could be selected from the group consisting of PPG-6 Decyltetradeceth-30, PPG-13 Decyltetradeceth-24, PPG-5 Ceteth-20, and PPG-8 Ceteth-20.

In an embodiment, the one or more nonionic emulsifiers having an HLB from 9 to 12 may be chosen from alkylpolyglucosides (e.g., C12-20 alkyl glucoside), polyglyceryl-10 laurate, polyglyceryl-2 dipolyhydroxystearate, polyglyceryl-2 polyhydroxystearate, polyglyceryl-3 caprylate, polyglyceryl-3 laurate, polyglyceryl-3 methylglucose distearate, polyglyceryl-3 oleate, polyglyceryl-3 palmitate, polyglyceryl-3 polyricinoleate, polyglyceryl-3 ricinoleate, polyglyceryl-5 laurate, polyglyceryl-6 dicaprate, polyglyceryl-6 oleate, polyglyceryl-6 stearate, and a mixture thereof.

### Glyceryl Fatty Esters

Nonlimiting examples of glyceryl fatty esters (or simply glyceryl esters) include glyceryl behenate, glyceryl erucate, glyceryl hydroxystearate, glyceryl lanolate, glyceryl laurate, glyceryl linoleate, glyceryl myristate, glyceryl palmitate lactate, glyceryl stearate, glyceryl distearate, or a mixture thereof. preferred glyceryl esters include glyceryl stearate, glyceryl ricinoleate, or a mixture thereof.

In various embodiments, the one or more glyceryl esters have are chosen from glyceryl esters that are solid at a temperature of below 30°C.

Suitable non-limiting examples of glyceryl esters having an HLB of about 3 to about 6 are chosen from glyceryl behenate, glyceryl erucate, glyceryl hydroxystearate, glyceryl isostearate, glyceryl lanolate, glyceryl laurate, glyceryl linoleate, glyceryl myristate, glyceryl oleate, ***glyceryl stearate,*** glyceryl dioleate, glyceryl distearate, or a mixture thereof. In at least one instance, the glyceryl ester comprises ***glyceryl stearate,*** glyceryl ricinoleate, or a mixture thereof.

In some instances, the glyceryl esters may be chosen from esters of an oligomeric glycerol, arachidyl propionate, phytosterol esters, triglycerides of fatty acids and derivatives thereof, noncrosslinked polyesters resulting from the poly condensation between a linear or branched C4-C50 dicarboxylic acid or polycarboxylic acid and a C2-C50 diol or polyol, aliphatic esters of an ester resulting from the esterification of an aliphatic hydroxycarboxylic acid ester with an aliphatic carboxylic acid, and a mixture thereof. Non-limiting examples of glyceryl esters include glyceryl behenate, glyceryl erucate, glyceryl hydroxystearate, glyceryl isostearate, glyceryl lanolate, glyceryl laurate, glyceryl linoleate, glyceryl myristate, glyceryl oleate, glyceryl stearate, glyceryl stearate citrate, glyceryl distearate, glyceryl laurate, or a mixture thereof. In at least one instance the glyceryl ester comprises glyceryl stearate, bis-diglyceryl polyacyladipate, or a mixture thereof. In at least one other instance, the glyceryl ester comprises glyceryl stearate.

### Nonionic Emulsifiers having an HLB of about 16 to about 18

Nonlimiting examples of nonionic emulsifiers having an HLB of about 16 to about 18 include ethoxylated emulsifiers, for example, ethoxylated fatty acids, ethoxylated sorbitan fatty esters, and mixtures thereof. In some instances, it is preferably that the nonionic emulsifiers having an HLB of about 16 to about 18 include one or more ethoxylated fatty acids.

Nonlimiting examples of ethoxylated fatty acids include the adducts of ethylene oxide with esters of lauric acid, palmitic acid, stearic acid or behenic acid, and mixtures thereof, especially those containing from 9 to 100 oxyethylene groups, such as PEG-9 to PEG-50 laurate (as the INCI names: PEG-9 laurate to PEG-50 laurate); PEG-9 to PEG-50 palmitate (as the INCI names: PEG-9 palmitate to PEG-50 palmitate); PEG-9 to PEG-50 stearate (as the INCI names: PEG-9 stearate to PEG-50 stearate); PEG-9 to PEG-50 palmitostearate; PEG-9 to PEG-50 behenate (as the INCI names: PEG-9 behenate to PEG-50 behenate); polyethylene glycol 100 EO monostearate (INCI name: PEG-100 stearate); and mixtures thereof.

Nonlimiting examples of ethoxylated sorbitan fatty esters include polysorbate-20 (POE(20) sorbitan monolaurate), polysorbate-21 (POE(4) sorbitan monolaurate), polysorbate-40 (POE(20) sorbitan monopalmitate), polysorbate-60 (POE(20) sorbitan monostearate), polysorbate-61 (POE(4) sorbitan monostearate), polysorbate-65 (POE(20) sorbitan tristearate), polysorbate-80 (POE(20)sorbitan monooleate), polysorbate-81 (POE(4) sorbitan monooleate), polysorbate 85 (POE(20) Sorbitan Trioleate), sorbitan isostearate, sorbitan monolaurate, sorbitan monooleate, sorbitan monopalmitate, sorbitan monostearate, sorbitan sesquioleate, sorbitan trioleate and sorbitan tristearateand a mixture thereof.

### Polyglycerol-Based Emulsifiers

Nonlimiting examples include polyglyceryl 10-stearate, polyglyceryl-3-caprate, polyglyceryl-3-diisostearate, polyglyceryl-3 methylglucose distearate, or a mixture thereof. In various embodiments, the polyglycerol-based emulsifiers may be polyglycerol esters of fatty acids having a structure in accordance with the following formula:

$$R^1\text{-}(OCH_2\text{-}\overset{OR^2}{\overset{|}{C}}H\text{-}CH_2O)_n\text{-}R^3$$

wherein n is from 2 to 20 or from 2 to 10 or from 2 to 5, or is 2, 3, 4, 5, 6, 7, 8, 9, or 10, and $R^1$, $R^2$ and $R^3$ each may independently be a fatty acid moiety or hydrogen, provided that at least one of $R^1$, $R^2$, and $R^3$ is a fatty acid moiety. For instance, $R^1$, $R^2$ and $R^3$ may be saturated or unsaturated, straight or branched, and have a length of $C_1$-$C_{40}$, $C_1$-$C_{30}$, $C_1$-$C_{25}$, or $C_1$-$C_{20}$, $C_1$-$C_{16}$, or $C_1$-$C_{10}$. Additionally, non-limiting examples of nonionic polyglycerol esters of fatty acids include polyglyceryl-4 caprylate/caprate, polyglyceryl-10 caprylate/caprate, polyglyceryl-4 caprate, polyglyceryl-10 caprate, polyglyceryl-4 laurate, polyglyceryl-5 laurate, polyglyceryl-6 laurate, polyglyceryl-10 laurate, polyglyceryl-10 cocoate, polyglyceryl-10 myristate, polyglyceryl-10 oleate, polyglyceryl-10 stearate, and mixtures thereof.

In certain embodiments, the polyglycerol-based emulsifier may be chosen from polyglyceryl esters of C12-22 saturated, unsaturated and branched chain fatty acids, such as polyglyceryl-4 isostearate, polyglyceryl-3 oleate, polyglyceryl-2-sesquioleate, triglyceryl diisostearate, diglyceryl monooleate, tetraglyceryl monooleate, and mixtures thereof. Non-limiting examples of glyceryl esters can include glyceryl caprylate, glyceryl caprate, glyceryl cocoate, glyceryl laurate, and combinations thereof.

In some embodiments, particularly useful polyglycerol-based emulsifiers include polyglyceryl methylglucose surfactants, such as polyglyceryl-3 methylglucose distearate, polyglyceryl-6 methylglucose distearate, polyglyceryl-10 methyl glucose distearate, and mixtures thereof.

**Crosslinked Emulsifying Silicone Elastomers**

Various types of crosslinked silicone emulsifying elastomers are known. They typically contain at least one hydrophilic moiety such as polyoxyalkylenated groups. Typically, these polyoxyalkylenated silicone elastomers are crosslinked organopolysiloxanes that may be obtained by a crosslinking addition reaction of diorganopolysiloxane comprising at least one hydrogen bonded to silicon and of a polyoxyalkylene comprising at least two ethylenically unsaturated groups. In at least one embodiment, the polyoxyalkylenated crosslinked organo-polysiloxanes are obtained by a crosslinking addition reaction of a diorganopolysiloxane comprising at least two hydrogens each bonded to a silicon, and a polyoxyalkylene comprising at least two ethylenically unsaturated groups, optionally in the presence of a platinum catalyst.

In various embodiments, useful crosslinked emulsifying silicone elastomers include, among others, polyoxyalkylenated emulsifying silicone elastomers (e.g., dimethicone/PEG-10/15 crosspolymer and PEG-10 Dimethicone), polyglycerolated emulsifying silicone elastomers, and mixtures thereof

Nonlimiting examples of polyglycerolated silicone elastomers include Shin-Etsu's KSG series, such as KSG-710 which is dimethicone/polyglycerin-3 crosspolymer dispersed in dimethicone; or lauryl dimethicone/polyglycerin-3 crosspolymer dispersed in a variety of solvents such as isododecane, dimethicone, triethylhexanoin, sold under the Shin-Etsu tradenames KSG-810, KSG-820, KSG-830, or KSG-840. Also suitable are silicones sold by Dow Corning under the tradenames 9010 and DC9011.

In an embodiment, linear or branched type crosslinked emulsifying silicone elastomers may be used. Exemplary but nonlimiting crosslinked emulsifying silicone elastomers include those with the following International Nomenclature of Cosmetic Ingredients (INCI) designations: Bis-Butyldimethicone Polyglyceryl-3; Bis-PEG/PPG-14/14 Dimethicone; Bis-butyldimethicone Polyglyceryl-3; Bis-isobutyl PEG/PPG-10/7 Dimethicone copolymer; Bis-PEG/PPG-18/6 Dimethicone; Bis-PEG/PPG-20/20 Dimethicone; Bis-PEG/PPG-16/16 PEG/PPG-16/16 Dimethicone; Bis(PPG-7 Undeceneth-21-Dimethicone; Cetyl Dimethicone PEG-7 Acetate; Cetyl PEG-8 Dimethicone; Cetyl PEG/PPG-15/16 Butyl Ether Dimethicone; Cetyl PEG/PPG-15/15 Butyl Ether Dimethicone; Cetyl PEG/PPG-7/3 Dimethicone; Cetyl PEG/PPG-10/1 Dimethicone; Dimethicone PEG-15 Acetate; Dimethicone PEG-7 Cocoate; Dimethicone PEG-7 Phosphate; Dimethicone PEG-IO Phosphate; Dimethicone PEG/PPG-7/4 Phosphate; Dimethicone PEG/PPG-12/4 Phosphate; Dimethicone PEG-7 Undecylenate; Lauryl Dimethicone PEG-IO Phosphate; Isopolyglyceryl-3 Dimethicone; Isopolyglyceryl-3 Dimethiconol; Isostearyl Carboxyldecyl PEG-8 Dimethicone; Lauryl Methicone PEG-10 Phosphate; Lauryl PEG-8 Dimethicone; Lauryl PEG-10 Methyl Ether Dimethicone; Lauryl PEG/PPG-18/18 Methicone; PEG-6 Methyl Ether Dimethicone; PEG-7 Methyl Ether Dimethicone; PEG-9 Methyl Ether Dimethicone; PEG-10 Methyl Ether Dimethicone; PEG-11 Methyl Ether Dimethicone; PEG-11 Methyl Ether Dimethicone; PEG-32 Methyl Ether Dimethicone; PEG-PEG/PPG-28/21 Acetate Dimethicone; PEG/PPG-22/22 Butyl Ether Dimethicone; PEG/PPG-23/23 Butyl Ether Dimethicone; PEG/PPG-24/18 Butyl Ether Dimethicone; PEG/PPG-3/10 Dimethicone; PEG/PPG-4/12 Dimethicone; PEG/PPG-6/11 Dimethicone; PEG/PPG-8/14 Dimethicone; PEG/PPG-12/16 Dimethicone; PEG/PPG-12/18 Dimethicone; PEG/PPG-14/4 Dimethicone; PEG/PPG-15/5 Dimethicone; PEG/PPG-15/15 Dimethicone; PEG/PPG-16/2 Dimethicone; PEG/PPG-16/8 Dimethicone; PEG/PPG-17/18 Dimethicone; PEG/PPG-18/12 Dimethicone; PEG/PPG-19/19 Dimethicone; PEG/PPG-20/6 Dimethicone; PEG/PPG-20/15 Dimethicone; PEG/PPG-20/20 Dimethicone; PEG/PPG-20/29 Dimethicone; PEG/PPG-22/23 Dimethicone; PEG/PPG-22/24 Dimethicone; PEG/PPG-25/25 Dimethicone; PEG/PPG-27/27 Dimethicone; PEG/PPG-30/10 Dimethicone; PEG/PPG-10/3 Oleyl Ether Dimethicone; PEG-8 trisiloxane; Polyglyceryl-3 Polydimethylsiloxyethyl Dimethicone; PPG-12 Butyl Ether Dimethicone; Silicone Quaternium-17; TEA-Dimethicone PEG-7 Phosphate; and mixtures thereof.

In an embodiment, nonlimiting examples of crosslinked emulsifying silicone elastomers that may be included in the cosmetic compositions include dimethicone/dimethicone PEG/PPG 15 crosspolymer; dimethicone PEG-10 crosspolymer; dimethicone PEG-10/15 crosspolymer; dimethicone PEG-15 crosspolymer; dimethicone polyglycerin-3 crosspolymer; dimethicone PPG-20 crosspolymer; lauryl dimethicone PEG-15 crosspolymer; lauryl dimethicone polyglycerin-3 crosspolymer; PEG-8 dimethicone polysorbate-20 crosspolymer; PEG-10 dimethicone/vinyl dimethicone crosspolymer;

PEG-10 lauryl dimethicone crosspolymer; PEG-15/lauryl dimethicone crosspolymer; PEG-15 laurylpolydimethylsiloxy ethyl crosspolymer; and mixtures thereof.

In an embodiment, mention may be made of dimethicone crosspolymer, cetearyl dimethicone crosspolymer, cetearyldimethicone/vinyldimethicone crosspolymer, dimethicone/vinyldimethicone crosspolymer, dimethicone/polyethylene glycol (PEG)-10 crosspolymer, dimethicone/PEG-15 crosspolymer, dimethicone/polyglyceryl-3 crosspolymer, dimethicone/silsesquioxane copolymer, dimethicone/phenylvinyl dimethicone crosspolymer, vinyl dimethicone/lauryl dimethicone crosspolymer, dimethicone/bis-isobutyl polypropylene glycol (PPG)-20 crosspolymer, PEG-12 dimethicone/PPG-20 crosspolymer, lauryl dimethicone PEG-15 crosspolymer, lauryl dimethicone/polyglycerin-3 crosspolymer, and mixtures thereof.

In a preferred embodiment, the one or more crosslinked emulsifying silicone elastomers are chosen from dimethicone/PEG-10/15 crosspolymer, PEG-15 lauryl dimethicone crosspolymer, PEG-15 lauryl dimethicone crosspolymer, PEG-15 lauryl dimethicone crosspolymer, PEG-10 lauryl dimethicone crosspolymer, PEG-15 lauryl dimethicone crosspolymer, PEG-12 dimethicone crosspolymers, dimethicone/dimethicone PEG/PPG 15 crosspolymer, dimethicone PEG-10 crosspolymer, dimethicone PEG-10/15 crosspolymer, dimethicone PEG-15 crosspolymer, dimethicone polyglycerin-3 crosspolymer, dimethicone PPG-20 crosspolymer, lauryl dimethicone PEG-15 crosspolymer, lauryl dimethicone polyglycerin-3 crosspolymer, PEG-8 dimethicone polysorbate-20 crosspolymer, PEG-10 dimethicone/vinyl dimethicone crosspolymer, PEG-10 lauryl dimethicone crosspolymer, PEG-15/lauryl dimethicone crosspolymer, PEG-15 laurylpolydimethylsiloxy ethyl crosspolymer, and mixtures thereof.

## Silicone Oils

Nonlimiting examples of silicones include dimethicone, dimethiconol, dimethiconol, cyclomethicone, polysilicone-11, phenyl trimethicone, trimethylsilylamodimethicone, and stearoxytrimethylsilane. In a preferred embodiment, the one or more silicones oils are non-volatile silicon oils. Useful silicone oils include polydimethylsiloxanes (PDMSs), polydimethylsiloxanes comprising alkyl or alkoxy groups which are pendent and/or at the end of the silicone chain, which groups each contain from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates. Other examples of silicone oils that may be mentioned include volatile linear or cyclic silicones, such as those with a viscosity 8 centistokes (8x106 m2/s) and/or containing from 2 to 7 silicon atoms. These silicones optionally comprise alkyl or alkoxy groups containing from 1 to 10 carbon atoms. Non-limiting examples of volatile silicone oils include octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, or mixtures thereof.

In a preferred embodiment, the cosmetic compositions include one or more silicone oils chosen from dimethicone, dimethiconol, cyclomethicone, polysilicone-11, phenyl trimethicone, and amodimethicone; and preferably includes dimethicone.

In an embodiment, the cosmetic compositions include one or more amino functionalized silicones. Nonlimiting examples include amodimethicone, bis-hydroxy/methoxy amodimethicones, bis-cetearyl amodimethicone, amodimethicone, bis(C13-15 alkoxy) PG amodimethicones, aminopropyl phenyl trimethicones, aminopropyl dimethicones, bis-amino PEG/PPG-41/3 aminoethyl PG-propyl dimethicones, caprylyl methicones, and a mixture thereof. Amodimethicone is a particularly useful amino functionalized silicone.

In a preferred embodiment the one or more silicone oils are chosen from dimethicone, dimethiconol, cyclopentasiloxane, cyclomethicone, cyclotetrasiloxane, cyclohexasiloxane, cycloheptasiloxane, decamethylcyclopentasiloxane, cyclotetrasiloxane, cyclotrisiloxane, capryldimethicone, caprylyl trimethicone, caprylyl methicone, cetearylmethicone, hexadecylmethicone, hexylmethicone, lauryl methicone, myristyl methicone, phenyl methicone, stearyl methicone, stearyl dimethicone, behenyl dimethicone, trifluoropropyl methicone, cetyl dimethicone, polyphenylmethylsiloxane, dimethylpolysiloxane, methylphenylpolysiloxane, methyltrimethicone, diphenylsiloxyphenyl trimethicone, and phenyl trimethicone, and mixtures thereof. Preferably, one of the one or more silicone oils is dimethicone, and the comsetic compositions optionally include one or more additional silicone oils.

## Miscellaneous Ingredients

Miscellaneous ingredients are ingredients that are compatible with the cosmetic compositions and do not disrupt or materially affect the basic and novel properties of the cosmetic compositions. Miscellaneous ingredients commonly used in cosmetics are known in the art. Non-limiting examples include miscellaneous emulsifiers/surfactant different from the crosslinked emulsifying silicone elastomers of (d), preservatives, fragrances, pH adjusters, salts, buffers, antioxidants, flavonoids, vitamins, botanical extracts, UV filtering agents, proteins, protein hydrolysates and/or isolates, hydrotropes, pearlescent agents, fillers, colorants, mattifying agents, further skin active agents, depigmenting agents, anti-wrinkle

agents, *etc.* In a preferred embodiment, the cosmetic compositions of the instant disclosure include one or more skin active agents, in particular, madecassoside. Nonlimiting examples of various miscellaneous ingredients that may optionally be include (or excluded) from the cosmetic compositions is provided below.

*Miscellaneous Emulsifiers/Surfactants*

Miscellaneous emulsifiers/surfactants may optionally be included in the cosmetic compositions. Miscellaneous emulsifiers/surfactants are those that are different from the crosslinked emulsifying silicone elastomers of (d). The miscellaneous emulsifiers/surfactants may be nonionic, anionic, cationic, and/or amphoteric/zwitterionic.

*Antioxidants*

Examples of antioxidants include tocopherols (e.g. d-α-tocopherol, d-β-tocopherol, d-γ-tocopherol, d-delta-tocopherol), tocotrienols (e.g. d-α-tocotrienol, d-β-tocotrienol, d-γ.-tocotrienol, d-delta-tocotrienol,) and vitamin E (α-tocopherol acetate). These compounds may be isolated from natural sources, prepared by synthetic means, or mixtures thereof. Tocotrienol-enriched vitamin E preparations may be obtained by fractionating vitamin E preparations to remove a portion of tocopherols and recover a preparation more highly concentrated in tocotrienol. Useful tocotrienols are natural products isolated, for example, from wheat germ oil, grain, or palm oil using high performance liquid chromatography, or isolated by alcohol extraction and/or molecular distillation from barley, brewer's grain or oats. As used herein, the term "tocotrienols" includes tocotrienol-rich-fractions obtained from these natural products as well as the pure compounds. The increased glutathione peroxidase activity protects the skin from oxidative damage.

Vitamin C and derivatives may be used, including ascorbic acid, sodium ascorbate, and the fat soluble esters tetrahexyldecyl ascorbate and ascorbyl palmitate, magnesium ascorbyl phosphate, ascorbyl-glucoside, glucosamine ascorbate, ascorbyl acetate, etc. Additionally, extracts from plants containing a high amount of vitamin C such as camu berry (Myrciaria dubia), acerola, emblica officinalis, and bioflavonoids from rose hip and citrus may be used including water soluble bioflavonoids such as hesperidin methyl chalcone may also be used.

Sesame (Sesamum indicum) or sesame lignan may also be added. Sesame and its lignans (the fibrous compounds associated with the sesame) act as antioxidants. Sesame seed lignans significantly enhance vitamin E activity.

In addition, carotenoids, particularly the xanthophyll type, are also useful antioxidants that can be used. The xanthopyll type carotenoids include molecules, such as lutein, canthaxantin, cryptoxanthin, zeaxanthin and astaxanthin. Xanthophylls protect compounds, such as vitamin A, vitamin E, and other carotenoids.

Flavonoids can also function as antioxidants. In some instances, the flavonoid is a flavanone (derivative of 2,3-dihydro-2-phenylchromen-4-one). Flavones include: Butin, Eriodictyol, Hesperetin, Hesperidin, Homoeriodictyol, Isosakuranetin, Naringenin, Naringin, Pinocembrin, Poncirin, Sakuranetin, Sakuranin, and Sterubin. The flavonoid may be a flavanonol (derivative of 3-hydroxy-2,3-dihydro-2-phenylchromen-4-one). Flavanols include: Taxifolin, Aromadedrin, Chrysandroside A, Chrysandroside B, Xeractinol, Astilbin, and Fustin. The flavonoid may be a flavone (derivative of 2-phenylchromen-4-one). Flavones include: Apigenin, Luteolin, Tangeritin, Chrysin, Baicalein, Scutellarein, Wogonin, Synthetic Flavones: Diosmin, and Flavoxate. The flavonoid may be a flavonol (derivative of 3-hydroxy-2-phenylchromen-4-one). Flavonols include: 3-Hydroxyflavone, Azaleatin, Fisetin, Galangin, Gossypetin, Kaempferide, Kaempferol, Isorhamnetin, Morin, Myricetin, Natsudaidain, Pachypodol, Quercetin, Rhamnazin, Rhamnetin, Azalein, Hyperoside, Isoquercitin, Kaempferitrin, Myricitrin, Quercitrin, Robinin, Rutin, Spiraeoside, Xanthorhamnin, Amurensin, Icariin, and Troxerutin. The flavonoid may be a flavan-3-ol (derivatives of 2-phenyl-3,4-dihydro-2H-chromen-3-ol). Flavan-3-ols include: Catechin, Epicatechin, Epigallocatechin, Epicatechin gallate, Epigallocatechin gallate, Epiafzelechin, Fisetinidol, Guibourtinidol, Mesquitol, and Robinetinidol. The flavonoid may be a flavan-4-ol (derivative of 2-phenylchroman-4-ol). Flavan-4-ols include: Apiforol and Luteoforol. The flavonoid may be an isoflavone (derivative of 3-phenylchromen-4-one). Isoflavones include: Genistein, Daidzein, Biochanin A, Formononetin, and the Equol metabolite from Daidzein.

The antioxidant may be an anthocyanidin (derivative of 2-phenylchromenylium cation). Anthocyanidins include: Aurantinidin, Cyanidin, Delphinidin, Europinidin, Luteolinidin, Pelargonidin, Malvidin, Peonidin, Petunidin, Rosinidin, and Xanthone.

The antioxidant may be a Dihydrochalcone (derivative of 1,3-diphenyl-1-propanone). Dihydrochalcones include: Phloretin, Dihydrochalcone phloretin Phlorizin, Aspalathin, Naringin dihydrochalcone, Neohesperidin dihydrochalcone, and Nothofagin. Without limiting the mode of action of the invention, dihydrochalcones may exert an antioxidant effect by reducing reactive free radicals, like reactive oxygen and reactive nitrogen species.

The antioxidant may be an anthocyanin. Anthocyanins and their derivatives are antioxidants. Anthocyanins encompasses a class of flavonoid compounds that are naturally occurring, water-soluble compounds, responsible for the red, purple, and blue colors of many fruits, vegetables, cereal grains, and flowers. Additionally, anthocyanins are collagenase inhibitors. The inhibition of collagenase helps in the prevention and reduction of wrinkles, increase in skin

elasticity, etc., which are caused by a reduction in skin collagen. The anthocyanins may be obtained from any portion of various plant sources, such as the fruit, flower, stem, leaves, root, bark, or seeds. One of skill in the art will understand that certain portions of the plant may contain higher natural levels of anthocyanins, and, therefore, those portions are used to obtain the desired anthocyanins. In some instances, antioxidants may include one or more betacyanin. Betacyanins, like anthocyanins, may be obtained from natural sources and are antioxidants.

The antioxidant may be a Phenylpropanoid (derivatives of cinnamic acid). Phenylpropanoids include: Cinnamic acid, Caffeic acid, Ferulic acid, Transferulic acid (including its antioxidant pharmacore 2,6-dihydroxyacetophenome), 5-Hydroxyferulic acid, Sinapic acid, Coumaryl alcohol, Coniferyl alcohol, Sinapyl alcohol, Eugenol, Chavicol, Safrole, P-coumaric acid, and Sinapinic acid. Without limiting the mode of action of the invention, Phenylpropanoids may neutralize free radicals.

The antioxidant may be a Chalcone (derivative of 1,3-diphenyl-2-propen-1-one). Chalcones include: Butein, Okanin, Carthamin, Marein, Sophoradin, Xanthohumol, Flavokvain A, Flavokavain B, Flavokavin C, and synthetic Safalcone.

The antioxidant may be a Curcuminoid. Curcuminoids include: Curcumin, Desmethoxycurcumin, bis-Desmethoxycurcumin, Tetrahydrocurcumin, and Tetrahydrocurcuminoids. Curcumin and tetrahydrocurcuminoids may be derived from rhizomes of Curcuma longa. Tetrahydrocurcumin, a metabolite of curcumin, has been found to be a more potent antioxidant and more stable compared to curcumin.

The antioxidant may be a Tannin. Tannins include: Tannin, Terflavin B, Glucogallin, Dgallic acid, and Quercitannic acid.

The antioxidant may be a stilbenoid. Stilbenoids include: Resveratrol, Pterostilbene, and Piceatannol. Resveratrol may include, but is not limited to, 3,5,4'-trihydroxystilbene, 3,4,3',5'-tetrahydroxystilbene (piceatannol), 2,3',4,5'-tetrahydroxystilbene (oxyresveratrol), 4,4'-dihydroxystilbene, and alpha and beta glucoside, galactoside and mannoside derivatives thereof.

The antioxidant may be a Coumarin (derivatives of 2H-chromen-2-one). Coumarins include: 4-Hydroxycoumarin, Umbelliferone, Aesculetin, Herniarin, Auraptene, and Dicoumarol.

The antioxidant may be a Carotenoid. Carotenoids include: beta-Carotene, alpha-Carotene, gamma-Carotene, beta-Cryptoxanthin, Lycopene, Lutein, and Idebenone. Sesame (Sesamum indicum) or sesame lignan may also be added. Sesame and its lignans (the fibrous compounds associated with the sesame) act as antioxidants. Sesame seed lignans significantly enhance vitamin E activity.

The antioxidant may be: a Xanthone, Butylated Hydroxytoluene, 2,6-Di-tert-butylphenol, 2,4-Dimethyl-6-tert-butylphenol, Gallic acid, Eugenol, Uric acid, alpha-Lipoic acid, Ellagic acid, Chicoric acid, Chlorogenic acid, Rosmarinic acid, Salicylic acid, Acetylcysteine, S-Allyl cysteine, Barbigerone, Chebulagic acid, Edaravone, Ethoxyquin, Glutathione, Hydroxytyrosol, Idebenone, Melatonin, N-Acetylserotonin, Nordihydroguaiaretic acid, Oleocanthal, Oleuropein, Paradol, Piceatannol, Probucol, Propyl gallate, Protocatechuic acid, Pyritinol, Rutin, Secoisolariciresinol diglucoside, Sesamin, Sesamol, Silibinin, Silymarin, Theaflavin, Theaflavin digallate, Thmoquinone, Trolox, Tyrosol, Polyunsaturated fatty acids, and sulfur-based antioxidants such as Methionine or Lipoic acid.

*Skin active agents*

Nonlimiting examples of skin active agents include madecassoside, retinoic acid, benzoyl peroxide, sulfur, vitamin B6 (pyridoxine or) chloride, selenium, samphire--the cinnamon extract blends, tea and octanoylglycine such as--15 Sepicontrol A5 TEA from Seppic--the mixture of cinnamon, sarcosine and octanoylglycine marketed especially by Seppic under the trade name Sepicontrol A5- -zinc salts such as zinc gluconate, zinc pyrrolidonecarboxylate (or zinc pidolate), zinc lactate, zinc aspartate, zinc carboxylate, zinc salicylate 20, zinc cysteate;-derivatives particularly copper and copper pidolate as Cuivridone Solabia--extracts from plants of Arnica montana, Cinchona succirubra, Eugenia caryophyllata, Humulus lupulus, Hypericum perforatum, Mentha pipenta 25 Rosmarinus officinalis, Salvia officinalis and Thymus vulgaris, all marketed for example by Maruzen-extracts of meadowsweet (Spiraea ulmaria), such as that sold under the name Sebonormine by Silab--extracts of the alga Laminaria saccharina, such as that sold under the 30 name Phlorogine by Biotechmarine--the root extracts of burnet mixtures (Sanguisorba officinalis/Poterium officinale), rhizomes of ginger (Zingiber officinalis) and cinnamon bark (Cinnamomum cassia), such as that sold under the name Sebustop by Solabia--extracts of flaxseed such as that sold under the name Linumine by Lucas Meyer--Phellodendron extracts such as those sold under the name Phellodendron extract BG by Maruzen or Oubaku liquid B by Ichimaru Pharcos--of argan oil mixtures extract of Serenoa serrulata (saw palmetto) extract and sesame seeds such as that sold under the name Regu SEB by Pentapharmmixtures of extracts of willowherb, of Terminalia chebula, nasturtium and of bioavailable zinc (microalgae), such as that sold under the name Seborilys Green Tech;--extracts of Pygeum afrianum such as that sold under the name Pygeum afrianum sterolic lipid extract by Euromed--extracts of Serenoa serrulata such as those sold under the name Viapure Sabal by Actives International, and those sold by the company Euromed--of extracts of plantain blends, Berberis aquifolium and sodium salicylate 20 such as that sold under the name Seboclear Rahn--extract of clove

as that sold under the name Clove extract powder by Maruzen--argan oil such as that sold under the name Lipofructyl Laboratories Serobiologiques; 25--lactic protein filtrates, such as that sold under the name Normaseb by Sederma--the seaweed laminaria extracts, such as that sold under the name Laminarghane by Biotechmarine--oligosaccharides seaweed Laminaria digitata, such as that sold under the name Phycosaccharide 30 AC by the company Codif--extracts of sugar cane such as that sold under the name Policosanol by the company Sabinsa, the sulfonated shale oil, such as that sold under the name Ichtyol Pale by Ichthyol-extracts of meadowsweet (Spiraea ulmaria) such as that sold under the name Cytobiol Ulmaire by societeLibiol--sebacic acid, especially sold in the form of a sodium polyacrylate gel under the name Sebosoft by Sederma--glucomannans extracted from konjac tuber and modified with alkylsulfonate chains such as that sold under the name Biopol Beta by Arch Chemical--extracts of Sophora angustifolia, such as those sold under the name Sophora powder or Sophora extract by Bioland-extracts of cinchona bark succirubra such as that sold under the name Red Bark HS by Alban Muller--extracts of Quillaja saponaria such as that sold under the name 15 Panama wood HS by Alban Muller--glycine grafted onto an undecylenic chain, such as that sold under the name Lipacide UG OR by SEPPIC--the mixture of oleanolic acid and nordihydroguaiaretic acid, such as that sold under the form of a gel under the name AC.Net by Sederma; 20--phthalimidoperoxyhexanoic acid--citrate tri (C12-C13) sold under the name COSMACOL.RTM ECI by Sasol; trialkyl citrate (C14-C15) sold under the name COSMACOL.RTM. ECL by Sasol--10-hydroxydecanoic acid, including mixtures acid-hydroxydecanoic October 25, sebacic acid and 1,10-decandiol such as that sold under the name Acnacidol BG by Vincience and mixtures thereof.

*Depigmenting agents*

Nonlimiting examples of depigmenting agents include alpha and beta arbutin, ferulic acid, lucinol and its derivatives, kojic acid, resorcinol and derivatives thereof, tranexamic acid and derivatives thereof, gentisic acid, homogentisic, methyl gentisate or homogentisate, dioic acid, D pantheteine calcium sulphonate, lipoic acid, ellagic acid, vitamin B3, linoleic acid and its derivatives, certain compounds derived from plants such as chamomile, bearberry, the aloe family (vera, ferox, bardensis), mulberry, skullcap, a water kiwi fruit (Actinidia chinensis) marketed by Gattefosse, an extract of Paeonia suffruticosa root, such as that sold by Ichimaru Pharcos under the name Liquid Botanpi Be an extract of brown sugar (Saccharum officinarum) such as molasses extract marketed by Taiyo Kagaku under the name Liquid Molasses, without this list being exhaustive. Particular depigmenting agents include alpha and beta arbutin, ferulic acid, kojic acid, resorcinol and derivatives, D pantheteine calcium sulfonate, lipoic acid, ellagic acid, vitamin B3, a water kiwi fruit (Actinidia chinensis) marketed by Gattefosse, an extract of Paeonia suffruticosa root, such as that sold by the company Ichimaru Pharcos under the name Botanpi Liquid B.

*Anti-wrinkle agent*

The term "anti-wrinkle agent" refers to a natural or synthetic compound producing a biological effect, such as the increased synthesis and/or activity of certain enzymes, when brought into contact with an area of wrinkled skin, this has the effect of reducing the appearance of wrinkles and/or fine lines. Nonlimiting examples of anti-wrinkle agents include: desquamating agents, anti-glycation agents, inhibitors of NO-synthase, agents stimulating the synthesis of dermal or epidermal macromolecules and/or preventing their degradation, agents for stimulating the proliferation of fibroblasts and/or keratinocytes, or for stimulating keratinocyte differentiation reducing agents; muscle relaxants and/or dermo-decontracting agents, anti-free radical agents, and mixtures thereof. Examples of such compounds are: adenosine and its derivatives and retinoids other than retinol (as discussed above, such as retinol palmitate), ascorbic acid and its derivatives such as magnesium ascorbyl phosphate and ascorbyl glucoside; nicotinic acid and its precursors such as nicotinamide; ubiquinone; glutathione and precursors thereof such as L-2-oxothiazolidine-4-carboxylic acid, the compounds C-glycosides and their derivatives as described in particular in EP-1345919, in particular C-beta-D-xylopyranoside-2-hydroxy-propane as described in particular in EP-1345919, plant extracts including sea fennel and extracts of olive leaves, as well as plant and hydrolysates thereof such as rice protein hydrolysates or soybean proteins; algal extracts and in particular laminaria, bacterial extracts, the sapogenins such as diosgenin and extracts of Dioscorea plants, in particular wild yam, comprising: the $\alpha$-hydroxy acids, f3-hydroxy acids, such as salicylic acid and n-octanoyl-5-salicylic oligopeptides and pseudodipeptides and acyl derivatives thereof, in particular acid {2-[acetyl-(3-trifluoromethyl-phenyl)-amino]-3-methyl-} acetic acid and lipopeptides marketed by the company under the trade names SEDERMA Matrixyl 500 and Matrixyl 3000; lycopene, manganese salts and magnesium salts, especially gluconates, and mixtures thereof. In at least one case, the skin tightening composition includes adenosine derivatives, such as non-phosphate derivatives of adenosine, such as in particular the 2'-deoxyadenosine, 2',3'-adenosine isopropoylidene; the toyocamycine, 1-methyladenosine, N-6-methyladenosine; adenosine N-oxide, 6-methylmercaptopurine riboside, and the 6-chloropurine riboside. Other derivatives include adenosine receptor agonists such as adenosine phenylisopropyl ("PIA"), 1-methylisoguanosine, N6-cyclohexyladenosine (CHA), N6-cyclopentyladenosine (CPA), 2-chloro-N6-cyclopentyladenosine, 2-chloroadenosine, N6-phenyladenosine, 2-phenylaminoadenosine, MECA, N 6-phenethyladenosine, 2-p-(2-carboxy-ethyl) phenethyl-amino-5'-

-N-ethylcarboxamido adenosine (CGS-21680), N-ethylcarboxamido-adenosine (NECA), the 5'(N-cyclopropyl)-carboxamidoadenosine, DPMA (PD 129.944) and metrifudil.

As already noted, skin active agents may be included as one or more of the miscellaneous ingredients. With respect to the total amount of skin active agents in the cosmetic compositions, if present, the total amount of skin active agents may be from greater than zero to about 9 wt.%, greater than zero to about 8 wt.%, greater than zero to about 7 wt.%, greater than zero to about 6 wt.%, greater than zero to about 5 wt.%, greater than zero to about 4 wt.%, greater than zero to about 3 wt.%, greater than zero to about 2 wt.%; about 10 ppm to about 10 wt.% (100,000 ppm), about 10 ppm to about 5 wt.% (50,000 ppm), about 10 ppm to about 2.5 wt.% (25,000 ppm), about 10 ppm to about 1 wt.% (10,000 ppm), about 10 ppm to about 0.5 wt.% (5,000 ppm), about 10 ppm to about 0.3 wt.% (3,000 ppm), about 10 ppm to about 0.2 wt.% (2,000 ppm), about 10 ppm to about 0.1 wt.% (1,000 ppm), about 10 ppm to 500 ppm; about 0.1 to about 10 wt.%, about 0.1 to about 5 wt.%, about 0.1 to about 2.5 wt.%, about 0.1 to about 1 wt.%, about 0.1 to about 0.5 wt.%; about 1 to about 10 wt.%, about 1 to about 8 wt.%, about 1 to about 6 wt.%, about 1 to about 5 wt.%, about 1 to about 4 wt.%, about 1 to about 3 wt.%; about 2 to about 10 wt.%, about 2 to about 8 wt.%, about 2 to about 6 wt.%, about 2 to about 5 wt.%, about 2 to about 4 wt.%; about 3 to about 10 wt.%, about 3 to about 8 wt.%, about 3 to about 6 wt.%, about 3 to about 5 wt.%; about 4 to about 10 wt.%, about 4 to about 8 wt.%, or about 4 to about 6 wt.%, based on the total weight of the cosmetic composition.

**Example 15**

(Miscibility Data)

Equal parts of various compositions where physically mixed together to determine whether they were miscible with one another. The degree of miscibility was assessed on a scale from 1 to 5, where 1 indicates very poor miscibility (i.e., formulation of clumps, sedimentation, coagulation, phase separation, etc.) and 5 indicates excellent miscibility (no clumps, sedimentation, coagulation, phase separation, etc.). The results are presented in the tables below.

| | Anhydrous Example 15A | Anhydrous Example 15B | Oil in Water Example 1C | Anhydrous + Oil in Water Example 15A + Example 1C |
|---|---|---|---|---|
| Water in Silicone Example 6B | 5 | 5 | 5 | 5 |
| Oil in Water Example 3B | 5 | 5 | 5 | 5 |
| Oil in Water Example 9B | 4 | 4 | 5 | 5 |
| Water in Silicone Commercial Benchmark | 1 | 1 | 2 | 4 |
| Anhydrous Commercial Benchmark | 1 | 1 | 1 | 1 |

| | Anhydrous Example 15A | Anhydrous Example 15B | Oil in Water Example 1B | Anhydrous + Oil in Water Example 15A + Example 1B |
|---|---|---|---|---|
| Water in Silicone Example 11C | 2 | 2 | 2 | 5 |

**DEFINITIONS**

Miscibility as used herein relates to the ability of one or more cosmetic composition to be physically mixed and form a uniform mixture for immediate application to the skin without visible signs of clumping, sedimentation, coagulation, and phase separation.

As used herein, a "gel emulsion" is also referred to in the art as "emulsion gel." A gel emulsion is an oil in water emulsion, which is a composite structure of oil droplets within a gel matrix. They can be categorized as emulsion-filled gels and emulsion particulate gels.

As used herein, the terms "comprising," "having," and "including" (or "comprise," "have," and "include") are used in their open, non-limiting sense. The phrase "consisting essentially of" limits the scope of a claim to the specified materials or steps and those that do not materially affect the basic and novel characteristics of the claimed invention.

The terms "a," "an," and "the" are understood to encompass the plural as well as the singular.

Thus, the term "a mixture thereof" also relates to "mixtures thereof." Throughout the disclosure, if the term "a mixture thereof" is used, following a list of elements as shown in the following example where letters A-F represent the

elements: "one or more elements selected from the group consisting of A, B, C, D, E, F, or mixtures thereof." The term, "a mixture thereof" does not require that the mixture include all of A, B, C, D, E, and F (although all of A, B, C, D, E, and F may be included). Rather, it indicates that a mixture of any two or more of A, B, C, D, E, and F can be included. In other words, it is equivalent to the phrase "one or more elements chosen from A, B, C, D, E, F, and a mixture of any two or more of A, B, C, D, E, and F."

Likewise, the term "a salt thereof" also relates to "salts thereof." Thus, where the disclosure refers to "an element selected from the group consisting of A, B, C, D, E, F, a salt thereof, or mixtures thereof," it indicates that that one or more of A, B, C, D, and F may be included, one or more of a salt of A, a salt of B, a salt of C, a salt of D, a salt of E, and a salt of F may be included, or a mixture of any two of A, B, C, D, E, F, a salt of A, a salt of B, a salt of C, a salt of D, a salt of E, and a salt of F may be included.

The salts referred to throughout the disclosure may include salts having a counter-ion such as an alkali metal, alkaline earth metal, or ammonium counterion. This list of counterions, however, is non-limiting. Appropriate counterions for the components described herein are known in the art.

The expression "one or more" means "at least one" and thus includes individual components as well as mixtures/combinations.

The term "plurality" means "more than one" or "two or more."

An "alkyl radical" is a linear or branched saturated hydrocarbon-based group, particularly $C_1$-$C_8$, more particularly $C_1$-$C_6$, preferably $C_1$-$C_4$ such as methyl, ethyl, isopropyl and tert-butyl;

An "alkoxy radical" is a alkyl-oxy wherein alkyl is as described herein before ;

An "alkenyl radical" is a linear or branched unsaturated hydrocarbon-based group, particularly $C_2$-$C_8$, more particularly $C_2$-$C_6$, preferably $C_2$-$C_4$ such as ethylenyl, propylenyl;

An "alkylene radical" is a linear or branched divalent saturated $C_1$-$C_8$, in particular $C_1$-$C_6$, preferably $C_1$-$C_4$ hydrocarbon-based group such as methylene, ethylene or propylene.

Some of the various categories of components identified for the cosmetic compositions may overlap. In such cases where overlap may exist and the composition/product includes two overlapping components (or more than two overlapping components), an overlapping component does not represent more than one component. As an example, a fatty acid may be considered both a "non-triglyceride and non-aromatic fatty emollient" and a "surfactant/emulsifier." If a particular composition/product includes both a non-triglyceride and non-aromatic fatty emollient component and an surfactant/emulsifier component, a single type of fatty acid can serve as only a non-triglyceride and non-aromatic fatty emollient or a surfactant/emulsifier (a single fatty acid does not serve as both the non-triglyceride and non-aromatic fatty emollient component and the surfactant/emulsifier component).

All percentages, parts and ratios herein are based upon the total weight of the compositions of the present invention, unless otherwise indicated.

All ranges and values disclosed herein are inclusive and combinable. For examples, any value or point described herein that falls within a range described herein can serve as a minimum or maximum value to derive a sub-range, etc. Furthermore, all ranges provided are meant to include every specific range within, and combination of sub-ranges between, the given ranges. Thus, a range from 1-5, includes specifically points 1, 2, 3, 4 and 5, as well as sub-ranges such as 2-5, 3-5, 2-3, 2-4, 1-4, etc.; and points of 1, 2, 3, 4, and 5 includes ranges and sub-ranges of 1-5, 2-5, 3-5, 2-3, 2-4, 1-4, etc.

Other than in the operating examples, or where otherwise indicated, all numbers expressing quantities of ingredients and/or reaction conditions may be modified with the term "about," whether or not expressly stated.

Additionally, all numbers are intended to represent exact values as additional embodiments, whether or not modified by the term "about." For example, "an amount of about 1%" can be modified to refer to exactly 1%. As a further example, "an amount of 1%" can be modified to refer to "about 1%." Unless otherwise indicated, the term "about" is understood to encompass a range of +/- 10% from the stated number. However, in some embodiments, the term may be defined to encompass narrower ranges, for example, +/- 1%, 2%, 3%, 4%, 5%, 6%, 7%, 8%, 9%, and 10% from the stated number.

The term "surfactants" and "emulsifiers" include salts of the surfactants and emulsifiers even if not explicitly stated. In other words, whenever the disclosure refers to a surfactant or emulsifier, it is intended that salts are also encompassed to the extent such salts exist, even though the specification may not specifically refer to a salt (or may not refer to a salt in every instance throughout the disclosure), for example, by using language such as "a salt thereof" or "salts thereof." Sodium and potassium are common cations that form salts with surfactants and emulsifiers. However, additional cations such as ammonium ions, or alkanolammonium ions such as monoethanolammonium or triethanolammonium ions, may also form salts of surfactants.

The term "substantially free" or "essentially free" as used herein means the specific material may be present in small amounts that do not materially affect the basic and novel characteristics of the claimed invention. For instance, there may be less than 2% by weight of a specific material added to a composition, based on the total weight of the composition (provided that an amount of less than 2% by weight does not materially affect the basic and novel characteristics of the

claimed invention). Similarly, a composition "substantially free" or "essentially free" of a stated material may include less than 1.5 wt.%, less than 1 wt.%, less than 0.5 wt.%, less than 0.1 wt.%, less than 0.05 wt.%, or less than 0.01 wt.%, or none of the specified material. The term "substantially free" or "essentially free" as used herein may also mean that the specific material is not added to the composition but may still be present in a raw material that is included in the composition.

Furthermore, all components that are positively set forth in the instant disclosure may be negatively excluded from the claims, e.g., a claimed composition may be "free," "essentially free" (or "substantially free") of one or more components that are positively set forth in the instant disclosure. As an example, Silicones can optionally be included in the cosmetic compositions but preferably the compositions are free or essentially free from silicones. Silicones are synthetic polymers made up of repeating units of siloxane, elemental silicon and oxygen, combined with other elements, most often carbon and hydrogen. Thus, silicones are also called polysiloxanes. In some instances, cosmetic compositions of the instant case can be free or essentially free from dimethicones, amomdimethicones, dimethiconols, cyclosiloxanes, siloxanes, etc.

## Claims

**1.** A kit comprising two or more separately contained cosmetic compositions that are miscible with one another upon mixing, wherein each of the two or more compositions comprise:

(a) a viscosity of at least 1.8 Pa.s (1,800 cPs) or a storage modulus (G') greater than the loss modulus (G") for a viscosity below 1.8 Pa.s (1,800 cPs); and
(b) a maximum yield stress of 9,000 Pa, for example, a maximum yield stress below 7,500 Pa, below 5,000 Pa, below 2,500 Pa, below 2,000 Pa, below 1,500 Pa, or below 1,250 Pa; and
wherein the two or more cosmetic compositions are chosen from:

(i) oil in water emulsions comprising at least 40 wt.% of water and about 1 to about 20 wt.% of an oil phase, based on the total weight of the oil in water emulsion;
(ii) water in silicone emulsions comprising at least 70 wt.% of an aqueous phase (internal phase), based on the total weight of the silicone emulsion; and
(iii) anhydrous compositions, wherein at least 90 wt.% of ingredients forming the anhydrous compositions have a log P value of 2 or less, for example, 1.5 or less, 1 or less, 0.75 or less, 0.5 or less, 0.25 less, or 0.

**2.** The kit of claim 1, wherein at least one of the two or more compositions is an oil in water emulsion.

**3.** The kit of claim 2, wherein the oil in water emulsion comprises:

(a) trifluoromethylphenyl valylglycine, ceramides or a mixture thereof, and/or
(b) retinol; and/or
(c) hydroxypropyl tetrahydropyrantriol, for example, about 10 to about 40 wt.% of hydroxypropyl tetrahydropyrantriol, based on the total weight of the oil in water emulsion, and/or
(d) 4-t-butylcyclohexanol, and/or
(e) acetyl trifluoromethylphenyl valylglycine, for example, about 1 to about 5 wt.% of acetyl trifluoromethylphenyl valylglycine.

**4.** The kit of claim 1, wherein at least one of the two or more compositions is a water in silicone emulsion, preferably wherein the water in silicone emulsion comprises hydroxypropyl tetrahydropyrantriol, for example, about 15 to about 60 wt.% of hydroxypropyl tetrahydropyrantriol, based on the total weight of the water in silicone emulsion.

**5.** The kit of claim 1, wherein at least one of the two or more compositions is an anhydrous composition, preferably wherein the anhydrous composition comprises ascorbic acid, ferulic acid, or a mixture thereof, for example, about 5 to about 20 wt.% of ascorbic acid and/or about 0.5 to about 10 wt.% of ferulic acid, based on the total weight of the anhydrous composition.

**6.** The kit of any one of the above claims, wherein the kit is an apparatus for independently dispensing a specified amount of each of the two or more cosmetic compositions, wherein the apparatus comprises a dispensing assembly configured to receive a plurality of cartridges in which the two or more cosmetic compositions are separately contained and a receiving area into which each of the two or more cosmetic compositions is dispensed.

7. The apparatus of claim 6, wherein each cartridge of the plurality of cartridges contains a different cosmetic composition.

8. The apparatus of claim 6 or 7, wherein the apparatus further comprises:

a memory configured to receive and store dispensing information which includes the specified amount of cosmetic composition to be dispensed from each cartridge; and
circuitry configured to obtain the dispensing information from the memory and to control the dispensing assembly to dispense the specified amount of the cosmetic composition to be dispensed from each cartridge.

9. A method for treating skin comprising:

(A) mixing two or more cosmetic compositions that are miscible with one another upon mixing, wherein each of the two or more compositions comprise:

(a) a viscosity of at least 1.8 Pa.s (1,800 cPs) or a storage modulus (G') greater than the loss modulus (G") for a viscosity below 1.8 Pa.s (1,800 cPs); and
(b) a maximum yield stress of 9,000 Pa, for example, a maximum yield stress below 7,500 Pa, below 5,000 Pa, below 2,500 Pa, below 2,000 Pa, below 1,500 Pa, or below 1,250 Pa; and
wherein the two or more cosmetic compositions are chosen from:

(i) oil in water emulsions comprising at least 40 wt.% of water and about 1 to about 20 wt.% of an oil phase, based on the total weight of the oil in water emulsion;
(ii) water in silicone emulsions comprising at least 70 wt.% of an aqueous phase (internal phase), based on the total weight of the silicone emulsion; and
(iii) anhydrous compositions, wherein at least 90 wt.% of ingredients forming the anhydrous compositions have a log P value of log P value of 2 or less, for example, 1.5 or less, 1 or less, 0.75 or less, 0.5 or less, 0.25 less, or 0; and

(B) applying the mixture to the skin.

10. The method of claim 9, wherein at least one of the two or more compositions is an oil in water emulsion.

11. The method of claim 10, wherein the oil in water emulsion comprises:

(a) trifluoromethylphenyl valylglycine, and/or
(b) retinol, and/or
(c) hydroxypropyl tetrahydropyrantriol, for example, about 10 to about 40 wt.% of hydroxypropyl tetrahydropyrantriol, based on the total weight of the oil in water emulsion, and/or
(d) 4-t-butylcyclohexanol, and/or
(e) acetyl trifluoromethylphenyl valylglycine, for example, about 1 to about 5 wt.% of acetyl trifluoromethylphenyl valylglycine.

12. The method of claim 11, wherein the water in silicone emulsion comprises hydroxypropyl tetrahydropyrantriol, for example, about 15 to about 60 wt.% of hydroxypropyl tetrahydropyrantriol, based on the total weight of the water in silicone emulsion.

13. The method of any one of claim 9-12, wherein two or more composition are included in an apparatus for independently dispensing a specified amount of each of the two or more cosmetic compositions, wherein the apparatus comprises a dispensing assembly configured to receive a plurality of cartridges in which the two or more cosmetic compositions are separately contained and a receiving area into which each of the two or more cosmetic compositions is dispensed.

14. The method of claim 13, wherein each cartridge of the plurality of cartridges contains a different cosmetic composition.

15. The method of claim 13 or 14, wherein the apparatus further comprises:

a memory configured to receive and store dispensing information which includes the specified amount of cosmetic composition to be dispensed from each cartridge; and

circuitry configured to obtain the dispensing information from the memory and to control the dispensing assembly to dispense the specified amount of the cosmetic composition to be dispensed from each cartridge.

## Patentansprüche

1. Kit, umfassend zwei oder mehrere separat enthaltene kosmetische Zusammensetzungen, die beim Mischen miteinander mischbar sind, wobei jede der zwei oder mehreren Zusammensetzungen Folgendes umfasst:

    (a) eine Viskosität von mindestens 1,8 Pa.s (1800 cPs)

        oder einen Speichermodul (G') größer als
        das Verlustmodul (G") für eine Viskosität unter 1,8 Pa.s (1800 cPs);
        und

    (b) eine maximale Streckspannung von 9000 Pa, zum Beispiel eine maximale Streckspannung unter 7500 Pa, unter 5000 Pa, unter 2500 Pa, unter 2000 Pa, unter 1500 Pa oder unter 1250 Pa; und
    wobei die zwei oder mehreren kosmetischen Zusammensetzungen ausgewählt sind aus:

        (i) Öl-in-Wasser-Emulsionen, umfassend mindestens 40 Gewichts-% Wasser und etwa 1 bis etwa 20 Gewichts-% einer Ölphase, bezogen auf das Gesamtgewicht der Öl-in-Wasser-Emulsion;
        (ii) Wasser-in-Silikon-Emulsionen, umfassend mindestens 70 Gewichts-% einer wässrigen Phase (innere Phase), bezogen auf das Gesamtgewicht der Silikonemulsion; und
        (iii) wasserfreie Zusammensetzungen, wobei mindestens 90 Gewichts-% der Bestandteile, die die wasserfreien Zusammensetzungen bilden, einen log P-Wert von 2 oder weniger, beispielsweise 1,5 oder weniger, 1 oder weniger, 0,75 oder weniger, 0,5 oder weniger, 0,25 oder weniger, oder 0 aufweisen.

2. Kit nach Anspruch 1, wobei mindestens eine der zwei oder mehreren Zusammensetzungen eine Öl-in-Wasser-Emulsion ist.

3. Kit nach Anspruch 2, wobei die Öl-in-Wasser-Emulsion Folgendes umfasst:

    (a) Trifluormethylphenylvalylglycin, Ceramide oder ein Gemisch davon, und/oder
    (b) Retinol; und/oder
    (c) Hydroxypropyltetrahydropyrantriol, zum Beispiel etwa 10 bis etwa 40 Gewichts-% Hydroxypropyltetrahydropyrantriol, bezogen auf das Gesamtgewicht der Öl-in-Wasser-Emulsion, und/oder
    (d) 4-t-Butylcyclohexanol und/oder
    (e) Acetyltrifluormethylphenylvalylglycin, zum Beispiel etwa 1 bis etwa 5 Gewichts-% Acetyltrifluormethylphenylvalylglycin.

4. Kit nach Anspruch 1, wobei mindestens eine der zwei oder mehreren Zusammensetzungen eine Wasser-in-Silikon-Emulsion ist, wobei die Wasser-in-Silikon-Emulsion vorzugsweise Hydroxypropyltetrahydropyrantriol umfasst, beispielsweise etwa 15 bis etwa 60 Gewichts-% Hydroxypropyltetrahydropyrantriol, bezogen auf das Gesamtgewicht der Wasser-in-Silikon-Emulsion.

5. Kit nach Anspruch 1, wobei mindestens eine der zwei oder mehreren Zusammensetzungen eine wasserfreie Zusammensetzung ist, wobei die wasserfreie Zusammensetzung vorzugsweise Ascorbinsäure, Ferulasäure oder eine Mischung davon umfasst, beispielsweise etwa 5 bis etwa 20 Gewichts-% Ascorbinsäure und/oder etwa 0,5 bis etwa 10 Gewichts-% Ferulasäure, bezogen auf das Gesamtgewicht der wasserfreien Zusammensetzung.

6. Kit nach einem der vorherigen Ansprüche, wobei das Kit eine Vorrichtung zum unabhängigen Abgeben einer spezifischen Menge jeder der zwei oder mehreren kosmetischen Zusammensetzungen ist, wobei die Vorrichtung eine Abgabeanordnung umfasst, die konfiguriert ist, um eine Vielzahl von Kartuschen, in denen die zwei oder mehreren kosmetischen Zusammensetzungen separat enthalten sind, und einen Aufnahmebereich, in den jede der zwei oder mehr kosmetischen Zusammensetzungen abgegeben wird, aufzunehmen.

7. Vorrichtung nach Anspruch 6, wobei jede Kartusche der Vielzahl von Kartuschen eine andere kosmetische Zusammensetzung enthält.

8. Vorrichtung nach Anspruch 6 oder 7, wobei die Vorrichtung ferner Folgendes umfasst:

einen Speicher, der konfiguriert ist, um Abgabeinformationen zu empfangen und zu speichern, die die spezifizierte Menge der kosmetischen Zusammensetzung beinhaltet, die aus jeder Kartusche abgegeben werden soll; und
eine Schaltung, die konfiguriert ist, um die Abgabeinformationen aus dem Speicher zu erhalten und die Abgabeanordnung zu steuern, um die spezifizierte Menge der kosmetischen Zusammensetzung abzugeben, die aus jeder Kartusche abzugeben ist.

9. Verfahren zur Hautbehandlung, umfassend:

(A) Mischen von zwei oder mehreren kosmetischen Zusammensetzungen, die beim Mischen miteinander mischbar sind, wobei jede der zwei oder mehreren Zusammensetzungen Folgendes umfasst:

(a) eine Viskosität von mindestens 1,8 Pa.s (1800 cPs)

oder einen Speichermodul (G') größer als
als der Verlustmodul (G") bei einer Viskosität unter 1,8 Pa.s (1800 cPs)

und
(b) eine maximale Streckspannung von 9000 Pa, zum Beispiel eine maximale Streckspannung unter 7500 Pa, unter 5000 Pa, unter 2500 Pa, unter 2000 Pa, unter 1500 Pa oder unter 1250 Pa; und
wobei die zwei oder mehreren kosmetischen Zusammensetzungen ausgewählt sind aus:

(i) Öl-in-Wasser-Emulsionen, umfassend mindestens 40 Gewichts-% Wasser und etwa 1 bis etwa 20 Gewichts-% einer Ölphase, bezogen auf das Gesamtgewicht der Öl-in-Wasser-Emulsion;
(ii) Wasser-in-Silikon-Emulsionen, umfassend mindestens 70 Gewichts-% einer wässrigen Phase (innere Phase), bezogen auf das Gesamtgewicht der Silikonemulsion; und
(iii) wasserfreie Zusammensetzungen, wobei mindestens 90 Gewichts-% der Bestandteile, die die wasserfreien Zusammensetzungen bilden, einen log P-Wert von log P-Wert von 2 oder weniger, beispielsweise 1,5 oder weniger, 1 oder weniger, 0,75 oder weniger, 0,5 oder weniger, 0,25 oder weniger, oder 0 aufweisen; und

(B) Auftragen des Gemischs auf die Haut.

10. Verfahren nach Anspruch 9, wobei mindestens eine der zwei oder mehreren Zusammensetzungen eine Öl-in-Wasser-Emulsion ist.

11. Verfahren nach Anspruch 10, wobei die Öl-in-Wasser-Emulsion Folgendes umfasst:

(a) Trifluormethylphenylvalylglycin, und/oder
(b) Retinol, und/oder
(c) Hydroxypropyltetrahydropyrantriol, zum Beispiel etwa 10 bis etwa 40 Gewichts-% Hydroxypropyltetrahydropyrantriol, bezogen auf das Gesamtgewicht der Öl-in-Wasser-Emulsion, und/oder
(d) 4-t-Butylcyclohexanol und/oder
(e) Acetyltrifluormethylphenylvalylglycin, zum Beispiel etwa 1 bis etwa 5 Gewichts-% Acetyltrifluormethylphenylvalylglycin.

12. Verfahren nach Anspruch 11, wobei die Wasser-in-Silikon-Emulsion Hydroxypropyltetrahydropyrantriol enthält, zum Beispiel etwa 15 bis etwa 60 Gewichts-% Hydroxypropyltetrahydropyrantriol, bezogen auf das Gesamtgewicht der Wasser-in-Silikon-Emulsion.

13. Verfahren nach einem der Ansprüche 9 bis 12, wobei zwei oder mehrere Zusammensetzungen in einer Vorrichtung zum unabhängigen Abgeben einer spezifizierten Menge von jeder der zwei oder mehreren kosmetischen Zusammensetzungen enthalten sind, wobei die Vorrichtung eine Abgabeanordnung umfasst, die konfiguriert ist, um eine Vielzahl von Kartuschen, in denen die zwei oder mehreren kosmetischen Zusammensetzungen separat enthalten sind, und einen Aufnahmebereich, in den jede der zwei oder mehr kosmetischen Zusammensetzungen abgegeben wird, aufzunehmen.

14. Verfahren nach Anspruch 13, wobei jede Kartusche der Vielzahl von Kartuschen eine andere kosmetische Zusammensetzung enthält.

15. Verfahren nach Anspruch 13 oder 14, wobei die Vorrichtung ferner Folgendes umfasst:

einen Speicher, der konfiguriert ist, um Abgabeinformationen zu empfangen und zu speichern, die die spezifizierte Menge der kosmetischen Zusammensetzung beinhaltet, die aus jeder Kartusche abgegeben werden soll; und
eine Schaltung, die konfiguriert ist, um die Abgabeinformationen aus dem Speicher zu erhalten und die Abgabeanordnung zu steuern, um die spezifizierte Menge der kosmetischen Zusammensetzung abzugeben, die aus jeder Kartusche abzugeben ist.

**Revendications**

1. Kit comprenant deux compositions cosmétiques ou plus contenues séparément qui sont miscibles entre elles après mélange, dans lequel chacune des deux compositions ou plus comprend :

(a) une viscosité d'au moins 1,8 Pa.s (1 800 cPs)

ou un module de stockage (G') supérieur au
module de perte (G") pour une viscosité inférieure à 1,8 Pa.s (1 800 cPs) ;

et
(b) une limite d'élasticité maximale de 9 000 Pa, par exemple une limite d'élasticité maximale inférieure à 7 500 Pa, inférieure à 5 000 Pa, inférieure à 2 500 Pa, inférieure à 2 000 Pa, inférieure à 1 500 Pa ou inférieure à 1 250 Pa ; et
dans lequel les deux compositions cosmétiques ou plus sont choisies parmi :

(i) des émulsions huile dans l'eau comprenant au moins 40 % en poids d'eau et de 1 à 20 % en poids d'une phase huileuse, sur la base du poids total de l'émulsion huile dans l'eau ;
(ii) des émulsions d'eau dans du silicone comprenant au moins 70 % en poids d'une phase aqueuse (phase interne), sur la base du poids total de l'émulsion de silicone ; et
(iii) des compositions anhydres, dans lequel au moins 90 % en poids des ingrédients formant les compositions anhydres présentent une valeur log P de 2 ou moins, par exemple, 1,5 ou moins, 1 ou moins, 0,75 ou moins, 0,5 ou moins, 0,25 ou moins, ou 0.

2. Kit selon la revendication 1, dans lequel au moins une des deux ou plus compositions est une émulsion huile dans l'eau.

3. Kit selon la revendication 2, dans lequel l'émulsion huile dans l'eau comprend :

(a) de la trifluorométhylphényl-valylglycine, des céramides
ou un mélange de ceux-ci, et/ou
(b) du rétinol ; et/ou
(c) de l'hydroxypropyl-tétrahydropyrantriol, par exemple d'environ 10 à environ 40 % en poids d'hydroxypropyl-tétrahydropyrantriol, sur la base du poids total de l'émulsion huile dans l'eau, et/ou
(d) du 4-t-butylcyclohexanol, et/ou
(e) de l'acétyl-trifluorométhylphényl-valylglycine, par exemple d'environ 1 à environ 5 % en poids d'acétyl-trifluorométhylphényl-valylglycine.

4. Kit selon la revendication 1, dans lequel au moins une des deux ou plus compositions est une émulsion d'eau dans du silicone, de préférence dans lequel l'émulsion d'eau dans du silicone comprend de l'hydroxypropyl-tétrahydropyrantriol, par exemple, environ 15 à environ 60 % en poids d'hydroxypropyl-tétrahydropyrantriol, sur la base du poids total de l'émulsion d'eau dans du silicone.

5. Kit selon la revendication 1, dans lequel au moins une des deux compositions ou plus est une composition anhydre, de préférence dans lequel la composition anhydre comprend de l'acide ascorbique, de l'acide férulique ou un mélange

de ceux-ci, par exemple, environ 5 à environ 20 % en poids d'acide ascorbique et/ou environ 0,5 à environ 10 % en poids d'acide férulique, sur la base du poids total de la composition anhydre.

**6.** Kit selon l'une quelconque des revendications précédentes, dans lequel le kit est un appareil permettant de distribuer indépendamment une quantité spécifiée de chacune des deux compositions cosmétiques ou plus, dans lequel l'appareil comprend un ensemble de distribution configuré pour recevoir une pluralité de cartouches dans lesquelles les deux ou plus compositions cosmétiques sont contenues séparément et une zone de réception dans laquelle chacune des deux ou plus compositions cosmétiques est distribuée.

**7.** Appareil selon la revendication 6, dans lequel chaque cartouche de la pluralité de cartouches contient une composition cosmétique différente.

**8.** Appareil selon la revendication 6 ou 7, dans lequel l'appareil comprend en outre :

une mémoire configurée pour recevoir et stocker des informations de distribution qui incluent la quantité spécifiée de composition cosmétique à distribuer à partir de chaque cartouche ; et
un circuit configuré pour obtenir les informations de distribution à partir de la mémoire et pour commander l'ensemble de distribution afin de distribuer la quantité spécifiée de la composition cosmétique à distribuer à partir de chaque cartouche.

**9.** Procédé de traitement de la peau comprenant :

(A) le mélange de deux ou plus compositions cosmétiques qui sont miscibles entre elles après mélange, dans lequel chacune des deux ou plusieurs compositions comprend :

(a) une viscosité d'au moins 1,8 Pa.s (1 800 cPs)

ou un module de stockage (G') supérieur au
module de perte (G") pour une viscosité inférieure à 1,8 Pa.s (1 800 cPs) ;

et
(b) une limite d'élasticité maximale de 9 000 Pa, par exemple une limite d'élasticité maximale inférieure à 7 500 Pa, inférieure à 5 000 Pa, inférieure à 2 500 Pa, inférieure à 2 000 Pa, inférieure à 1 500 Pa ou inférieure à 1 250 Pa ; et
dans lequel les deux compositions cosmétiques ou plus sont choisies parmi :

(i) des émulsions huile dans l'eau comprenant au moins 40 % en poids d'eau et de 1 à 20 % en poids d'une phase huileuse, sur la base du poids total de l'émulsion huile dans l'eau ;
(ii) des émulsions d'eau dans du silicone comprenant au moins 70 % en poids d'une phase aqueuse (phase interne), sur la base du poids total de l'émulsion de silicone ; et
(iii) des compositions anhydres, dans lequel au moins 90 % en poids des ingrédients formant les compositions anhydres présentent une valeur log P de 2 ou moins, par exemple, 1,5 ou moins, 1 ou moins, 0,75 ou moins, 0,5 ou moins, 0,25 ou moins, ou 0 ; et

(B) l'application du mélange sur la peau.

**10.** Procédé selon la revendication 9, dans lequel au moins une des deux ou plus compositions est une émulsion huile dans l'eau.

**11.** Procédé selon la revendication 10, dans lequel l'émulsion huile dans l'eau comprend :

(a) de la trifluorométhylphényl-valylglycine, et/ou
(b) du rétinol, et/ou
(c) de l'hydroxypropyl-tétrahydropyrantriol, par exemple d'environ 10 à environ 40 % en poids d'hydroxypropyl-tétrahydropyrantriol, sur la base du poids total de l'émulsion huile dans l'eau, et/ou
(d) du 4-t-butylcyclohexanol, et/ou
(e) de l'acétyl-trifluorométhylphényl-valylglycine, par exemple d'environ 1 à environ 5 % en poids d'acétyl-trifluorométhylphényl-valylglycine.

**12.** Procédé selon la revendication 11, dans lequel l'émulsion d'eau dans du silicone comprend de l'hydroxypropyltétrahydropyrantriol, par exemple, environ 15 à environ 60 % en poids d'hydroxypropyl -étrahydropyrantriol, sur la base du poids total de l'émulsion d'eau dans du silicone.

**13.** Procédé selon l'une quelconque des revendications 9 à 12, dans lequel deux compositions ou plus sont incluses dans un appareil pour distribuer indépendamment une quantité spécifiée de chacune des deux ou plus compositions cosmétiques, dans lequel l'appareil comprend un ensemble de distribution configuré pour recevoir une pluralité de cartouches dans lesquelles les deux ou plus compositions cosmétiques sont contenues séparément et une zone de réception dans laquelle chacune des deux ou plus compositions cosmétiques est distribuée.

**14.** Procédé selon la revendication 13, dans lequel chaque cartouche de la pluralité de cartouches contient une composition cosmétique différente.

**15.** Procédé selon la revendication 13 ou 14, dans lequel l'appareil comprend en outre :

une mémoire configurée pour recevoir et stocker des informations de distribution qui incluent la quantité spécifiée de composition cosmétique à distribuer à partir de chaque cartouche ; et
un circuit configuré pour obtenir les informations de distribution à partir de la mémoire et pour commander l'ensemble de distribution afin de distribuer la quantité spécifiée de la composition cosmétique à distribuer à partir de chaque cartouche.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63284239
- FR 2200833
- WO 2017207805 A1
- EP 1345919 A


### Non-patent literature cited in the description


- **D. SATAS**. Handbook of Pressure Sensitive Adhesive Technology, vol. 9, 155-157
- Cosmetic Raw Material Analysis and Quality. Hydrocarbons, Glycerides, Waxes and Other Esters. 1994, vol. I